# EUROPEAN PATENT SPECIFICATION

(11) **EP 0 621 864 B1**
(45) Date of publication and mention of the grant of the patent: **27.12.1996**
(21) Application number: 92921277.7
(22) Date of filing: 14.10.1992
(51) Int. Cl.: C07D 333/60, C07D 409/12, C07D 417/12, A01N 43/12, A01N 43/40, A01N 43/16, A01N 43/60, A01N 43/78

(54) **2-(2-BENZO(b)THIENYL)-3-METHOXYACRYLATE AND -2-IMINOACETATE DERIVATIVES TO BE USED AS PESTICIDES**
2-(2-BENZO B THIENYL)-3-METHOXYACRYLAT- UND -2-IMINOACETAT-DERIVATE ZUR VERWENDUNG ALS PESTIZIDE
DERIVES DE -2-IMINOACETATE-3-METHOXYACRYLATE ET 2-(2-BENZO(b)THIENYLE), UTILISES COMME PESTICIDES

(30) Priority: 25.10.1991 CH 3137/91
(43) Date of publication of application: 02.11.1994
(73) Proprietor: CIBA-GEIGY AG, 4002 Basel (CH)
(72) Inventor: TRAH, Stephan, D-7800 Freiburg (DE); GANTZ, François, F-68170 Rixheim (FR)
(86) International application number: EP9202370
(87) International publication number: WO9308183

(56) References cited:
- EP-A- 0 414 575
- EP-A- 0 433 233
- EP-A- 0 460 575
- WO-A-90/07493

## Description

The invention relates to compounds of the formula 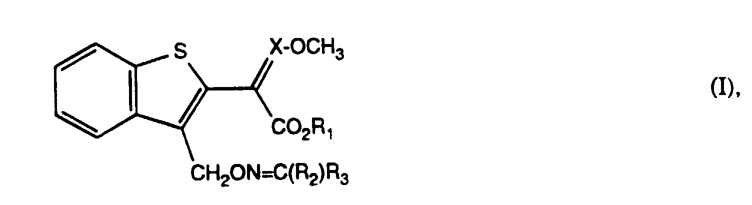 in which:
X is a nitrogen atom or a group of the formula CH (Ia);
R₁ is C₁-C₄alkyl; and
either R₂ and R₃ independently of one another are hydrogen, C₁-C₄alkyl, halo-C₁-C₄alkyl, C₁-C₄alkoxy, C₁-C₄alkoxymethyl, C₁-C₄alkylthio, C₁-C₄alkylthiomethyl, an unsubstituted or substituted aryl, heteroaryl, aryl-C₁-C₄alkyl, heteroaryl-C₁-C₄alkyl, aryloxy-C₁-C₄alkyl, heteroaryloxy-C₁-C₄alkyl, arylthio-C₁-C₄alkyl, heteroarylthio-C₁-C₄alkyl, arylcarbonyl or heteroarylcarbonyl group, C₂-C₄alkenyl, an unsubstituted or substituted aryl-C₂-C₄alkenyl or heteroaryl-C₂-C₄alkenyl group, C₂-C₄alkynyl, C₃-C₆cycloalkyl or cyano,
or R₂ and R₃ together with the carbon atom to which R₂ and R₃ are attached are an unsubstituted or substituted saturated or unsaturated, carbo- or heterocyclic 4- to 7-membered ring to which an unsubstituted or substituted benzene ring may be fused; in free form or, if appropriate, in salt form, to a process for the preparation and to the use of these compounds, and to pesticides whose active ingredient is selected from amongst these compounds, in free form or, if appropriate, in salt form, and to a process for the preparation and to the use of these compositions.

Some of the compounds I can exist in the form of salts. This is because they form acid addition salts when they have at least one basic centre. These acid addition salts are formed for example with strong inorganic acids such as mineral acids, for example sulfuric acid, a phosphoric acid or a hydrohalic acid, with strong organic carboxylic acids such as unsubstituted or substituted, for example halogen-substituted, C₁-C₄alkanecarboxylic acids, for example acetic acids, such as optionally unsaturated dicarboxylic acids, for example oxalic acid, malonic acid, maleic acid, fumaric acid or phthalic acid, such as hydroxycarboxylic acids, for example ascorbic acid, lactic acid, malic acid, tartaric acid or citric acid, or such as benzoic acid, or with organic sulfonic acids such as unsubstituted or substituted, for example halogen-substituted, C₁-C₄alkane- or arylsulfonic acids, for example methane- or p-toluenesulfonic acid. Agrochemically advantageous salts are preferred within the scope of the invention; but it also embraces salts which are disadvantageous for agrochemical purposes, for example salts which are toxic to bees or fish, and which are employed, for example, for isolating or purifying free compounds I or, if appropriate, agrochemically advantageous salts thereof. Since the compounds I in free form and in the form of salts thereof are closely related, the free compounds I or salts thereof hereinabove and hereinafter are, if appropriate, also to be understood analogously as meaning the corresponding salts, or the free compounds I.

Unless otherwise defined, the general terms used hereinabove and hereinafter have the meanings given below.

Unless otherwise defined, carbon-containing groups and compounds contain in each case 1 up to and including 4, in particular 1 or 2, carbon atoms (C atoms).

Aryl R₂ and/or R₃ - as a group per se and as structural element of other groups and compounds such as aryl-C₁-C₄alkyl, aryloxy-C₁-C₄alkyl, aryltio-C₁-C₄alkyl, arylcarbonyl and aryl-C₂-C₆alkenyl groups - is a carbocyclic aromatic group, has in particular 6 to 14 carbon atoms and is, for example naphthyl, phenanthryl or in particular phenyl.

Heteroaryl R₂ and/or R₃ - as a group per se and as structural element of other groups and compounds such as heteroaryl-C₁-C₄alkyl, heteroaryloxy-C₁-C₄alkyl, heteroarylthio-C₁-C₄alkyl, heteroarylcarbonyl and heteroaryl-C₂-C₄alkenyl groups - is a heterocyclic aromatic group, has in particular 5 to 14 ring members of which 1 to 3 members are hetero atoms selected from the group comprising the oxygen, the sulfur and the nitrogen atom, and is, for example, furyl, benzofuryl, thienyl, benzothienyl, pyrrolyl, indolyl, carbazolyl, oxazolyl, thiazolyl, imidazolyl, triazolyl, pyridyl, quinolyl, pyridazinyl, phthalazinyl, pyrimidinyl, quinazolinyl, pyrazinyl, quinoxalinyl or phenanthridinyl. Preferred heteroaryl radicals R₂ and/or R₃ are furyl, benzofuryl, thienyl, benzothienyl, pyrrolyl and thiazolyl and in particular pyridyl.

Substituted aryl R₂ and/or R₃ - as a group per se and as structural element of other groups and compounds such as substituted aryl-C₁-C₄alkyl, aryloxy-C₁-C₄alkyl, arylthio-C₁-C₄alkyl, arylcarbonyl and aryl-C₂-C₄alkenyl groups, is aryl R₂ and/or R₃ which is substituted by one, two or three substituents selected from the group comprising halogen, cyano, cyanomethoxy, nitro, trimethylsilyl, C₁-C₄alkyl, halo-C₁-C₄alkyl, C₁-C₄alkoxy, halo-C₁-C₄alkoxy, C₁-C₄alkoxymethyl, aryloxymethyl, heteroaryloxymethyl, arylmethoxy, heteroarylmethoxy, C₃-C₆cycloalkylmethoxy, C₁-C₄alkylthio, C₁-C₄alkydthiomethyl, arylthiomethyl, heteroarylthiomethyl, C₂-C₄alkenyloxy, C₂-C₄alkylnyloxy, aryloxy, heteroaryloxy, C₁-C₄alkanesulfinylmethyl, C₁-C₄alkanesulfonylmethyl, arylsulfinylmethyl, arylsulfonylmethyl, heteroarylsulfinylmethyl, heteroarylsulfonylmethyl, the groups of the formula N(R₄)R₅ (Ib) and the groups of the formula CH₂N(R₄)R₅ (Ic), where either R₄ and R₅ in each case independently of one another are hydrogen, C₁-C₄alkyl, aryl, aryl-C₁-C₄alkyl or heteroaryl, or R₄ and R₅ together with the nitrogen atom to which R₄ and R₅ are attached form a pyrrolidine, piperidine or morpholine ring which is unsubstituted or substituted by one or two substituents selected from the group comprising C₁-C₄alkyl, and/or substituted aryl R₂ and/or R₃ is aryl R₂ and/or R₃ which is substituted on two adjacent carbon atoms by straight-chain C₃-C₅alkylene which is unsubstituted or has one, two or three substituents selected from the group comprising C₁-C₄alkyl, C₁-C₄alkoxy, halogen, phenyl and oxo, and in which one or two non-adjacent methylene groups can be replaced by oxygen, for example methylenedioxy or ethylenedioxy.

Substituted heteroaryl R₂ and/or R₃ - as a group per se and as structural element of other groups and compounds such as substituted heteroaryl-C₁-C₄alkyl, heteroaryloxy-C₁-C₄alkyl, heteroarylthio-C₁-C₄alkyl, heteroarylcarbonyl and heteroaryl-C₂-C₄alkenyl groups, this heteroaryl R₂ and/or R₃ which is substituted by one, two or three substituents selected from the group comprising halogen, cyano, cyanomethoxy, nitro, trimethylsilyl, C₁-C₄alkyl, halo-C₁-C₄alkyl, C₁-C₄alkoxy, halo-C₁-C₄alkoxy, C₁-C₄alkoxymethyl, aryloxymethyl, heteroaryloxymethyl, arylmethoxy, heteroarylmethoxy, C₃-C₆cycloalkylmethoxy, C₁-C₄alkylthio, C₁-C₄alkylthiomethyl, arylthiomethyl, heteroarylthiomethyl, C₂-C₄alkenyloxy, C₂-C₄alkynyloxy, aryloxy, heteroaryloxy, C₁-C₄alkanesulfinylmethyl, C₁-C₄alkanesulfonylmethyl, arylsulfinylmethyl, arylsulfonylmethyl, heteroarylsulfinylmethyl, heteroarylsulfonylmethyl, the groups of the formula N(R₄)R₅ (Ib) and the groups of the formula CH₂N(R₄)R₅ (Ic), where either R₄ and R₅ in each case independently of one another are hydrogen, C₁-C₄alkyl, aryl, aryl-C₁-C₄alkyl or heteroaryl or R₄ and R₅ together with the nitrogen atom to which R₄ and R₅ are attached form a pyrrolidine, pyridine or morpholine ring which is unsubstituted or substituted by one or two substituents selected from the group comprising C₁-C₄alkyl.

Aryl and heteroaryl as structural elements of substituents defined for substituted aryl and heteroaryl R₂ and/or R₃ such as aryloxymethyl, heteroaryloxymethyl, arylmethoxy, heteroarylmethoxy, arylthiomethyl, heteroarylthiomethyl, aryloxy, heteroaryloxy, arylsulfinylmethyl, arylsulfonylmethyl, heteroarylsulfinylmethyl and heteroarylsulfonylmethyl substituents and of aryl, aryl-C₁-C₄alkyl and heteroaryl groups in substituents Ib and Ic-as a group per se and as structural element of other groups and compounds - are as defined in aryl and heteroaryl R₂ and/or R₃.

An unsaturated carbocyclic or heterocyclic ring of R₂, R₃ and the carbon atom to which R₂ and R₃ are attached contains a single double bond or two or three non-cumulated double bonds, the maximum number of double bonds which is possible from the structural point of view being a function of the size of the ring and the number and nature of the hetero atoms.

An example of a carbocyclic ring of R₂, R₃ and the carbon atom to which R₂ and R₃ are attached and to which a benzene ring can be fused is the cyclobutane, cyclopentane, cyclopentene, cyclohexane, tetrahydronaphthalene, cyclohexene, cycloheptane ring or in particular the indane ring.

An example of a heterocyclic ring of R₂, R₃ and the carbon atom to which R₂ and R₃ are attached and to which a benzene ring can be fused has 1 to 3 hetero atoms selected from the group comprising the oxygen, the sulfur and the nitrogen atom and is the oxetane, pyrrolidine, triazole, piperidine, tetrahydroquinoline, chromane, thiochromane, morpholine, dihydroazepine ring or in particular the 5,6-dihydro-2H-1,4-thiazine ring.

A substituted carbocyclic or heterocyclic ring of R₂, R₃ and the carbon atom to which R₂ and R₃ are attached is substituted by one, two or three substituents selected from the group comprising C₁-C₄alkyl and, if desired, phenyl which is substituted as indicated for substituted aryl R₂ and/or R₃.

A substituted benzene ring which is fused to a carbocyclic or heterocyclic ring of R₂, R₃ and the carbon atom to which R₂ and R₃ are attached is substituted as indicated for substituted aryl R₂ and/or R₃.

Alkyl - as a group per se and as structural element of other groups and compounds such as haloalkyl, alkoxy, haloalkoxy, alkoxymethyl, alkylthio, alkylthiomethyl, alkanesulfinylmethyl, alkanesulfonylmethyl, arylalkyl, heteroarylalkyl, aryloxyalkyl, heteroaryloxyalkyl, arylthioalkyl and heteroarylthioalkyl - is, in each case with due consideration of the number of carbon atoms contained in each individual case of the respective group or compound, either straight-chain, i.e. methyl, ethyl, propyl or butyl, or branched, for example isopropyl, isobutyl, sec-butyl or tert-butyl.

Alkenyl - as a group per se and as structural element of other groups and compounds such as alkenyloxy, arylalkenyl and heteroarylalkenyl - is, in each case with due consideration of the number of carbon atoms contained in each individual case in the respective group or compound, either straight-chain, for example ethenyl, propen-1-yl or but-1-en-1-yl, or branched, for example propen-2-yl or but-1-en-2-yl.

Alkynyl - as a group per se and as structural element of other groups and compounds such as alkynyloxy - is, in each case with due consideration of the number of carbon atoms contained in each individual case, either straight-chain, for example ethynyl, propyn-1-yl or but-1-yn-1-yl, or branched, for example propyn-2-yl or but-1-yn-2-yl.

Cycloalkyl - as a group per se and as structural element for the groups and compounds such as cycloalkylmethoxy - is, in each case with due consideration of the number of carbon atoms contained in each individual case, for example cyclopropyl, cyclobutyl, cyclopentyl or cyclohexyl.

In alkoxy-, alkylthio-, alkanesulfinyl-, alkanesulfonyl-, aryl-, heteroaryl-, aryloxy-, heteroaryloxy-, arylthio-, heteroarylthio-, arylsulfinyl-, heteroarylsulfinyl-, arylsulfonyl-, heteroarylsulfonyl- or cycloalkyl-substituted carbon-containing groups and compounds such as alkoxymethyl, alkylthiomethyl, alkanesulfinylmethyl, alkanesulfonylmethyl, arylalkyl, heteroarylalkyl, arylalkenyl, heteroarylalkenyl, arylmethoxy, heteroarylmethoxy, aryloxymethyl, heteroaryloxymethyl, aryloxyalkyl, heteroaryloxy-alkyl, arylthiomethyl, heteroarylthiomethyl, arylthioalkyl, heteroarylthioalkyl, arylsulfinylmethyl, heteroarylsulfinylmethyl, arylsulfonylmethyl, heteroarylsulfonylmethyl and cycloalkylmethoxy, one of the hydrogen atoms in the unsubstituted basic structure on which these groups and compounds are based is replaced by alkoxy, alkylthio, alkanesulfinyl, alkanesulfonyl, aryl, heteroaryl, aryloxy, heteroaryloxy, arylthio, heteroarylthio, arylsulfinyl, heteroarylsulfinyl, arylsulfonyl, heteroarylsulfonyl or cycloalkyl.

Halogen - as a group per se and as structural element of other groups and compounds such as haloalkyl and haloalkoxy - is fluorine, chlorine or bromine, furthermore iodine.

In halogen-substituted carbon-containing groups and compounds such as haloalkyl and haloalkoxy at least one of the hydrogen atoms in the non-halogenated basic structure on which these groups and compounds are based is replaced by a halogen atom; however, it is also possible for two or more than two, for example - in the case of perhalogenation - all, of the hydrogen atoms in the non-halogenated basic structure on which these groups and compounds are based, to be replaced independently of one another by halogen atoms. Examples which may be mentioned are -CH₂F, -CHF₂, -CF₃, -CH₂Cl, -CHCl₂, -CCl₃, -CCl₂CCl₃, -CH₂Br, -CH₂CH₂Br, -CHBrCl, -OCH₂F, -OCHF₂ and OCF₃.

Preferred within the scope of the invention are
(1) Compounds of the formula I in which:
   X is a nitrogen atom or a group Ia;
   R₁ is C₁-C₄alkyl; and
   either R₂ and R₃ independently of one another are hydrogen, C₁-C₄alkyl, halo-C₁-C₄alkyl, C₁-C₄alkoxy, C₁-C₄alkoxymethyl, C₁-C₄alkylthio, C₁-C₄alkylthiomethyl, C₂-C₄alkenyl, C₂-C₄alkynyl, C₃-C₆cycloalkyl, cyano, an aryl, aryl-C₁-C₄alkyl, aryloxy-C₁-C₄alkyl, arylthio-C₁-C₄alkyl, aryl-C₂-C₄alkenyl or arylcarbonyl group which is unsubstituted or substituted by one, two or three substituents selected from the group comprising halogen, cyano, cyanomethoxy, nitro, trimethylsilyl, C₁-C₄alkyl, halo-C₁-C₄alkyl, C₁-C₄alkoxy, halo-C₁-C₄alkoxy, C₁-C₄alkoxymethyl, aryloxymethyl, heteroaryloxymethyl, arylmethoxy, heteroarylmethoxy, C₃-C₆cycloalkylmethoxy, C₁-C₄alkylthio, C₁-C₄alkylthiomethyl, arylthiomethyl, heteroarylthiomethyl, C₂-C₄alkenyloxy, C₂-C₄alkynyloxy, aryloxy, heteroaryloxy, C₁-C₄alkanesulfinylmethyl, C₁-C₄alkanesulfonylmethyl, arylsulfinylmethyl, arylsulfonylmethyl, heteroarylsulfinylmethyl, heteroarylsulfonylmethyl, the groups Ib and the groups Ic, where either R₄ and R₅ in each case independently of one another are hydrogen, C₁-C₄alkyl, aryl, aryl-C₁-C₄alkyl or heteroaryl, or R₄ and R₅ together with the nitrogen atom to which R₄ and R₅ form attached are a pyrrolidine, piperidine or morpholine ring each of which is unsubstituted or substituted by one or two substituents selected from the group comprising C₁-C₄alkyl, and/or this aryl, aryl-C₁-C₄alkyl, aryloxy-C₁-C₄alkyl, arylthio-C₁-C₄alkyl, aryl-C₂-C₄alkenyl or arylcarbonyl group, is substituted on two adjacent carbon atoms by straight-chain C₃-C₅alkylene which is unsubstituted or has one, two or three substituents selected from the group comprising C₁-C₄alkyl, C₁-C₄alkoxy, halogen, phenyl and oxo and in which one or two non-adjacent methylene groups can be replaced by oxygen, or a heteroaryl, heteroaryl-C₁-C₄alkyl, heteroaryloxy-C₁-C₄alkyl, heteroarylthio-C₁-C₄alkyl, heteroaryl-C₂-C₄alkenyl or heteroarylcarbonyl group, each of which is unsubstituted or substituted by one, two or three substituents selected from the group comprising halogen, cyano, cyanomethoxy, nitro, trimethylsilyl, C₁-C₄alkyl, halo-C₁-C₄alkyl, C₁-C₄alkoxy, halo-C₁-C₄alkoxy, C₁-C₄alkoxymethyl, aryloxymethyl, heteroaryloxymethyl, arylmethoxy, heteroarylmethoxy, C₃-C₆cycloalkylmethoxy, C₁-C₄alkylthio, C₁-C₄alkylthiomethyl, arylthiomethyl, heteroarylthiomethyl, C₂-C₄alkenyloxy, C₂-C₄alkynyloxy, aryloxy, heteroaryloxy, C₁-C₄akanesulfinylmethyl, C₁-C₄alkanesulfonylmethyl, arylsulfinylmethyl, arylsulfonylmethyl, heteroarylsulfinylmethyl, heteroarylsulfonylmethyl, the groups Ib and the groups Ic, where either R₄ and R₅ in each case independently of one another are hydrogen, C₁-C₄alkyl, aryl, aryl-C₁-C₄alkyl or heteroaryl, or R₄ and R₅ together with the nitrogen atom to which R₄ and R₅ are attached form a pyrrolidine, piperidine or morpholine ring, each of which is unsubstituted or substituted by one or two substituents selected from the group comprising C₁-C₄alkyl,
   or R₂ and R₃ together with the carbon atom to which R₂ and R₃ are attached form a ring which is unsubstituted or substituted by one, two or three substituents selected from the group comprising C₁-C₄alkyl, phenyl and phenyl which is substituted by one, two or three substituents selected from the group comprising halogen, cyano, cyanomethoxy, nitro, trimethylsilyl, C₁-C₄alkyl, halo-C₁-C₄alkyl, C₁-C₄alkoxy, halo-C₁-C₄alkoxy, C₁-C₄alkoxymethyl, aryloxymethyl, heteroaryloxymethyl, arylmethoxy, heteroarylmethoxy, C₃-C₆cycloalkylmethoxy, C₁-C₄alkylthio, C₁-C₄alkylthiomethyl, arylthiomethyl, heteroarylthiomethyl, C₂-C₄alkenyloxy, C₂-C₄alkynyloxy, aryloxy, heteroaryloxy, C₁-C₄alkanesulfinylmethyl, C₁-C₄alkanesulfonylmethyl, arylsulfinylmethyl, arylsulfonylmethyl, heteroarylsulfinylmethyl, heteroarylsulfonylmethyl, the groups Ib and the groups Ic, where either R₄ and R₅ in each case independently of one another are hydrogen, C₁-C₄alkyl, aryl, aryl-C₁-C₄alkyl or heteroaryl, or R₄ and R₅ together with the nitrogen atom to which R₄ and R₅ are attached form a pyrrolidine, piperidine or morpholine ring, each of which is unsubstituted or substituted by one or two substituents selected from the group comprising C₁-C₄alkyl, and/or this phenyl is substituted on two adjacent carbon atoms by a straight-chain C₃-C₅alkylene which is unsubstituted or has one, two or three substituents selected from the group comprising C₁-C₄alkyl, C₁-C₄alkoxy, halogen, phenyl and oxo, and in which one or two non-adjacent methylene groups can be replaced by oxygen, this ring being saturated or having a single double bond or two or three non-cumulative double bonds, where the maximum number of double bonds which is possible from the structural point of view is a function of the size of the ring and the number and nature of the hetero atoms, and this ring being a 4- to 7-membered carbocylic ring or heterocyclic ring having 1 to 3 hetero atoms selected from the group comprising the oxygen, the sulfur and the nitrogen atom, and to which ring a benzene ring can be fused which is unsubstituted or substituted by one, two or three substituents selected from the group comprising halogen, cyano, cyanomethoxy, nitro, trimethylsilyl, C₁-C₄alkyl halo-C₁-C₄alkyl, C₁-C₄alkoxy, halo-C₁-C₄alkoxy, C₁-C₄alkoxymethyl, aryloxymethyl, heteroaryloxymethyl, arylmethoxy, heteroarylmethoxy, C₃-C₆cycloalkylmethoxy, C₁-C₄alkylthio, C₁-C₄alkylthiomethyl, arylthiomethyl, heteroarylthiomethyl, C₂-C₄alkenyloxy, C₂-C₄alkynyloxy, aryloxy, heteroaryloxy, C₁-C₄alkanesulfinylmethyl, C₁-C₄alkanesulfonylmethyl, arylsulfinylmethyl, arylsulfonylmethyl, heteroarylsulfinylmethyl, heteroarylsulfonylmethyl, the groups Ib and the groups Ic, where either R₄ and R₅ in each case independently of one another are hydrogen, C₁-C₄alkyl, aryl, aryl-C₁-C₄alkyl or heteroaryl, or R₄ and R₅ together with the nitrogen atom to which R₄ and R₅ are attached form a pyrrolidine, piperidine or morpholine ring, each of which is unsubstituted or substituted by one or two substituents selected from the group comprising C₁-C₄alkyl, and/or this benzene ring is substituted on two adjacent carbon atoms by straight-chain C₃-C₅alkylene which is unsubstituted or has one, two or three substituents selected from the group comprising C₁-C₄alkyl, C₁-C₄alkoxy, halogen, phenyl and oxo, and in which one or two non-adjacent methylene groups can be replaced by oxygen,
   where "aryl" - as a group per se and as structural element of other groups - in each case independently of one another - is a carboxcyclic aromatic group having 6 to 14 carbon atoms, and "heteroaryl" - as a group per se and as structural element of other groups - in each case independently of one another is a heterocyclic aromatic group having 5 to 14 ring members of which 1 to 3 members are hetero atoms selected from the group comprising the oxygen, the sulfur and the nitrogen atom; in free form or, if appropriate, in salt form;
(2) compounds of the formula I in which:

   X is a nitrogen atom or a group Ia;
   R₁ is C₁-C₄alkyl; and
   either R₂ and R₃ independently of one another are hydrogen, C₁-C₄alkyl, halo-C₁-C₄alkyl, C₁-C₄alkoxymethyl, C₁-C₄alkylthio, C₁-C₄alkylthiomethyl, C₃-C₆cycloalkyl, cyano, or a benzyl, phenyl or naphthyl group, each of which is unsubstituted or substituted by one, two or three substituents selected from the group comprising halogen, nitro, C₁-C₄alkyl, halo-C₁-C₄alkyl, C₁-C₄alkoxy, halo-C₁-C₄alkoxy, C₃-C₆cycloalkylmethoxy, cyano, cyanomethoxy, C₁-C₄alkylthio, C₁-C₄alkylthiomethyl, pyrimidinylthiomethyl, C₂-C₄alkenyloxy, C₂-C₄alkynyloxy, phenoxy and C₁-C₄alkanesulfinylmethyl, or each of which is substituted on two adjacent carbon atoms by straight-chain C₁-C₃alkylenedioxy, or are a pyridyl, furyl, benzofuryl, thienyl, benzothienyl, pyrrolyl or thiazolyl group, each of which is unsubstituted or substituted by one, two or three substituents selected from the group comprising halogen and C₁-C₄alkyl,
   or R₂ and R₃ together with the carbon atom to which R₂ and R₃ are attached are a cyclopentane or 5,6-dihydro-2H-1,4-thiazine ring, each of which is unsubstituted or substituted by one, two or three substituents selected from the group comprising phenyl which is substituted by one, two or three substituents selected from the group comprising halogen and C₁-C₄alkoxy, to which cyclopentane or 5,6-dihydro-2H-1,4-thiazine ring an unsubstituted benzene ring can be fused; in free form or, if appropriate, in salt form;
(3) compounds of the formula I in which:
   R₁ is C₁-C₄alkyl; and either
   (a) X is a nitrogen atom; and
   R₂ and R₃ independently of one another are C₁-C₄alkyl, C₃-C₆cycloalkyl or a phenyl or naphthyl group, each of which is unsubstituted or substituted by one, two or three substituents selected from the group comprising C₁-C₄alkoxy, halogen, halo-C₁-C₄alkyl, halo-C₁-C₄alkoxy, C₃-C₆cycloalkylmethoxy, C₂-C₄alkenyloxy and C₂-C₄alkynyloxy, or this phenyl or naphthyl group is substituted on two adjacent carbon atoms by straight-chain C₁-C₃alkylenedioxy, or are an unsubstituted pyridyl or benzofuryl group; or
   (b) X is a group Ia; and
   either R₂ and R₃ independently of one another are hydrogen, C₁-C₄alkyl, halo-C₁-C₄alkyl, C₁-C₄alkoxymethyl, C₁-C₄alkylthio, C₁-C₄alkylthiomethyl, C₃-C₆cycloalkyl, cyano, or a benzyl, phenyl or naphthyl group which is unsubstituted or substituted by one, two or three substituents selected from the group comprising halogen, nitro, C₁-C₄alkyl, halo-C₁-C₄alkyl, C₁-C₄alkoxy, halo-C₁-C₄alkoxy, C₃-C₆cycloalkylmethoxy, cyano, cyanomethoxy, C₁-C₄alkylthio, C₁-C₄alkylthiomethyl, pyrimidinylthiomethyl, C₂-C₄alkenyloxy, C₂-C₄alkynyloxy, phenoxy and C₁-C₄alkanesulfinylmethyl, or this benzyl, phenyl or naphthyl group is substituted on two adjacent carbon atoms by straight-chain C₁-C₃alkylenedioxy, or are a pyridyl, furyl, benzofuryl, thienyl, benzothienyl, pyrrolyl or thiazolyl group, each of which is unsubstituted or substituted by one, two or three substituents selected from the group comprising halogen and C₁-C₄alkyl, or R₂ and R₃ together with the carbon atom to which R₂ and R₃ are attached are a cyclopentane or 5,6-dihydro-2H-1,4-thiazine ring which is unsubstituted or substituted by one, two or three substituents selected from the group comprising phenyl which is substituted by one, two or three substituents selected from the group comprising halogen and C₁-C₄alkoxy, to which cyclopentane or 5,6-dihydro-2H-1,4-thiazine ring an unsubstituted benzene ring can be fused; in free form or, if appropriate, in salt form;
(4) compounds of the formula I in which:
   R₁ is C₁-C₄alkyl; and either
   (c) X is a nitrogen atom ;
   R₂ is a phenyl group which is unsubstituted or substituted by one or two substituents selected from the group comprising C₁-C₄alkoxy, C₂-C₄alkenyloxy, C₂-C₄alkynyloxy, C₃-C₄cycloalkylmethoxy, halogen, halo-C₁-C₄alkyl having 1 up to and including 3 halogen atoms and halo-C₁-C₄alkoxy having 1 up to and including 3 halogen atoms, or this phenyl group is substituted on two adjacent carbon atoms by straight-chain C₁-C₃alkylenedioxy, or R₂ is an unsubstituted naphth-2-yl, benzo[b]fur-2-yl or pyrid-2-yl group; and
   R₃ is C₁-C₄alkyl or C₃-C₄cycloalkyl; or
   (d) X is a group Ia; and
   either R₂ is C₁-C₄alkylthio, a phenyl group which is unsubstituted or substituted by one or two substituents selected from the group comprising halogen, C₁-C₄alkyl, halo-C₁-C₄alkyl having 1 up to and including 3 halogen atoms, C₁-C₄alkoxy, halo-C₁-C₄alkoxy having 1 up to and including 3 halogen atoms, C₃-C₄cycloalkylmethoxy, C₁-C₄alkylthio, C₁-C₄alkylthiomethyl, C₂-C₄alkenyloxy, C₂-C₄alkynyloxy, cyano, cyanomethoxy, nitro, phenoxy, pyrimidinylthiomethyl and C₁-C₄alkanesulfinylmethyl, or which is substituted on two adjacent carbon atoms by straight-chain C₁-C₃alkylenedioxy, or R₂ is a thien-2-yl, pyrid-2-yl or pyrid-3-yl group which is unsubstituted or monosubstituted by halogen, or a pyrrol-2-yl group which is unsubstituted or monosubstituted by C₁-C₄alkyl, or an unsubstituted naphth-2-yl, fur-2-yl, benzo[b]fur-2-yl or thiazol-2-yl group;
   and R₃ is hydrogen, C₁-C₄alkyl, halo-C₁-C₄alkyl having 1 up to and including 3 halogen atoms, C₁-C₄alkoxymethyl, C₁-C₄alkylthiomethyl, cyano or C₃-C₄cycloalkyl;
   or R₂ and R₃ together with the carbon atom to which R₂ and R₃ are attached are an unsubstituted indane ring whose carbon atom in the 1-position is part of the N=C double bond shown in formula I, or are a 5,6-dihydro-2H-1,4-thiazine ring which is unsubstituted or monosubstituted by monohalophenyl or mono-C₁-C₄alkoxyphenyl and whose carbon atom in the 2-position is part of the N=C double bond shown in formula I; in free form or, if appropriate, in salt form;
(5) compounds of the formula I in which:
   R₁ is C₁-C₄alkyl; and either
   (e) X is a nitrogen atom;
   R₂ is unsubstituted phenyl, 3- or 4-halophenyl, 3-halo-C₁-C₄alkylphenyl having 1 up to and including 3 halogen atoms, 2-halo-5-halo-C₁-C₄alkylphenyl having 1 up to and including 3 halogen atoms in the haloalkyl substituent, 3-C₁-C₄alkoxyphenyl, 3-halo-C₁-C₄alkoxyphenyl having 1 up to and including 3 halogen atoms, 3-C₂-C₄alkenyloxyphenyl, 3-C₂-C₄alkynyloxyphenyl, 3-C₃-C₄cycloalkylmethoxyphenyl, phenyl which is substituted in the 3- and 4-positions by straight-chain C₁-C₃alkylenedioxy, or an unsubstituted naphth-2-yl, benzo[b]fur-2-yl or pyrid-2-yl group; and
   R₃ is C₁-C₄alkyl or C₃-C₄cycloalkyl; or
   (f) X is a group Ia; and
   either R₂ is C₁-C₄alkylthio, unsubstituted phenyl, 2-, 3- or 4-halophenyl, 3,4-dihalophenyl in which the two halogen substituents are identical or different, 3- or 4-C₁-C₄alkylphenyl, 3-C₁-C₄alkyl-4-halophenyl, 2- or 3-halo-C₁-C₄alkylphenyl having 1 up to and including 3 halogen atoms, 3,5-bis(halo-C₁-C₄alkyl)phenyl having 1 up to and including 3 halogen atoms, 4-halo-3-halo-C₁-C₄alkylphenyl having 1 up to and including halogen atoms in the haloalkyl substituent, 2-halo-5-halo-C₁-C₄alkylphenyl having 1 up to and including 3 halogen atoms in the haloalkyl substituent, 3-C₁-C₄alkoxyphenyl, 3-halo-C₁-C₄alkoxyphenyl having 1 up to and including 3 halogen atoms, 3-C₃-C₄cycloalkylmethoxyphenyl, 2-C₁-C₄alkylthio-5-halo-C₁-C₄alkylphenyl having 1 up to and including 3 halogen atoms in the haloalkyl substituent, 4-C₁-C₄alkoxy-3-C₁-C₄alkylthiomethylphenyl, 3-C₂-C₄alkenyloxyphenyl, 3-C₂-C₄alkynyloxyphenyl, 4-phenoxyphenyl, 3- or 4-nitrophenyl, 3-cyanophenyl, 3-cyanomethoxyphenyl, 4-C₁-C₄alkoxy-3-nitrophenyl, 3-C₁-C₄alkanesulfinylmethyl-4-C₁-C₄alkoxyphenyl, 4-C₁-C₄alkoxy-3-pyrimidin-2-ylthiomethylphenyl, phenyl which is substituted in the 3-and 4-positions by straight-chain C₁-C₃alkylenedioxy, or an unsubstituted thien-2-yl, pyrid-2-yl or pyrid-3-yl, 6-halopyrid-2-yl, 5-halothien-2-yl, 1-C₁-C₄alkylpyrrol-2-yl or an unsubstituted naphth-2-yl, fur-2-yl, benzo[b]fur-2-yl or thiazol-2-yl group; and R₃ is hydrogen, C₁-C₄alkyl, halo-C₁-C₄alkyl having 1 up to and including 3 halogen atoms, C₁-C₄alkoxymethyl, C₁-C₄alkylthiomethyl, cyano or C₃-C₄cycloalkyl; or R₂ and R₃ together with the carbon atom to which R₂ and R₃ are attached are an unsubstituted indane ring whose carbon atom in the 1-position is part of the N=C double bond shown in formula I, or a 5,6-dihydro-2H-1,4-thiazine ring which is monosubstituted by 4-halophenyl or 4-C₁-C₄alkoxyphenyl and whose carbon atom in the 2-position is part of the N=C double bond shown in formula I; in free form or, if appropriate, in salt form;
(6) compounds of the formula I in which:
   X is a nitrogen atom;
   R₁ is C₁-C₂alkyl;
   R₂ is 3-fluorophenyl, 3-trifluoromethylphenyl, 3-trifluoromethoxyphenyl or unsubstituted pyrid-2-yl; and
   R₃ is C₁-C₂alkyl; in free form or, if appropriate, in salt form;
(7) compounds of the formula I in which:
   X is a nitrogen atom;
   R₁ is C₁-C₂alkyl;
   R₂ is 3-chlorophenyl, 4-chlorophenyl, 3-prop-1-yn-3-yloxyphenyl or 4-prop-1-yn-3-yloxyphenyl;
   R₃ is C₁-C₂alkyl; in free form or, if appropriate, in salt form;
(8) compounds of the formula I in which:
   X is a group Ia;
   R₁ is C₁-C₂alkyl;
   R₂ is unsubstituted phenyl, 3-fluorophenyl, 3-chlorophenyl, 3-bromophenyl, 3-trifluoromethylphenyl, 3-cyclopropylmethoxyphenyl, 3-allyloxyphenyl, 3,4-ethylenedioxyphenyl or unsubstituted pyrid-2-yl; and
   R₃ is C₁-C₂alkyl; in free form or, if appropriate, in salt form.

Particularly preferred compounds of the formula I within the scope of the invention are those mentioned in Examples H-1 to H-3.3, in free form or, if appropriate, in salt form.

Compounds of the formula I which are individually preferred within the scope of the invention are the following:
methyl 2-[3-(3-aza-2-oxa-4-phenylpent-3-en-1-yl)benzo[b]thien-2-yl]-3-methoxyacrylate (Example H-1);
methyl 2-{3-[3-aza-2-oxa-4-(3-trifluoromethylphenyl)pent-3-en-1-yl]benzo[b]thien-2-yl}-3-methoxyacrylate (Example H-2.1);
methyl 2-{3-[3-aza-4-(3-bromophenyl)-2-oxapent-3-en-1-yl]benzo[b]thien-2-yl}-3-methoxyacrylate (Example H-2.2);
methyl 2-{3-(3-aza-4-[3,4-ethylenedioxyphenyl)-2-oxapent-3-en-1-yl]benzo[b]thien-2-yl}3-methoxyacrylate (Example H-2.5);
methyl 2-[3-(3-aza-2-oxa-4-pyrid-2-ylpent-3-en-1-yl)benzo[b]thien-2-yl]-3-methoxyacrylate (Example H-2.7);
methyl 2-{3-[3-aza-4-(3-fluorophenyl)-2-oxapent-3-en-1-yl]benzo[b]thien-2-yl}-3-methoxyacrylate (Example H-2.9);
methyl 2-{3-[3-aza-4-(3-chlorophenyl)-2-oxapent-3-en-1-yl]benzo[b]thien-2-yl}-3-methoxyacrylate (Example H-2.10);
methyl 2-{3-[3-aza-2-oxa-4-(3-prop-1-en-3-yloxyphenyl)pent-3-en-1-yl]benzo[b]thien-2-yl}-3-methoxyacrylate (Example H-2.12);
methyl 2-{3-[3-aza-4-(3-cyclopropylmethoxyphenyl)-2-oxapent-3-en-1-yl]benzo[b]thien-2-yl}-3-methoxyacrylate (Example H-2.13);
methyl 2-{3-[3-aza-2-oxa-4-(3-trifluoromethylphenyl)pent-3-en-1-yl]benzo[b]thien-2-yl}-2-methoxyiminoacetate (Example H-2.15);
methyl 2-{3-[3-aza-2-oxa-4-(3-trifluoromethoxyphenyl)pent-3-en-1-yl]benzo[b]thien-2-yl}-2-methoxyiminoacetate (Example H-2.16);
methyl 2-{3-[3-aza-4-(3-fluorophenyl)-2-oxapent-3-en-1-yl]benzo[b]thien-2-yl}-2-methoxyiminoacetate (Example H-2.19);
methyl 2-[3-(3-aza-2-oxa-4-pyrid-2-ylpent-3-en-1-yl)benzo[b]thien-2-yl]-2-methoxyiminoacetate (Example H-2.20);
methyl 2-{3-[3-aza-2-oxa-4-(3-prop-1-in-3-yloxyphenyl)pent-3-en-1-yl]benzo[b]thien-2-yl}-2-methoxyiminoacetate (Example H-2.65) ;
methyl 2-{3-[3-aza-4-(4-chlorophenyl)-2-oxapent-3-en-1-yl]benzo[b]thien-2-yl}-2-methoxyiminoacetate (Example H-2.95),
methyl 2-{3-[3-aza-4-(4-methylphenyl)-2-oxapent-3-en-1-yl]benzo[b]thien-2-yl}-3-methoxyacrylate (Example H-2.103);
in each case in free form or, if appropriate, in salt form.

The invention furthermore relates to the process for the preparation of the compounds of the formula I, in free form or, if appropriate, in salt form, which comprises, for example, reacting a compound of the formula 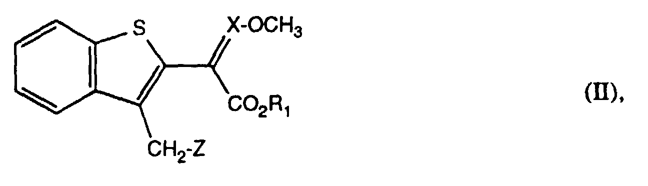 in which R₁ and X are as defined in formula I and Z is an easily detachable nucleofugic radical, in particular bromine, with a compound of the formula

HO-N=C(R₂)(R₃) (III),

in which R₂ and R₃ are as defined in formula I, or, if appropriate, with a salt thereof, preferably in the presence of a base, in the presence or absence of an inert solvent or diluent, and/or converting a compound of the formula I which can be obtained by the process or by another route, in free form or, if appropriate, in salt form, into a different compound of the formula I, and/or resolving an isomer mixture which can be obtained according to the process and isolating the desired isomer, and/or, if desired, converting a free compound of the formula I which can be obtained according to the process into a salt, or converting a salt of a compound of the formula I which can be obtained according to the process into the free compound of the formula I or, if appropriate, into a different salt

What has been said above about salts of compounds of the formula I applies analogously to starting materials listed hereinbefore and hereinafter with regard to the salts thereof.

The reactions described hereinbefore and hereinafter are carried out in a manner known per se, for example in the absence or, customarily, in the presence of a suitable solvent or diluent or a mixture of these, the reaction being carried out, as required, with cooling, at room temperature or with heating, for example in a temperature range from approx. -80°C to the boiling point of the reaction medium, preferably from approx. -20°C to approx. +150°C, and, if required, in a sealed container, under pressure, under an inert gas atmosphere and/or under anhydrous conditions. Detailed reaction conditions can be found in the examples.

The starting material mentioned hereinbefore and hereinafter which is used for preparing the compounds I in free form or, if appropriate, salt form, is known or can be prepared by methods known per se, for example by the instructions hereinafter.

Suitable as radicals Z are, for example: fluorine, chlorine, bromine, iodine, C₁-C₄alkylthio such as methylthio, ethylthio or propylthio, C₁-C₄alkanoyloxy such as acetoxy, (halo-)C₁-C₄alkanesulfonyloxy such as methanesulfonyloxy, ethanesulfonyloxy or trifluoromethanesulfonyloxy, or unsubstituted or substituted phenylsulfonyloxy such as benzenesulfonyloxy or p-toluenesulfonyloxy. A particularly preferred radical Z is bromine.

Suitable bases for facilitating the elimination of HZ are, for example, hydroxides, hydrides, amides, alkanolates, carbonates, dialkylamides or alkylsilylamides of alkali metals or alkaline earth metals, or alkylamines, alkylenediamines, free or N-alkylated, saturated or unsaturated cycloalkylamines, basic heterocycles, ammonium hydroxides as well as carbocyclic amines. Examples which may be mentioned are sodium hydroxide, sodium hydride, sodium amide, sodium methanolate, sodium carbonate, potassium tert-butanolate, potassium carbonate, lithium diisopropylamide, potassium bis(trimethylsilyl)amide, calcium hydride, triethylamine, triethylenediamine, cyclohexylamine, N-cyclohexyl-N,N-dimethylamine, N,N-diethylaniline, pyridine, 4-(N,N-dimethylamino)pyridine, N-methylmorpholine, benzyltrimethylammonium hydroxide as well as 1,5-diazabicyclo[5.4.0]undec-5-ene (DBU).

The reactants can be reacted with each other in pure form, i.e. without an addition of a solvent or diluent, for example in the melt. In most cases, however, the addition of an inert solvent or diluent, or a mixture of these, is advantageous. Examples of such solvents or diluents which may be mentioned are: aromatic, aliphatic and alicyclic hydrocarbons and halohydrocarbons such as benzene, toluene, xylene, chlorobenzene, bromobenzene, petroleum ether, hexane, cyclohexane, dichloromethane, trichloromethane, dichloroethane or trichloroethene; ethers such as diethyl ether, tert-butyl methyl ether, tetrahydrofuran or dioxane; ketones such as acetone or methyl ethyl ketone; alcohols such as methanol, ethanol, propanol, butanol, ethylene glycol or glycerol; amides such as N,N-dimethylformamide, N,N-dimethylacetamide, N-methylpyrrolidone or hexamethylphosphoric triamide; nitriles such as acetonitrile; and sulfoxides, such as dimethyl sulfoxide. Bases which are employed in excess, such as triethylamine, pyridine, N-methylmorpholine or N,N-diethylaniline, can also serve as solvents or diluents.

In a preferred embodiment of the process, a compound II is reacted with a compound III in which Z is bromine at room temperature, in an amide, preferably in N,N-dimethylformamide, and in the presence of a hydride, preferably in the presence of sodium hydride.

The compounds II are known or can be prepared analogously to known processes; such information can be found, for example, in the European Patent Application with the publication number 0 243 014.

The compounds III, in free form or, if appropriate, in salt form, are known or can be prepared analogously to known compounds.

A compound I which can be obtained by the process or by another route can be converted into a different compound I in a manner known per se, by replacing one or more substituents of the starting compound I in the customary manner by (a) different substituent(s) according to the invention.

For example,
- non-halogenated aromatic or heteroaromatic partial ring structures can be halogenated to give halogenated aromatic or heteroaromatic partial ring structures according to the invention; or
- halogen substituents in halogenated aromatic or heteroaromatic partial ring structures can be replaced by alkylthio, alkoxy, haloalkoxy, arylmethoxy, heteroarylmethoxy, alkenyloxy, alkynyloxy, cyano, cyanomethoxy, aryloxy, heteroaryloxy or cycloalkylmethoxy substituents according to the invention.

Depending on which reaction conditions and starting materials which are suitable therefor in each case are selected, it is possible to replace, in one reaction step, only one substituent by a different substituent according to the invention, or it is possible to replace, in the same reaction step, a plurality of substituents by different substituents according to the invention. For example, it is possible to introduce at the same time two or more identical halogen substituents into the same, or, if present, into a variety of (hetero)aromatic rings. Equally, it is possible, for example, additionally to substitute a (hetero)aromatic ring-within the scope of the definitions of variables according to the invention - which is already substituted, for example monosubstituted, by a certain substituent, for example chlorine, by one or more further identical substituents, for example chlorinated, i.e., for example, converted into the disubstituted or even higher substituted, for example dichlorinated or higher-chlorinated, state.

Salts of compounds I can be prepared in a manner known per se. For example, acid addition salts of compounds I are obtained by treatment with a suitable acid or suitable ion exchanger reagent.

Salts of compounds I can be converted into the free compounds I in the customary manner, acid addition salts for example by treatment with a suitable basic agent or a suitable ion exchanger reagent.

Salts of compounds I can be converted into different salts of compounds I in the manner known per se, acid addition salts for example into different acid addition salts, for example by treatment of a salt of an inorganic acid such as a hydrochloride with a suitable metal salt such as a sodium salt, barium salt or silver salt, of an acid, for example with silver acetate, in a suitable solvent in which an inorganic salt which forms, for example silver chloride, is insoluble and so precipitates from the reaction mixture.

Depending on the procedure and the reaction conditions, the compounds I which have salt-forming properties, can be obtained in free form or in the form of salts.

Some of the compounds I, in free form or, if appropriate, in salt form, can exist in the form of one of the isomers which are possible, or as a mixture thereof, for example depending on number and absolute and relative configuration of the asymmetric carbon atoms in the form of pure isomers, such as antipodes and/or diastereomers, or as isomer mixtures such as enantiomer mixtures, for example racemates, diastereomer mixtures or racemate mixtures; atropisomerism, in particular, can also occur. The invention relates to the pure isomers as well as all isomer mixtures which are possible, and is hereinbefore and hereinafter to be understood analogously, even when stereochemical details are not mentioned specifically in each case.

Diastereomer mixtures and racemate mixtures of compounds I, in free form or, if appropriate, in salt form, which can be obtained by the process - as a function of the choice of starting materials and procedures - or by other routes can be separated on the basis of the physicochemical differences of the components in a known manner to give the pure diastereomers or racemates, for example by fractional crystallisation, distillation and/or chromatography.

Enantiomer mixtures which are obtainable accordingly, such as racemates, can be resolved by known methods to give the optical antipodes, for example by recrystallisation from an optically active solvent, by chromatography on chiral adsorbents, for example high pressure liquid chromatography (HPLC) on acetylcellulose, with the aid of suitable microorganisms, by cleavage with specific, immobilised enzymes, via the formation of inclusion compounds, for example using chiral crown ethers, where only one enantiomer is complexed, or by conversion into diastereomeric salts, for example by reacting a basic end substance racemate with an optically active acid such as carboxylic acid, for example camphoric acid, tartaric acid or mallic acid, or sulfonic acid, for example camphorsulfonic acid, and separation of the resulting diastereomer mixture in this way, for example on the basis of their differing solubilities by fractional crystallisation, to give the diastereomers, from which the desired enantiomer can be liberated by the action of suitable agents, for example basic agents.

Pure diastereomers or enantiomers can be obtained according to the invention not only by separation of suitable isomer mixtures, but also by generally known methods of diastereoselective, or enantioselective, synthesis, for example by carrying out the process according to the invention with educts having a correspondingly suitable stereochemistry.

If the individual components differ in their biological activity, it is advantageous to isolate, or synthesize, in each case the biologically more active isomer, for example enantiomer, or isomer mixture, for example enantiomer mixture.

The compounds I, in free form or, if appropriate, in salt form, can also be obtained in the form of their hydrates and/or include other solvents, for example those which may have been used for the crystallisation of compounds in solid form.

The invention relates to all those embodiments of the process in which a compound is used which can be obtained in any stage of the process as starting material or intermediate, and all, or some, of the missing steps are carried out, or a starting material is used in the form of a derivative, or salt and/or the racemates and antipodes thereof is used or, in particular, formed under the reaction conditions.

It is preferred to use those starting substances and intermediates in the process of the present invention, in each case in free form or, if appropriate, in salt form, which lead to the compounds I or, if appropriate, salts thereof, which have been described at the outset as particularly valuable.

In particular, the invention relates to the preparation processes described in Examples H-1 to H-3.3.

The invention also relates to the starting materials and intermediates, each in the free form or in the form of salts, which are used according to the invention for the preparation of the compounds I and/or, if appropriate, their salts and which are novel, and to their use and to processes for their preparations.

Derivatives of the 3-methoxyacrylates and the 2-methoxyiminoacetates which have fungicidal action are known, for example, from EP-A-433 233, EP-A-243 014 and EP-A-472300. Phenyl derivatives which possess an aldimino or ketiminooxymethyl group in addition are known from EP-A-414 153. The compounds I of the present invention, in free form or, if appropriate, in salt form, can be employed preventively and/or curatively in the agricultural sector and in related fields as active ingredients in the control of plant-injurious microorganisms. Compared with the closest compounds of the prior art, EP-A-433,233, which do not always meet the demands of fungicides, the compounds of formula I of the present invention exhibit significantly longer lasting efficacy. Even at low application concentrations, the active ingredients of the formula I according to the invention are distinguished not only by an outstanding action but also by being well tolerated by plants.

The active ingredients of the formula I according to the invention have a biocidal spectrum for controlling phytopathogenic microorganisms, in particular fungi, which is very favourable for practical requirements. They have very advantageous properties, in particular systemic properties, and can be used for the protection of a large number of crop plants. The pests which occur on plants or parts of plants (fruits, flowers, foliage, stalks, tubors, roots) of a variety of crops of useful plants can be held at bay or destroyed using the active ingredients of the formula I, and even parts of plants which grow at a later point in time are protected against phytopathogenic microorganisms.

Compounds I are active, for example, against the phytopathogenic fungi which belong to the following classes: Fungi imperfecti (for example Botrytis, Pyricularia, Helminthosporium, fusarium, Septoria, Cercospora and Alternaria) and Basidiomycetes (for example Rhizoctonia, Hemileia, Puccinia). Moreover, they are active against the classes of the Ascomycetes (for example Venturia and Erysiphe, Podosphaera, Monilinia, Uncinula) and of the Oomycetes (for example Phytophthora, Pythium, Plasmopara).

Furthermore, the compounds I can be employed as seed-dressing agents for the treatment of seed and other plant propagation material (fruits, tubors, grains, branches, roots) and of plant cuttings as a protection against fungal infections and against soil-borne phytopathogenic fungi.

The invention also relates to the compositions which comprise compounds I as active ingredient, in particular crop-protecting compositions, and to their use in the agricultural sector and in related fields. In addition, the invention also embraces the preparation of these compositions, which comprises intimately mixing the active substance with one or more of the substances, or substance groups, described hereinafter. The invention also includes a method for the treatment of plants, which is distinguished by application of the novel compounds I, or of the novel compositions.

Examples of target crops for the use in crop protection are, within the scope of the invention, the following plant species: cereals (wheat, barley, rye, oats, rice, maize, sorghum and related species); beet (sugar beet and fodder beet); pomaceous fruit, stone fruit and soft fruit (apples, pears, plums, peaches, almonds, cherries, strawberries, raspberries and blackberries); pulses (beans, lentils, peas, soybeans); oil crops (oilseed rape, mustard, poppy, olives, sunflowers, coconut, castor-oil plant, cacao, groundnuts); cucurbits (pumpkin, cucumbers, melons); fibre plants (cotton, flax, hemp, jute); citrus fruit (oranges, lemons, grapefruit, tangerines); vegetables (spinach, lettuce, asparagus, cabbage species, carrots, onions, tomatoes, potatoes, bell peppers); Lauraceae (avocado, cinnamonium, camphor) and plants such as tobacco, nuts, coffee, eggplants, sugar cane, tea, pepper, vines, hops, Musaceae and latex plants, as well as ornamental plants.

Active ingredients I are customarily used in the form of compositions and can be applied to the area or plant to be treated simultaneously, or in succession, with other active ingredients. These other active ingredients can be, for example, fertilisers, trace element promoters or other preparations which have an effect on the growth of the plants. However, selective herbicides as well as insecticides, fungicides, bactericides, nematicides, molluscicides or mixtures of a plurality of these preparations, if appropriate together with other carriers, surfactants or other application-enhancing additives, conventionally used in the art of formulation, can also be used.

Suitable auxiliaries such as carriers and additives can be solid or liquid and are substances expedient in the art of formulation, for example natural or regenerated mineral substances, solvents, dispersants, wetting agents, adhesives, thickeners, binders or fertilisers.

A preferred method for applying an active ingredient of the formula I, or an agrochemical composition comprising at least one of these active ingredients, is applying it to the foliage (foliar application). The frequency and rate of application depend on the risk of infestation with the pathogen in question. Alternatively, the active ingredients I can reach the plant via the soil through the root system (systemic action), by drenching the locus of the plant with a liquid preparation or incorporating the substances into the soil in solid form, for example in the form of granules (soil application). In the case of paddy rice, such granules can be metered into the flooded paddy field. Alternatively, the compounds II can be applied to seeds and other plant propagation material (coating) either by soaking the plant propagation material in a liquid preparation of the active ingredient or by coating them with a solid preparation.

The compounds of the formula I are employed as pure active ingredients or, preferably, together with auxiliaries conventionally used in the art of formulation. To this end, they are processed in a known manner to give, expediently, for example emulsion concentrates, spreadable pastes, directly sprayable or dilutable solutions, dilute emulsions, wettable powders, soluble powders, dusts or granules, for example by encapsulation in, for example, polymeric substances. The application methods such as spraying, atomising, dusting, scattering, painting on or pouring as well as the nature of the compositions are selected to suit the intended aims and the prevailing circumstances.

Advantageous application rates are generally 5 g to 2 kg of active ingredient (a.i). per hectare (ha), preferably 10 g to 1 kg of a.i./ha, in particular 20 g to 600 g of a.i./ha.

The formulations, i.e. the compositions, preparations or combinations comprising the active ingredient of the formula I and, if desired, a solid or liquid additive, are prepared in the known manner, for example by intimately mixing and/or grinding the active ingredient with auxiliaries such as solvents, solid carriers and, if appropriate, surface-active compounds (surfactants).

The following are possible as solvents: aromatic hydrocarbons, preferably the fractions C₈ to C₁₂, such as xylene mixtures or substituted naphthalenes, phthalic esters such as dibutyl phthalate or dioctyl phthalate, aliphatic hydrocarbons such as cyclohexane or paraffins, alcohols and glycols as well as their ethers and esters, such as ethanol, ethylene glycol, ethylene glycol monomethyl ether or ethylene glycol monoethyl ether, ketones such as cyclohexanone, strongly polar solvents such as N-methyl-2-pyrrolidone, dimethyl sulfoxide or dimethylformamide, and epoxidised or unepoxidised vegetable oils, such as epoxidised coconut oil or soya oil, and water.

Solid carriers which are generally used, for example for dusts and dispersible powders, are ground natural minerals, such as calcite, talc, kaolin, montmorillonite or attapulgite. To improve the physical properties, it is also possible to add highly-disperse silica or highly disperse absorptive polymers. Possible particular, adsorptive carriers for granules are either porous types such as pumice, brick grit, sepiolite or bentonite, or non-sorptive carrier materials, for example calcite or sand. Moreover, a large number of pregranulated materials of inorganic nature can be used, such as dolomite or comminuted plant residues.

Suitable surface-active compounds are non-ionic, cationic and/or anionic surfactants having good emulsifying, dispersing and wetting properties, depending on the nature of the active ingredient of the formula I to be formulated. Surfactants are also to be understood as meaning mixtures of surfactants.

Suitable anionic surfactants can be so-called water-soluble soaps or water-soluble synthetic surface-active compounds.

Examples which may be mentioned of non-ionic surfactants are nonylphenol polyethoxyethanols, castor oil polyglycol ethers, polypropylene/polyethylene oxide adducts, tributyphenoxypolyethyleneethanol, polyethylene glycol and octylphenoxypolyethoxyethanol.

Other suitable substances are fatty acid esters of polyoxyethylene sorbitan, such as polyoxyethylene sorbitan trioleate.

The cationic surfactants are mainly quaternary ammonium salts which contain at least one alkyl radical having 8 to 22 C atoms as N-substituent and lower, optionally halogenated alkyl, benzyl or lower hydroxyalkyl radicals as further substituents.

Other surfactants conventionally used in the art of formulation are known to those skilled in the art or can be found in the specialist literature.

As a rule, the agrochemical preparations comprise 0.1 to 99 percent by weight, in particular 0.1 to 95 percent by weight, of active ingredient of the formula I, 99.9 to 1 percent by weight, in particular 99.8 to 5 percent by weight, of a solid or liquid additive and 0 to 25 percent by weight, in particular 0.1 to 25 percent by weight of a surfactant.

While concentrated compositions are more preferred as commercially available goods, the end consumers uses, as a rule, dilute compositions.

The compositions can also comprise further additives such as stabilisers, defoamers, viscosity regulators, binders or adhesives, as well as fertilisers or other active ingredients to achieve specific effects.

The examples hereinafter illustrate the invention described hereinabove without imposing any restriction to the scope of the latter. Temperatures are given in degrees centigrade. The following abbreviations are used: Et = ethyl; iPr - isopropyl; Me = methyl; Pr = n-propyl; m.p. = melting point; MS = mass spectrum. In Examples F-1.1 to F-6.3, the term "%" denotes "percent by weight", the same applies to concentrations of solutions given in Examples B-1.1 to B-8, unless such concentrations are not given in other units. The term "active ingredient according to the invention" in Examples F-1.1 to F-6.3 and B-1.1 to B-8 is to be understood in each case as meaning a compound I, in free form or in the form of an agrochemically utilisable salt, in particular such a compound which is described as product in Examples H-1 to H-3.3, for example one of the products described in Examples H-1, H-2.1, H-2.2, H-2.5, H-2.7, H-2.9, H-2.10, H-2.12, H-2.13, H-2.15, H-2.16, H-2.19, H-2.20, H-2.65, H-2.95 and H-2.103.

### Examples H-1 to H-3.3: Preparation of compounds according to the invention

Example H-1: 1.5 g of methyl 2-(3-bromomethylbenzo[b] thien-2-yl)-3-methoxyacrylate and 0.6 g of acetophenone oxime are added to a suspension of 116 mg of sodium hydride in 15 ml of N,N-dimethylformamide. The reaction mixture is stirred for 30 minutes, acidified using acetic acid, rendered neutral using saturated sodium hydrogen carbonate solution, and extracted three times using ethyl acetate. The combined ethyl acetate phases are dried over anhydrous sodium sulfate and concentrated. The yellow oily residue is purified by chromatography on silica gel using hexane/diethyl ether (7:3) as the eluent. This gives methyl 2-[3-(3-aza-2-oxa-4-phenylpent-3-en-1-yl)benzo[b]thien-2-yl]-3-methoxyacrylate in the form of a colourless oil.

Other compounds of the general formula I which can be prepared analogously to the procedure described in Example H-1 or by means of a suitable other procedure of those mentioned above are the compounds listed in Table 1 hereinafter.

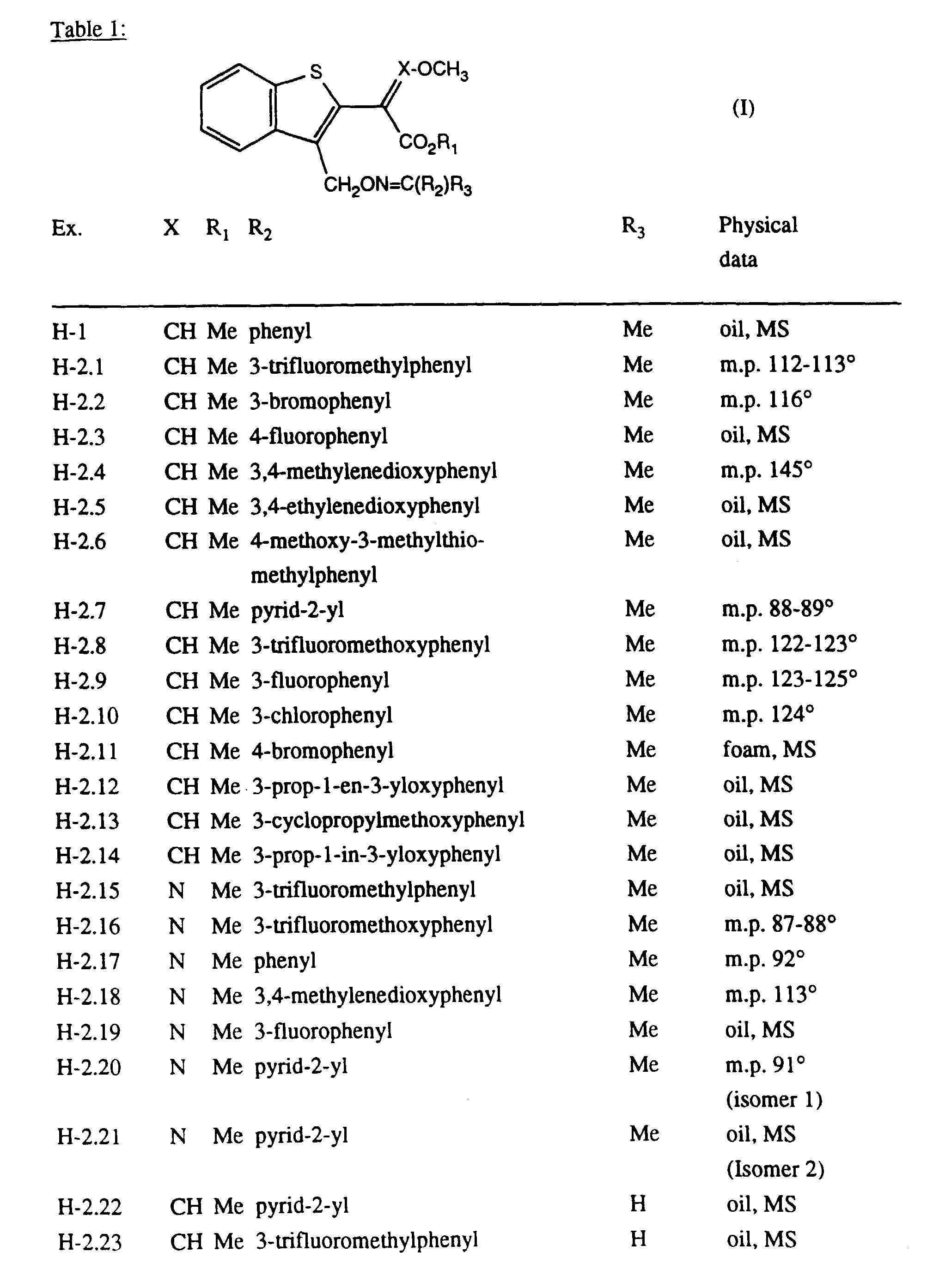

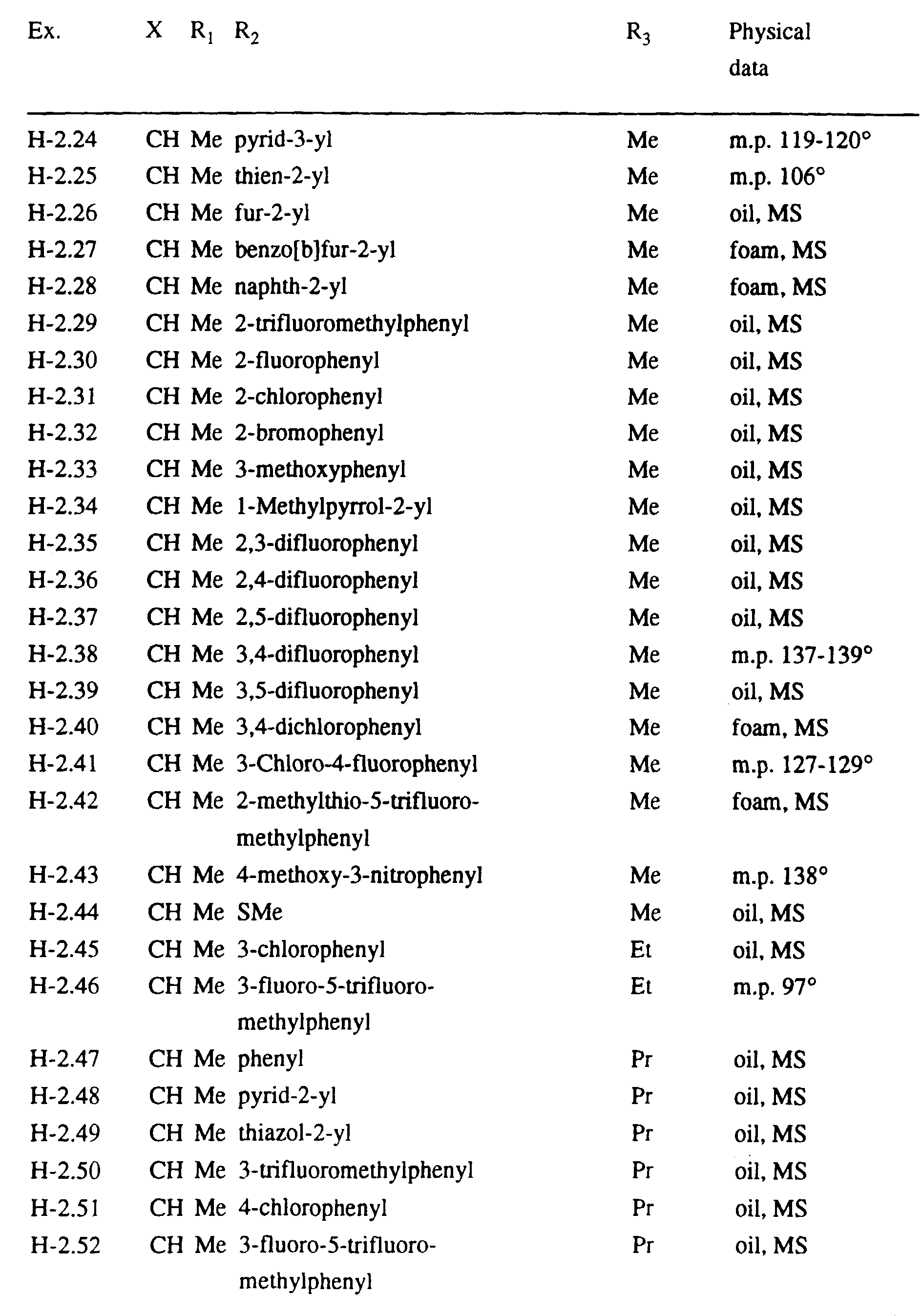

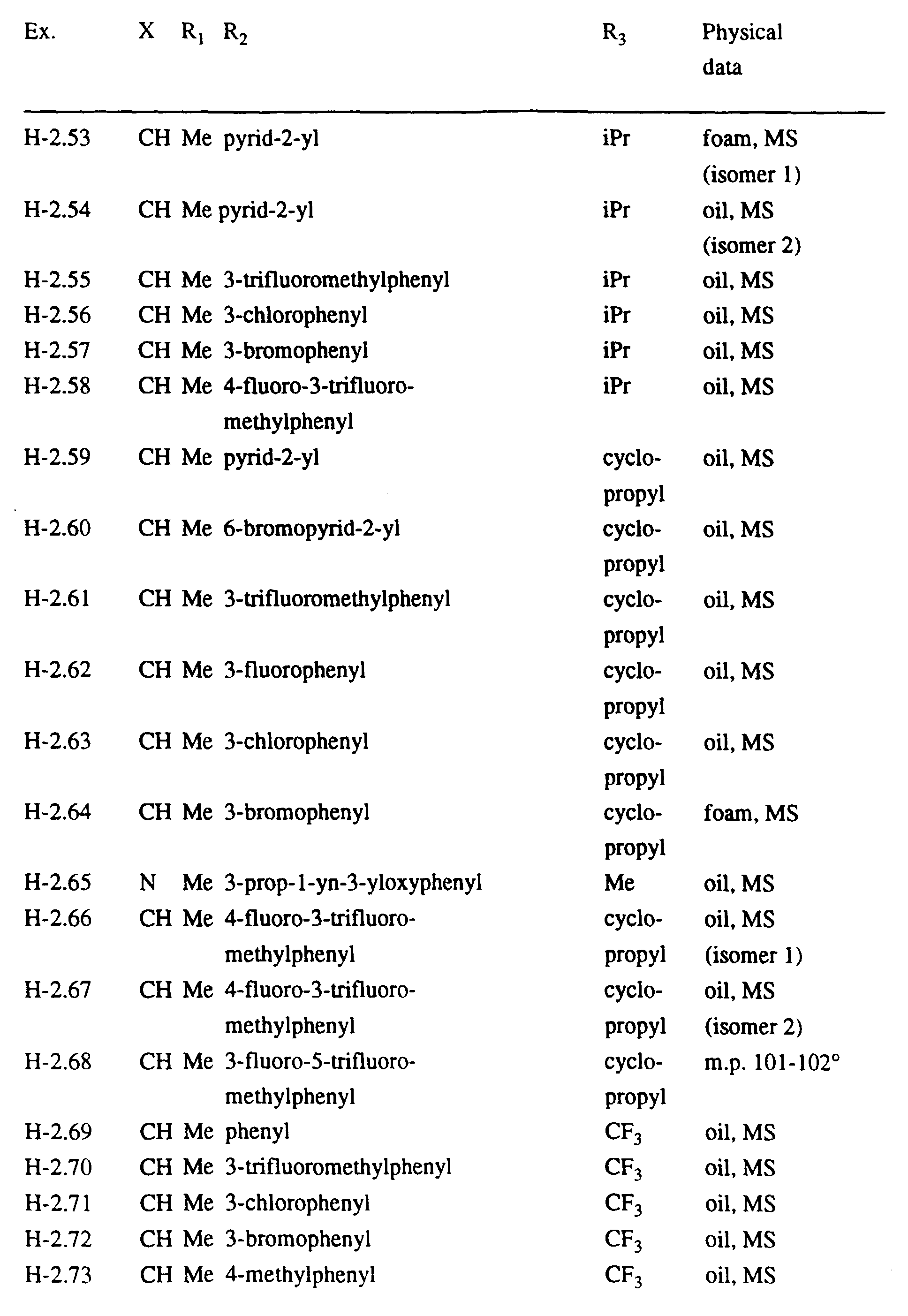

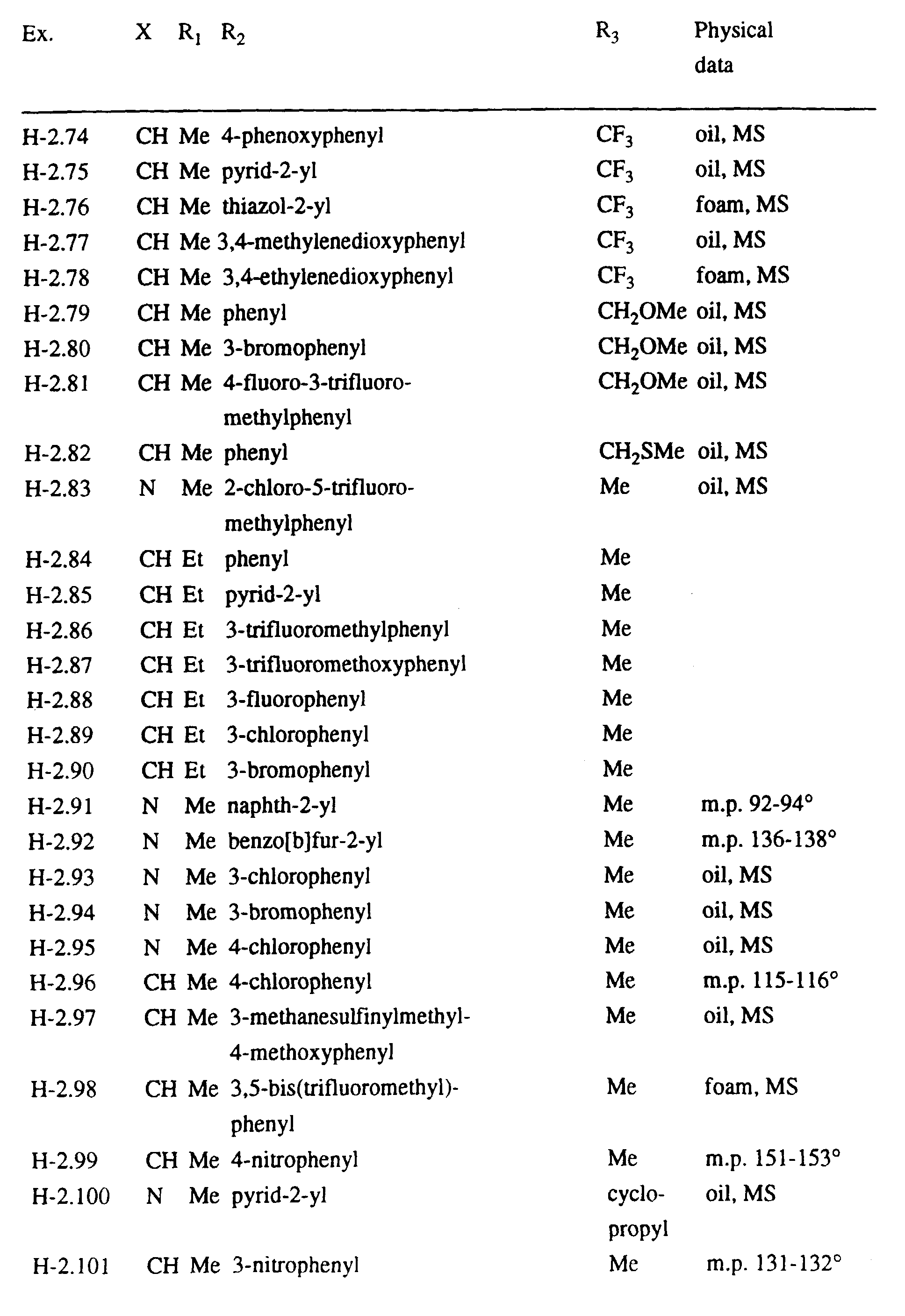

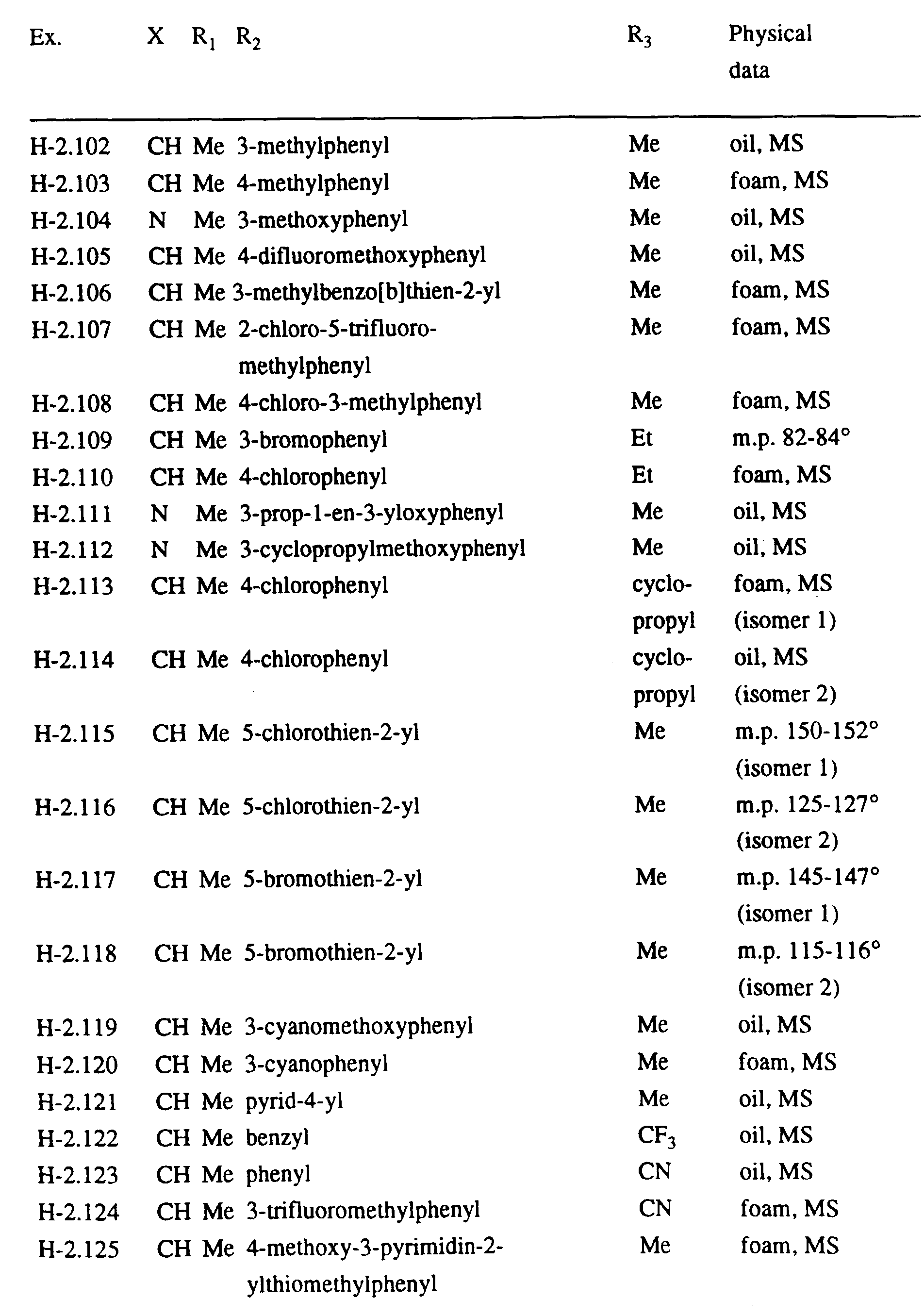

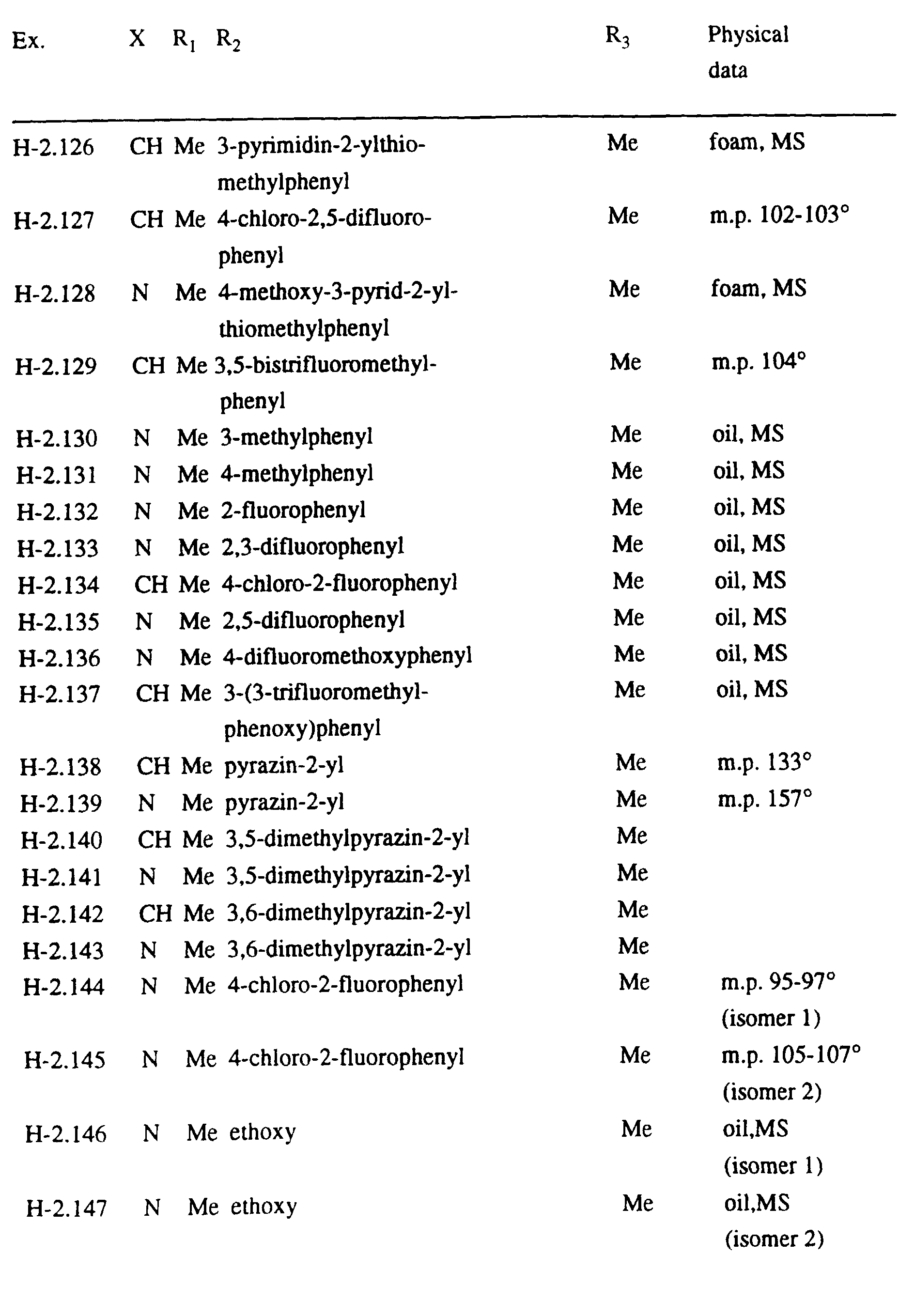

Other compounds of the general formula I which can be prepared analogously to the procedure described in Example H-1 or by means of a suitable other procedure of those indicated above are the compounds listed in Table 2 hereinafter.

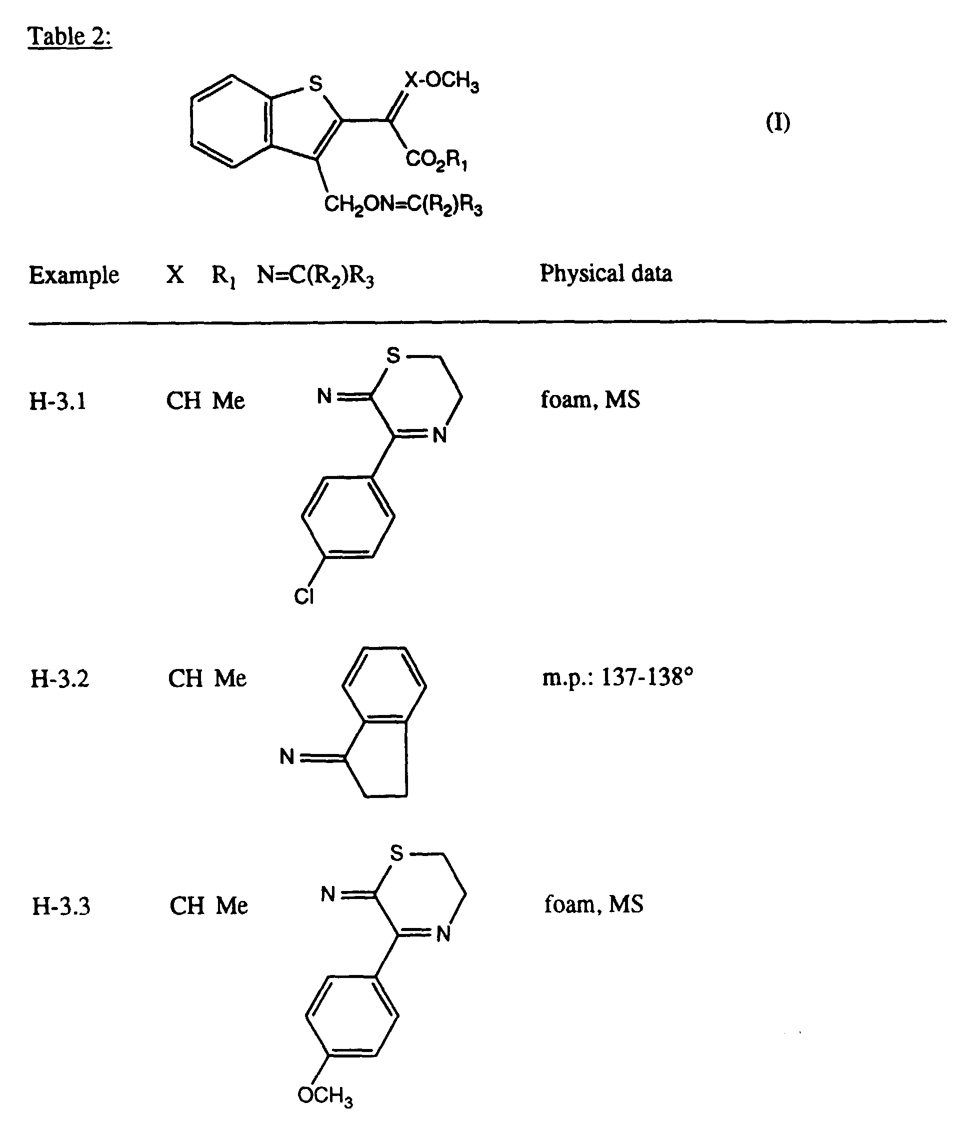

MS data of abovementioned examples.

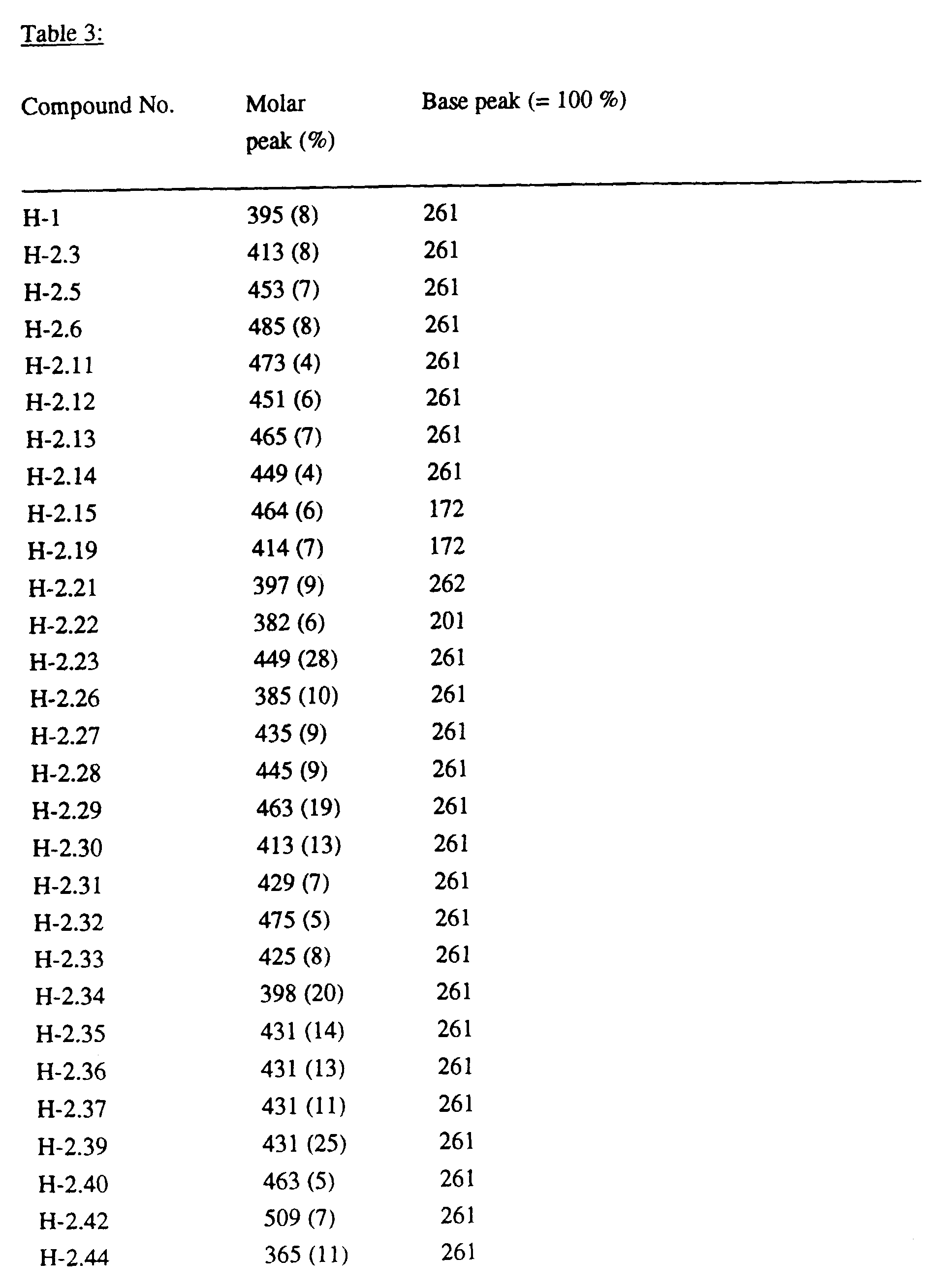

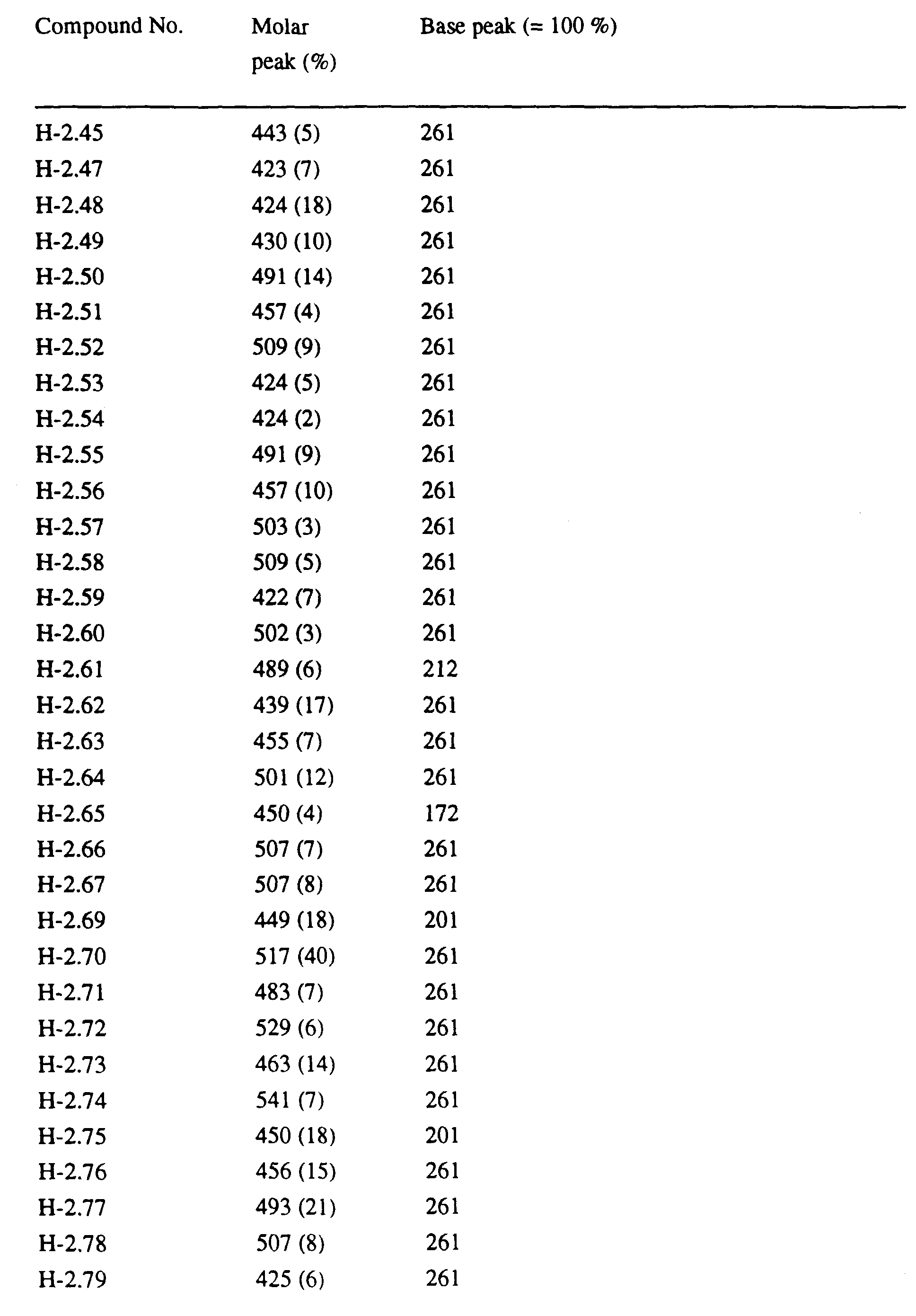

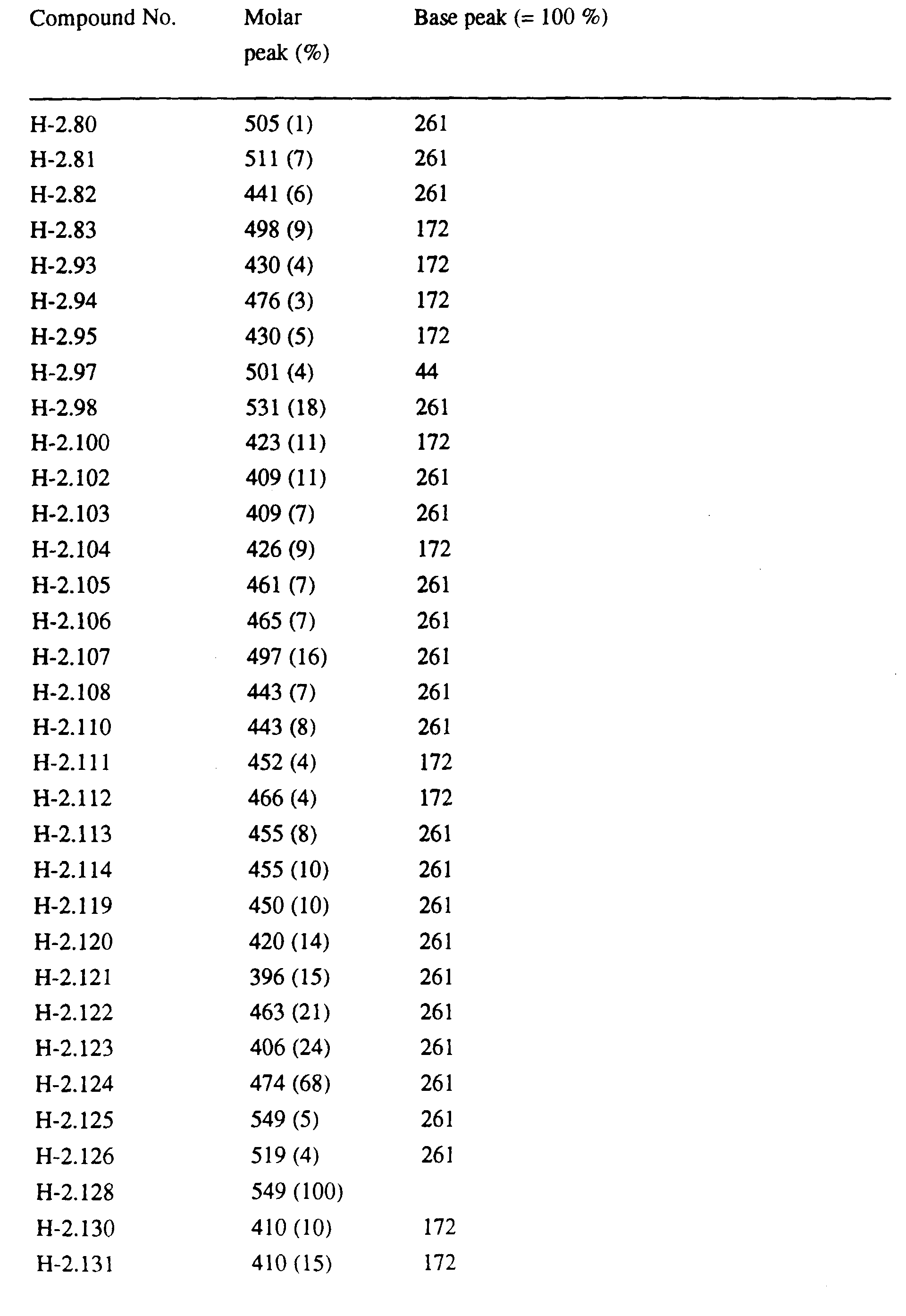

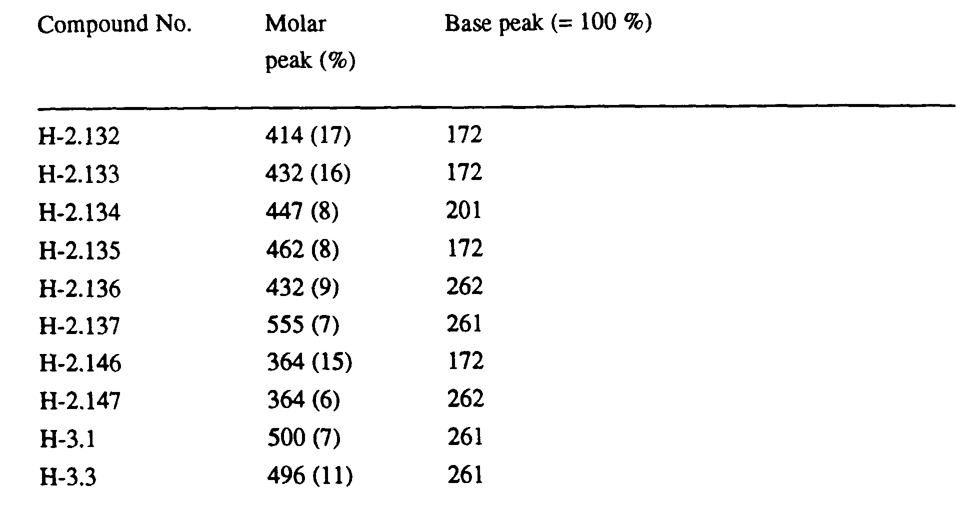

### Examples F-1.1 to F-6.3: Formulation of compounds according to the invention

### Examples F-1.1 to F-1.3: Emulsion concentrates

| Components | F-1.1 | F-1.2 | F-1.3 |
|---|---|---|---|
| Active ingredient according to the invention | 25% | 40% | 50% |
| Calcium dodecylbenzenesulfonate | 5% | 8% | 6% |
| Castor oil polyethylene glycol ether (36 mol of ethylene oxide units) | 5% | - | - |
| Tributylphenol polyethylene glycol ether (30 mol of ethylene oxide units) | - | 12% | 4% |
| Cyclohexanone | - | 15% | 20% |
| Xylene mixture | 65% | 25% | 20% |

Emulsions of any desired low concentration can be prepared from these emulsion concentrates by dilution with water.

### Example F-2: Emulsion concentrate

| Components | F-2 |
|---|---|
| Active ingredient according to the invention | 10% |
| Octylphenol polyethylene glycol ether (4 to 5 mol ethylene oxide units) | 3% |
| Calcium dodecylbenzenesulfonate | 3% |
| Castor oil polyglycol ether (36 mol of ethylene oxide units) | 4% |
| Cyclohexanone | 30% |
| Xylene mixture | 50% |

Emulsions of any desired low concentration can be prepared from this emulsion concentrate by dilution with water.

### Examples F-3.1 to F-3.4: Solutions

| Components | F-3.1 | F-3.2 | F-3.3 | F-3.4 |
|---|---|---|---|---|
| Active ingredient according to the invention | 80% | 10% | 5% | 95% |
| Propylene glycol monomethyl ether | 20% | - | - | - |
| Polyethylene glycol (relative molecular weight: 400 atomic mass units) | - | 70% | - | - |
| N-Methylpyrrolid-2-one | - | 20% | - | - |
| Epoxidised coconut oil | - | - | 1% | 5% |
| Petroleum spirit (boiling range: 160-190°) | - | - | 94% | - |

The solutions are suitable for use in the form of microdrops.

### Examples F-4.1 to F-4.4: Granules

| Components | F-4.1 | F-4.2 | F-4.3 | F-4.4 |
|---|---|---|---|---|
| Active ingredient according to the invention | 5% | 10% | 8% | 21% |
| Kaolin | 94% | - | 79% | 54% |
| Highly-disperse silica | 1% | - | 13% | 7% |
| Attapulgite | - | 90% | - | 18% |

The active ingredient according to the invention is dissolved in dichloromethane, the solution is sprayed onto the carrier, and the solvent is subsequently evaporated in vacuo.

### Examples F-5.1 and F-5.2: Dusts

| Components | F-5.1 | F-5.2 |
|---|---|---|
| Active ingredient according to the invention | 2% | 5% |
| Highly-disperse silica | 1% | 5% |
| Talc | 97% | - |
| Kaolin | - | 90% |

Ready-to-use dusts are obtained by intimately mixing all components.

### Examples F-6. to F-6.3: Wettable powders

| Components | F-6.1 | F-6.2 | F-6.3 |
|---|---|---|---|
| Active ingredient according to the invention | 25% | 50% | 75% |
| Sodium ligninsulfonate | 5% | 5% | - |
| Sodium lauryl sulfate | 3% | - | 5% |
| Sodium diisobutylnaphthalenesulfonate | - | 6% | 10% |
| Octylphenol polyethylene glycol ether (7 to 8 mol of ethylene oxide units)- | | 2% | - |
| Highly-disperse silica | 5% | 10% | 10% |
| Kaolin | 62% | 27% | - |

All components are mixed thoroughly, and the mixture is ground thoroughly in a suitable mill. Wettable powders are obtained from which suspensions of any desired concentration can be prepared with water.

### Examples B-1.1 to B-8: Biological action of compounds according to the invention

### Example B-1.1: Systemic action against Pythium ultimum of sugar beet

Test method: Mycelium of Pythium ultimum is mixed with soil (500 ml of mycelium suspension/10 1 of soil), and 250 ml plastic dishes are filled with the mixture. After incubation at 10° for four days, 10 seeds of the sugar beet plant to be tested are placed into each dish. The next day, 50 ml of an aqueous spray mixture comprising one of the active ingredients according to the invention (0.002% of active ingredient) are poured over each dish. The action of the active ingredient is assessed after an incubation phase for seven days at 10° followed by an incubation phase for four days at 22°, on the basis of number and appearance of the emerged plants.
Test result: Active ingredients according to the invention have a good systemic action against Pythium ultimum on sugar beet.

### Example B-1.2: Systemic action against Pythium ultimum on maize

Test method: The test is carried out analogously to the procedure described in Example B-1.1.
Test result: Active ingredients according to the invention have a good systemic action against Pythium ultimum on maize.

### Example B-2: Action against Puccinia graminis on wheat

a) Residual-protective action
   Test method: 6 days after sowing, wheat plants are sprayed with an aqueous spray mixture prepared with a wettable powder comprising one of the active ingredients according to the invention (0.02% of active ingredient) to drip point and, 24 hours later, infected with a uredospore suspension of the fungus. After an incubation time of 48 hours (conditions: 95 to 100 percent relative atmospheric humidity at 20°), the plants are placed in a greenhouse at 22°. The evaluation of the action of the active ingredient is based on assessment of the rust pustule development 12 days after infection.
   Test result: Active ingredients according to the invention have a good residual-protective action against Puccinia graminis on wheat, for example the active ingredients of Examples H-1, H-2.1, H-2.5, H-2.10, H-2.12, H-2.13, H-2.16, H-2.19, H-2.20, H-2.65, H-2.95 and H-2.103 reduce the fungus infestation to 20 to 5%. In contrast, the fungus infestaton of infected control plants which have not been treated with the active ingredient is 100%.
b) Systemic action
   Test method: 5 days after sowing, an aqueous spray mixture prepared with a wettable powder comprising one of the active ingredients according to the invention (0.006% of active ingredient relative to the soil volume) is poured next to wheat plants. Care is taken that the spray mixture does not come in contact with aerial parts of the plants. 48 hours later, the plants are infected with a uredospore suspension of the fungus. After an incubation time of 48 hours (conditions: 95 to 100 percent relative atmospheric humidity at 20°), the plants are placed in a greenhouse at 22°. The evaluation of the action of the active ingredient is based on assessment of the rust pustule development 12 days after infection.
   Test result: Active ingredients according to the invention have a good systemic action against Puccinia graminis on wheat, for example the active ingredients of Examples H-1, H-2.65 and H-2.95 reduce the fungus infestation to below 20%. In contrast, the fungus infestation of infected control plants which have not been treated with the active ingredient is 100%.

### Example B-3: Action against Phytophthora infestans on tomatoes

a) Residual-protective action
   Test method: Tomato plants are grown for three weeks and then sprayed to drip point with an aqueous spray mixture prepared with a wettable powder comprising one of the active ingredients according to the invention (0.02% of active ingredient) and, 24 hours later, infected with a sporangia suspension of the fungus. The evaluation of the action of the active ingredient is based on an assessment of the fungus infestation 5 days after infection during which period a relative atmospheric humidity of 90 to 100 percent and a temperature of 20° were maintained.
   Test result: Active ingredients according to the invention have a good residual-protective action against Phytophthora infestans on tomato, for example the active ingredients of Examples H-1, H-2.20 and H-2.103 reduce the fungus infestation to 5 to 0%, and the active ingredients of Examples H-2.5 and H-2.19 to 20 to 5%. In contrast, the fungus infestation of infected control plants which have not been treated with the active ingredient is 100%.
b) Systemic action
   Test method: Tomato plants are grown for three weeks, and then an aqueous spray mixture prepared with a wettable powder comprising one of the active ingredients according to the invention (0.006% of active ingredient relative to the soil volume) is poured next to the tomato plants. Care is taken that the spray mixture does not come in contact with aerial parts of the plants. After 48 hours, the plants are infected with a sporangia suspension of the fungus. The evaluation of the action of the active ingredient is based on an assessment of the fungus infestation 5 days after infection, during which period a relative atmospheric humidity of 90-100 percent and a temperature of 20° are maintained.
   Test result: Active ingredients according to the invention have a good systemic action against Phytophthora infestans on tomatoes.

### Example B-4: Residual-protective action against Cercospora arachidicola on groundnuts

Test method: Groundnut plants 10 to 15 cm in height are sprayed to drip point with an aqueous spray mixture prepared with a wettable powder comprising one of the active ingredients according to the invention (0.02% of active ingredient) and, 48 hours later, infected with a conidia suspension of the fungus. The plants are incubated for 72 hours at 21° and high atmospheric humidity and subsequently placed in a greenhouse until the typical lesions appear. The action of the active ingredient is evaluated 12 days after infection based on the number and size of the lesions.
Test result: Active ingredients according to the invention have a good residual-protective action against Cercospora arachidicola on groundnuts, for example active ingredients of Examples H-1, H-2.1, H-2.2, H-2.5, H-2.7, H-2.10, H-2.13, H-2.15, H-2.16, H-2.19 and H-2.20, H-2.65, H-2.95 and H-2.103 reduce the fungus infestation to 5 to 0%, and the active ingredients of Examples H-2.9 and H-2.12 reduce it to 20 to 5%. In contrast, the fungus infestation of infected control plants which have not been treated with the active ingredient is 100%.

### Example B-5: Action against Plasmopara viticola on vines

a) Residual-protective action
   Test method: Vine seedlings in the 4- to 5-leaf stage are sprayed to drip point with an aqueous spray mixture prepared with a wettable powder comprising one of the active ingredients according to the invention (0.02% of active ingredient) and, 24 hours later, infected with a sporangia suspension of the fungus. The evaluation of the action of the active ingredient is based on an assessment of the fungus infestation 6 days after infection during which period a relative atmospheric humidity of 95 to 100 percent and a temperature of 20° are maintained.
   Test result: Active ingredients according to the invention have a good preventive residual-protective action against Plasmopara viticola on vines, for example the active ingredients of Examples H-1, H-2.2, H-2.5, H-2.7, H-2.19, H-2.65 and H-2.95 reduce the fungus infestation to 5 to 0% and the active ingredients of Examples H-2.12, H-2.13 and H-2.20 reduce it to 20 to 5%. In contrast, the fungus infestation of infected control plants which have not been treated with the active ingredient is 100%.
b) Residual-protective action
   Test method: Vine seedlings in the 4- to 5-leaf stage are infected with a sporangia suspension of the fungus, incubated for 24 hours in a humid chamber (conditions: 95 to 100 percent relative atmospheric humidity at 20°) and then sprayed to drip point with an aqueous spray mixture prepared with a wettable powder comprising one of the active ingredients according to the invention (0.02% of active ingredient). After the spray coating has dried on, the plants are returned to the humid chamber. The evaluation of the action of the active ingredient is based on an assessment of the fungus infestation 6 days after infection.
   Test result: Active ingredients according to the invention have a good curative residual-protective action against Plasmopara viticola on vines.

### Example B-6: Action against Pyricularia oryzae on rice

a) Residual-protective action
   Test method: Rice plants are grown for two weeks and then sprayed to drip point with an aqueous spray mixture prepared with a wettable powder comprising one of the active ingredients according to the invention (0.02% of active ingredient) and, 48 hours later, infected with a conidia suspension of the fungus. The evaluation of the action of the active ingredient is based on an assessment of the fungus infestation 5 days after infection, during which period a relative atmospheric humidity of 95 to 100 percent and a temperature of 22° are maintained.
   Test result: Active ingredients according to the invention have a good residual-protective action against Pyricularia oryzae on rice.
b) Systemic action
   Test method: An aqueous spray mixture prepared with a wettable powder comprising one of the active ingredients according to the invention (0.006% of active ingredient relative to the soil volume) is poured next to 2-week-old rice plants. Care is taken that the spray mixture does not come in contact with aerial parts of the plants. The pots are then filled up with water to such an extent that the basal parts of the stems of the rice plants are submerged. After 96 hours, the plants are infected with a conidia suspension of the fungus. The evaluation of the action of the active ingredient is based on an assessment of the fungus infestation 5 days after infection, during which period a relative atmospheric humidity of 95 to 100 percent and a temperature of 24° are maintained.
   Test result: Active ingredients according to the invention have a good systemic action against Pyricularia oryzae on rice.

### Example B-7: Residual-protective action against Venturia inaequalis on apples

Test method: Apple cuttings having fresh shoots 10 to 20 cm in length are sprayed to drip point with an aqueous spray mixture prepared with a wettable powder comprising one of the active ingredients according to the invention (0.02% of active ingredient) and, 24 hours later, infected with a conidia suspension of the fungus. The plants are incubated for 5 days at a relative atmospheric humidity of 90 to 100 percent and placed for a further 10 days in a greenhouse at 20 to 24°. The evaluation of the action of the active ingredient is based on an assessment of the scab infestation 15 days after infection.
Test result: Active ingredients according to the invention show a good residual-protective action against Venturia inaequalis on apples.

### Example B-8: Action against Erysiphe graminis on barley

a) Residual-protective action
   Test method: Barley plants approximately 8 cm in height are sprayed to drip point with an aqueous spray mixture prepared with a wettable powder comprising one of the active ingredients according to the invention (0.02% of active ingredient) and, 3 to 4 hours later, dusted with conidia of the fungus. The infected plants are placed in a greenhouse at 22°. The evaluation of the action of the active ingredient is based on an assessment of the fungus infestation 10 days after infection.
   Test result: Active ingredients according to the invention have a good residual-protective action against Erysiphe graminis on barley, for example the active ingredients of Examples H-1, H-2.2, H-2.7, H-2.12, H-2.13, H-2.15, H-2.16, H-2.65 and H-2.95 reduce the fungus infestation to 5 to 0% and the active ingredient of Example H-2.10 reduces it to 20 to 5%. In contrast, the fungus infestation of infected control plants which have not been treated with the active ingredient is 100%.
b) Systemic action
   Test method: An aqueous spray mixture prepared with a wettable powder comprising one of the active ingredients according to the invention (0.002% of active ingredient relative to the soil volume) is poured next to barley plants approximately 8 cm in height. Care is taken that the spray mixture does not come in contact with aerial parts of the plants. 48 hours later, the plants are dusted with conidia of the fungus. The infected plants are placed in a greenhouse at 22°. The evaluation of the action of the active ingredient is based on an assessment of the fungus infestation 10 days after infection.
   Test result: Active ingredients according to the invention have a good systemic action against Erysiphe graminis on barley.

## Claims

1. A compound of the formula 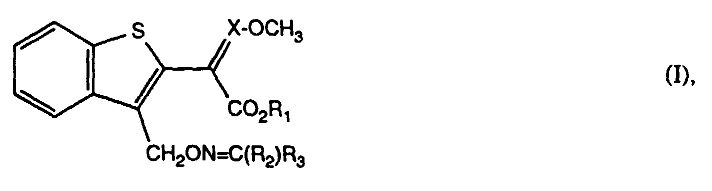 in which:
X is a nitrogen atom or a group of the formula CH (Ia);
R₁ is C₁-C₄alkyl; and
either R₂ and R₃ independently of one another are hydrogen, C₁-C₄alkyl, halo-C₁-C₄alkyl, C₁-C₄alkoxy, C₁-C₄alkoxymethyl, C₁-C₄alkylthio, C₁-C₄alkylthiomethyl, an unsubstituted or substituted aryl, heteroaryl, aryl-C₁-C₄alkyl, heteroaryl-C₁-C₄alkyl-, aryloxy-C_{C}-C₄alkyl, heteroaryloxy-C₁-C₄alkyl, arylthio-C₁-C₄alkyl, heteroarylthio-C₁-C₄alkyl, arylcarbonyl or heteroarylcarbonyl group, C₂-C₄alkenyl, an unsubstituted or substituted aryl-C₂-C₄alkenyl or heteroaryl-C₂-C₄alkenyl group, C₂-C₄alkynyl, C₃-C₆cycloalkyl or cyano,
or R₂ and R₃ together with the carbon atom to which R₂ and R₃ are attached are an unsubstituted or substituted saturated or unsaturated, carbo- or heterocyclic 4- to 7-membered ring to which an unsubstituted or substituted benzene ring may be fused; in free form or, if appropriate, in salt form.

2. A compound according to claim 1 of the formula I, in which:
X is a nitrogen atom or a group Ia;
R₁ is C₁-C₄alkyl; and
either R₂ and R₃ independently of one another are hydrogen, C₁-C₄alkyl, halo-C₁-C₄alkyl, C₁-C₄alkoxy, C₁-C₄alkoxymethyl, C₁-C₄alkylthio, C₁-C₄alkylthiomethyl, C₂-C₄alkenyl, C₂-C₄alkynyl, C₃-C₆cycloalkyl, cyano, an aryl, aryl-C₁-C₄alkyl, aryloxy-C₁-C₄alkyl, arylthio-C₁-C₄alkyl, aryl-C₂-C₄alkenyl or arylcarbonyl group which is unsubstituted or substituted by one, two or three substituents selected from the group comprising halogen, cyano, cyanomethoxy, nitro, trimethylsilyl, C₁-C₄alkyl, halo-C₁-C₄alkyl, C₁-C₄alkoxy, halo-C₁-C₄alkoxy, C₁-C₄alkoxymethyl, aryloxymethyl, heteroaryloxymethyl, arylmethoxy, heteroarylmethoxy, C₃-C₆cycloalkylmethoxy, C₁-C₄alkylthio, C₁-C₄alkylthiomethyl, arylthiomethyl, heteroarylthiomethyl, C₂-C₄alkenyloxy, C₂-C₄alkynyloxy, aryloxy, heteroaryloxy, C₁-C₄alkanesulfinylmethyl, C₁-C₄alkanesulfonylmethyl, arylsulfinylmethyl, arylsulfonylmethyl, heteroarylsulfinylmethyl, heteroarylsulfonylmethyl, the groups of the formula N(R₄)R₅ (Ib) and the groups of the formula CH₂N(R₄)R₅ (Ic), where either R₄ and R₅ in each case independently of one another are hydrogen, C₁-C₄alkyl, aryl, aryl-C₁-C₄alkyl or heteroaryl, or R₄ and R₅ together with the nitrogen atom to which R₄ and R₅ are attached form a pyrrolidine, piperidine or morpholine ring each of which is unsubstituted or substituted by one or two substituents selected from the group comprising C₁-C₄alkyl, and/or this aryl, aryl-C₁-C₄alkyl, aryloxy-C₁-C₄alkyl, arylthio-C₁-C₄alkyl, aryl-C₂-C₄alkenyl or arylcarbonyl group, is substituted on two adjacent carbon atoms by straight-chain C₃-C₅alkylene which is unsubstituted or has one, two or three substituents selected from the group comprising C₁-C₄alkyl, C₁-C₄alkoxy, halogen, phenyl and oxo and in which one or two non-adjacent methylene groups can be replaced by oxygen, or a heteroaryl, heteroaryl-C₁-C₄alkyl, heteroaryloxy-C₁-C₄alkyl, heteroarylthio-C₁-C₄alkyl, heteroaryl-C₂-C₄alkenyl or heteroarylcarbonyl group, each of which is unsubstituted or substituted by one, two or three substituents selected from the group comprising halogen, cyano, cyanomethoxy, nitro, trimethylsilyl, C₁-C₄alkyl, halo-C₁-C₄alkyl,C₁-C₄alkoxy, halo-C₁-C₄alkoxy, C₁-C₄alkoxymethyl, aryloxymethyl, heteroaryloxymethyl, arylmethoxy, heteroarylmethoxy, C₃-C₆cycloalkylmethoxy, C₁-C₄alkylthio, C₁-C₄alkylthiomethyl, arylthiomethyl, heteroarylthiomethyl, C₂-C₄alkenyloxy, C₂-C₄alkynyloxy, aryloxy, heteroaryloxy, C₁-C₄alkanesulfinylmethyl, C₁-C₄alkanesulfonylmethyl, arylsulfinylmethyl, arylsulfonylmethyl, heteroarylsulfinylmethyl, heteroarylsulfonylmethyl, the groups Ib and the groups Ic, where either R₄ and R₅ in each case independently of one another are hydrogen, C₁-C₄alkyl, aryl, aryl-C₁-C₄alkyl or heteroaryl, or R₄ and R₅ together with the nitrogen atom to which R₄ and R₅ are attached form a pyrrolidine, piperidine or morpholine ring, each of which is unsubstituted or substituted by one or two substituents selected from the group comprising C₁-C₄alkyl,
or R₂ and R₃ together with the carbon atom to which R₂ and R₃ are attached form a ring which is unsubstituted or substituted by one, two or three substituents selected from the group comprising C₁-C₄alkyl, phenyl and phenyl which is substituted by one, two or three substituents selected from the group comprising halogen, cyano, cyanomethoxy, nitro, trimethylsilyl, C₁-C₄alkyl, halo-C₁-C₄alkyl, C₁-C₄alkoxy, halo-C₁-C₄alkoxy, C₁-C₄alkoxymethyl, aryloxymethyl, heteroaryloxymethyl, arylmethoxy, heteroarylmethoxy, C₃-C₆cycloalkylmethoxy, C₁-C₄alkylthio, C₁-C₄alkylthiomethyl, arylthiomethyl, heteroarylthiomethyl, C₂-C₄alkenyloxy, C₂-C₄alkynyloxy, aryloxy, heteroaryloxy, C₁-C₄alkanesulfinylmethyl, C₁-C₄alkanesulfonylmethyl, arylsulfinylmethyl, arylsulfonylmethyl, heteroarylsulfinylmethyl, heteroarylsulfonylmethyl, the groups Ib and the groups Ic, where either R₄ and R₅ in each case independently of one another are hydrogen, C₁-C₄alkyl, aryl, aryl-C₁-C₄alkyl or heteroaryl, or R₄ and R₅ together with the nitrogen atom to which R₄ and R₅ are attached form a pyrrolidine, piperidine or morpholine ring, each of which is unsubstituted or substituted by one or two substituents selected from the group comprising C₁-C₄alkyl, and/or this phenyl is substituted on two adjacent carbon atoms by a straight-chain C₃-C₅alkylene which is unsubstituted or has one, two or three substituents selected from the group comprising C₁-C₄alkyl, C₁-C₄alkoxy, halogen, phenyl and oxo, and in which one or two non-adjacent methylene groups can be replaced by oxygen, this ring being saturated or having a single double bond or two or three non-cumulative double bonds, where the maximum number of double bonds which is possible from the structural point of view is a function of the size of the ring and the number and nature of the hetero atoms, and this ring being a 4- to 7-membered carbocylic ring or heterocyclic ring having 1 to 3 hetero atoms selected from the group comprising the oxygen, the sulfur and the nitrogen atom, and to which ring a benzene ring can be fused which is unsubstituted or substituted by one, two or three substituents selected from the group comprising halogen, cyano, cyanomethoxy, nitro, trimethylsilyl, C₁-C₄alkyl, halo-C₁-C₄alkyl, C₁-C₄alkoxy, halo-C₁-C₄alkoxy, C₁-C₄alkoxymethyl, aryloxymethyl, heteroaryloxymethyl, arylmethoxy, heteroarylmethoxy, C₃-C₆cycloalkylmethoxy, C₁-C₄alkylthio, C₁-C₄alkylthiomethyl, arylthiomethyl, heteroarylthiomethyl, C₂-C₄alkenyloxy, C₂-C₄alkynyloxy, aryloxy, heteroaryloxy, C₁-C₄alkanesulfinylmethyl, C₁-C₄alkanesulfonylmethyl, arylsulfinylmethyl, arylsulfonylmethyl, heteroarylsulfinylmethyl, heteroarylsulfonylmethyl, the groups Ib and the groups Ic, where either R₄ and R₅ in each case independently of one another are hydrogen, C₁-C₄alkyl, aryl, aryl-C₁-C₄alkyl or heteroaryl, or R₄ and R₅ together with the nitrogen atom to which R₄ and R₅ are attached form a pyrrolidine, piperidine or morpholine ring, each of which is unsubstituted or substituted by one or two substituents selected from the group comprising C₁-C₄alkyl, and/or this benzene ring is substituted on two adjacent carbon atoms by straight-chain C₃-C₅alkylene which is unsubstituted or has one, two or three substituents selected from the group comprising C₁-C₄alkyl, C₁-C₄alkoxy, halogen, phenyl and oxo, and in which one or two non-adjacent methylene groups can be replaced by oxygen,
where "aryl" - as a group per se and as structural element of other groups - in each case independently of one another - is a carboxcyclic aromatic group having 6 to 14 carbon atoms, and "heteroaryl" - as a group per se and as structural element of other groups - in each case independently of one another is a heterocyclic aromatic group having 5 to 14 ring members of which 1 to 3 members are hetero atoms selected from the group comprising the oxygen, the sulfur and the nitrogen atom; in free form or, if appropriate, in salt form.

3. A compound according to claim 1 of the formula I, in which:
X is a nitrogen atom or a group Ia;
R₁ is C₁-C₄alkyl; and
either R₂ and R₃ independently of one another are hydrogen, C₁-C₄alkyl, halo-C₁-C₄alkyl, C₁-C₄alkoxymethyl, C₁-C₄alkylthio, C₁-C₄alkylthiomethyl, C₃-C₆cycloalkyl, cyano, or a benzyl, phenyl or naphthyl group, each of which is unsubstituted or substituted by one, two or three substituents selected from the group comprising halogen, nitro, C₁-C₄alkyl, halo-C₁-C₄alkyl, C₁-C₄alkoxy, halo-C₁-C₄alkoxy, C₃-C₆cycloalkylmethoxy, cyano, cyanomethoxy, C₁-C₄alkylthio, C₁-C₄alkylthiomethyl, pyrimidinylthiomethyl, C₂-C₄alkenyloxy, C₂-C₄alkynyloxy, phenoxy and C₁-C₄alkanesulfinylmethyl, or each of which is substituted on two adjacent carbon atoms by straight-chain C₁-C₃alkylenedioxy, or are a pyridyl, furyl, benzofuryl, thienyl, benzothienyl, pyrrolyl or thiazolyl group, each of which is unsubstituted or substituted by one, two or three substituents selected from the group comprising halogen and C₁-C₄alkyl,
or R₂ and R₃ together with the carbon atom to which R₂ and R₃ are attached are a cyclopentane or 5,6-dihydro-2H-1,4-thiazine ring, each of which is unsubstituted or substituted by one, two or three substituents selected from the group comprising phenyl which is substituted by one, two or three substituents selected from the group comprising halogen and C₁-C₄alkoxy, to which cyclopentane or 5,6-dihydro-2H-1,4-thiazine ring an unsubstituted benzene ring can be fused; in free form or, if appropriate, in salt form.

4. A compound according to claim 1 of the formula I, in which:
R₁ is C₁-C₄alkyl; and either
(a) X is a nitrogen atom; and
R₂ and R₃ independently of one another are C₁-C₄alkyl, C₃-C₆cycloalkyl or a phenyl or naphthyl group, each of which is unsubstituted or substituted by one, two or three substituents selected from the group comprising C₁-C₄alkoxy, halogen, halo-C₁-C₄alkyl, halo-C₁-C₄alkoxy, C₃-C₆cycloalkylmethoxy, C₂-C₄alkenyloxy and C₂-C₄alkynyloxy, or this phenyl or naphthyl group is substituted on two adjacent carbon atoms by straight-chain C₁-C₃alkylenedioxy, or are an unsubstituted pyridyl or benzofuryl group; or
(b) X is a group Ia; and
either R₂ and R₃ independently of one another are hydrogen, C₁-C₄alkyl, halo-C₁-C₄alkyl, C₁-C₄alkoxymethyl, C₁-C₄alkylthio, C₁-C₄alkylthiomethyl, C₃-C₆cycloalkyl, cyano, or a benzyl, phenyl or naphthyl group which is unsubstituted or substituted by one, two or three substituents selected from the group comprising halogen, nitro, C₁-C₄alkyl, halo-C₁-C₄alkyl, C₁-C₄alkoxy, halo-C₁-C₄alkoxy, C₃-C₆cycloalkylmethoxy, cyano, cyanomethoxy, C₁-C₄alkylthio, C₁-C₄alkylthiomethyl, pyrimidinylthiomethyl, C₂-C₄alkenyloxy, C₂-C₄alkynyloxy, phenoxy and C₁-C₄alkanesulfinylmethyl, or this benzyl, phenyl or naphthyl group is substituted on two adjacent carbon atoms by straight-chain C₁-C₃alkylenedioxy, or are a pyridyl, furyl, benzofuryl, thienyl, benzothienyl, pyrrolyl or thiazolyl group, each of which is unsubstituted or substituted by one, two or three substituents selected from the group comprising halogen and C₁-C₄alkyl, or R₂ and R₃ together with the carbon atom to which R₂ and R₃ are attached are a cyclopentane or 5,6-dihydro-2H-1,4-thiazine ring which is unsubstituted or substituted by one, two or three substituents selected from the group comprising phenyl which is substituted by one, two or three substituents selected from the group comprising halogen and C₁-C₄alkoxy, to which cyclopentane or 5,6-dihydro-2H-1,4-thiazine ring an unsubstituted benzene ring can be fused; in free form or, if appropriate, in salt form.

5. A compound according to claim 1 of the formula I, in which:
R₁ is C₁-C₄alkyl; and either
(c) X is a nitrogen atom;
R₂ is a phenyl group which is unsubstituted or substituted by one or two substituents selected from the group comprising C₁-C₄alkoxy, C₂-C₄alkenyloxy, C₂-C₄alkynyloxy, C₃-C₄cycloalkylmethoxy, halogen, halo-C₁-C₄alkyl having 1 up to and including 3 halogen atoms and halo-C₁-C₄alkoxy having 1 up to and including 3 halogen atoms, or this phenyl group is substituted on two adjacent carbon atoms by straight-chain C₁-C₃alkylenedioxy, or R₂ is an unsubstituted naphtyl-2-yl, benzo[b]fur-2-yl or pyrid-2-yl group; and
R₃ is C₁-C₄alkyl or C₃-C₄cycloalkyl; or
(d) X is a group Ia; and
either R₂ is C₁-C₄alkylthio, a phenyl group which is unsubstituted or substituted by one or two substituents selected from the group comprising halogen, C₁-C₄alkyl, halo-C₁-C₄alkyl having 1 up to and including 3 halogen atoms, C₁-C₄alkoxy, halo-C₁-C₄alkoxy having 1 up to and including 3 halogen atoms, C₃-C₄cycloalkylmethoxy, C₁-C₄alkylthio, C₁-C₄alkylthiomethyl, C₂-C₄alkenyloxy, C₂-C₄alkynyloxy, cyano, cyanomethoxy, nitro, phenoxy, pyrimidinylthiomethyl and C₁-C₄alkanesulfinylmethyl, or which is substituted on two adjacent carbon atoms by straight-chain C₁-C₃alkylenedioxy, or R₂ is a thien-2-yl, pyrid-2-yl or pyrid-3-yl group which is unsubstituted or monosubstituted by halogen, or a pyrrol-2-yl group which is unsubstituted or monosubstituted by C₁-C₄alkyl, or an unsubstituted naphth-2-yl, fur-2-yl, benzo[b]fur-2-yl or thiazol-2-yl group; and
R₃ is hydrogen, C₁-C₄alkyl, halo-C₁-C₄alkyl having 1 up to and including 3 halogen atoms, C₁-C₄alkoxymethyl, C₁-C₄alkylthiomethyl, cyano or C₃-C₄cycloalkyl;
or R₂ and R₃ together with the carbon atom to which R₂ and R₃ are attached are an unsubstituted indane ring whose carbon atom in the 1-position is part of the N=C double bond shown in formula I, or are a 5,6-dihydro-2H-1,4-thiazine ring which is unsubstituted or monosubstituted by monohalophenyl or mono-C₁-C₄alkoxyphenyl and whose carbon atom in the 2-position is part of the N=C double bond shown in formula I; in free form or, if appropriate, in salt form.

6. A compound according to claim 1 of the formula I, in which:
R₁ is C₁-C₄alkyl; and either
(e) X is a nitrogen atom;
R₂ is unsubstituted phenyl, 3- or 4-halophenyl, 3-halo-C₁-C₄alkylphenyl having 1 up to and including 3 halogen atoms, 2-halo-5-halo-C₁-C₄alkylphenyl having 1 up to and including 3 halogen atoms in the haloalkyl substituent, 3-C₁-C₄alkoxyphenyl, 3-halo-C₁-C₄alkoxyphenyl having 1 up to and including 3 halogen atoms, 3-C₂-C₄alkenyloxyphenyl, 3-C₂-C₄akynyloxyphenyl, 3-C₃-C₄cycloalkylmethoxyphenyl, phenyl which is substituted in the 3- and 4-positions by straight-chain C₁-C₃alkylenedioxy, or an unsubstituted naphth-2-yl, benzo[b]fur-2-yl or pyrid-2-yl group; and
R₃ is C₁-C₄alkyl or C₃-C₄cycloalkyl; or
(f) X is a group Ia; and
either R₂ is C₁-C₄alkylthio, unsubstituted phenyl, 2-, 3- or 4-halophenyl, 3,4-dihalophenyl in which the two halogen substituents are identical or different, 3- or 4-C₁-C₄alkylphenyl, 3-C₁-C₄alkyl-4-halophenyl, 2- or 3-halo-C₁-C₄alkylphenyl having 1 up to and including 3 halogen atoms, 3,5-bis(halo-C₁-C₄alkyl)phenyl having 1 up to and including 3 halogen atoms, 4-halo-3-halo-C₁-C₄alkylphenyl having 1 up to and including halogen atoms in the haloalkyl substituent, 2-halo-5-halo-C₁-C₄alkylphenyl having 1 up to and including 3 halogen atoms in the haloalkyl substituent, 3-C₁-C₄alkoxyphenyl, 3-halo-C₁-C₄alkoxyphenyl having 1 up to and including 3 halogen atoms, 3-C₃-C₄cycloalkylmethoxyphenyl, 2-C₁-C₄alkylthio-5-halo-C₁-C₄alkylphenyl having 1 up to and including 3 halogen atoms in the haloalkyl substituent, 4-C₁-C₄alkoxy-3-C₁-C₄alkylthiomethylphenyl, 3-C₂-C₄alkenyloxyphenyl, 3-C₂-C₄alkynyloxyphenyl, 4-phenoxyphenyl, 3- or 4-nitrophenyl, 3-cyanophenyl, 3-cyanomethoxyphenyl, 4-C₁-C₄alkoxy-3-nitrophenyl, 3-C₁-C₄alkanesulfinylmethyl-4-C₁-C₄alkoxyphenyl, 4-C₁-C₄alkoxy-3-pyrimidin-2-ylthiomethylphenyl, phenyl which is substituted in the 3-and 4-positions by straight-chain C₁-C₃alkylenedioxy, or an unsubstituted thien-2-yl, pyrid-2-yl or pyrid-3-yl, 6-halopyrid-2-yl, 5-halothien-2-yl, 1-C₁-C₄alkylpyrrol-2-yl or an unsubstituted naphth-2-yl, fur-2-yl, benzo[b]fur-2-yl or thiazol-2-yl group; and
R₃ is hydrogen, C₁-C₄alkyl, halo-C₁-C₄alkyl having 1 up to and including 3 halogen atoms, C₁-C₄alkoxymethyl, C₁-C₄alkylthiomethyl, cyano or C₃-C₄cycloalkyl;
or R₂ and R₃ together with the carbon atom to which R₂ and R₃ are attached are an unsubstituted indane ring whose carbon atom in the 1-position is part of the N=C double bond shown in formula I, or a 5,6-dihydro-2H-1,4-thiazine ring which is monosubstituted by 4-halophenyl or 4-C₁-C₄alkoxyphenyl and whose carbon atom in the 2-position is part of the N=C double bond shown in formula I; in free form or, if appropriate, in salt form.

7. A compound according to claim 1 of the formula I, in which:
X is a nitrogen atom;
R₁ is C₁-C₂alkyl;
R₂ is 3-fluorophenyl, 3-trifluoromethylphenyl, 3-trifluoromethoxyphenyl or unsubstituted pyrid-2-yl; and
R₃ is C₁-C₂alkyl; in free form or, if appropriate, in salt form.

8. A compound according to claim 1 of the formula I, in which:
X is a nitrogen atom;
R₁ is C₁-C₂alkyl;
R₂ is 3-chlorophenyl, 4-chlorophenyl, 3-prop-1-yn-3-yloxyphenyl or 4-prop-1-yn-3-yloxyphenyl;
R₃ is C₁-C₂alkyl; in free form or, if appropriate, in salt form.

9. A compound according to claim 1 of the formula I, in which:
X is a group Ia;
R₁ is C₁-C₂alkyl;
R₂ is unsubstituted phenyl, 3-fluorophenyl, 3-chlorophenyl, 3-bromophenyl, 3-trifluoromethylphenyl, 3-cyclopropylmethoxyphenyl, 3-allyloxyphenyl, 3,4-ethylenedioxyphenyl or unsubstituted pyrid-2-yl; and
R₃ is C₁-C₂alkyl; in free form or, if appropriate, in salt form.

10. A compound according to claim 1 of the formula I, selected from the group of compounds comprising
methyl 2-[3-(3-aza-2-oxa-4-phenylpent-3-en-1-yl)benzo[b]thien-2-yl]-3-methoxyacrylate,
methyl 2-{3-[3-aza-2-oxa-4-(3-trifluoromethylphenyl)pent-3-en-1-yl]benzo[b]thien-2-yl}-3-methoxyacrylate,
methyl 2-{3-[3-aza-4-(3-bromophenyl)-2-oxapent-3-en-1-yl}benzo[b]thien-2-yl)-3-methoxyacrylate,
methyl 2-{3-[3-aza-4-(3,4-ethylenedioxyphenyl)-2-oxapent-3-en-1-yl]benzo[b]thien-2-yl}3-methoxyacrylate,
methyl 2-[3-(3-aza-2-oxa-4-pyrid-2-ylpent-3-en-1-yl)benzo[b]thien-2-yl]-3-methoxyacrylate,
methyl 2-{3-[3-aza-4-(3-fluorophenyl)-2-oxapent-3-en-1-yl]benzo[b]thien-2-yl}-3-methoxyacrylate,
methyl 2-{3-[3-aza-4-(3-chlorophenyl)-2-oxapent-3-en-1-yl]benzo[b]thien-2-yl}-3-methoxyacrylate,
methyl 2-{3-[3-aza-2-oxa-4-(3-prop-1-en-3-yloxyphenyl)pent-3-en-1-yl]benzo[b]thien-2-yl}-3-methoxyacrylate,
methyl 2-{3-[3-aza-4-(3-cyclopropylmethoxyphenyl)-2-oxapent-3-en-1-yl]benzo[b]thien-2-yl}-3-methoxyacrylate,
methyl 2-{3-[3-aza-2-oxa-4-(3-trifluoromethylphenyl)pent-3-en-1-yl]benzo[b]thien-2-yl}-2-methoxyiminoacetate,
methyl 2-{3-[3-aza-2-oxa-4-(3-trifluoromethoxyphenyl)pent-3-en-1-yl]benzo[b]thien-2-yl}-2-methoxyiminoacetate,
methyl 2-{3-[3-aza-4-(3-fluorophenyl)-2-oxapent-3-en-1-yl]benzo[b]thien-2-yl}-2-methoxyiminoacetate,
methyl 2-[3-(3-aza-2-oxa-4-pyrid-2-ylpent-3-en-1-yl)benzo[b]thien-2-yl]-2-methoxyiminoacetate,
in each case in free form or, if appropriate, in salt form.

11. A compound according to claim 1 of the formula I, selected from the group of compounds comprising
methyl 2-{3-[3-aza-2-oxa-4-(3-prop-1-in-3-yloxyphenyl)pent-3-en-1-yl]benzo[b]thien-2-yl}-3-methoxyiminoacetate,
methyl 2-{3-[3-aza-4-(4-chlorophenyl)-2-oxapent-3-en-1-yl]benzo[b]thien-2-yl}-3-methoxyiminoacetate,
methyl 2-{3-[3-aza-4-(4-methylphenyl)-2-oxapent-3-en-1-yl]benzo[b]thien-2-yl}-3-methoxyacrylate,
in each case in free form or, if appropriate, in salt form.

12. A process for the preparation of a compound of the formula I, in free form or, if appropriate, in salt form, which comprises reacting a compound of the formula 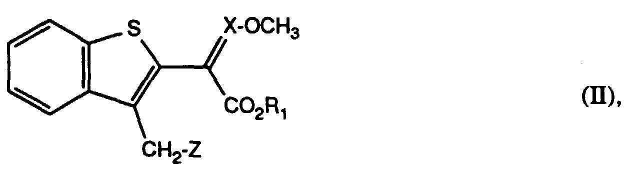 in which R₁ and X are as defined in formula I and Z is an easily detachable nucleofugic radical, in particular bromine, with a compound of the formula
HO-N = C(R₂)(R₃) (III),
in which R₂ and R₃ are as defined in formula I, or, if appropriate, with a salt thereof, preferably in the presence of a base, if appropriate in the presence of an inert solvent or diluent, and/or converting a compound of the formula I which can be obtained by the process or by another route, in free form or, if appropriate, in salt form, into a different compound of the formula I, and/or resolving an isomer mixture which can be obtained according to the process and isolating the desired isomer, and/or, if desired, converting a free compound of the formula I which can be obtained according to the process into a salt, or converting a salt of a compound of the formula I which can be obtained according to the process into the free compound of the formula I or, if appropriate, into a different salt

13. A composition for protecting plants against infestation with pests, which comprises at least one compound according to claim 1, in free form or in salt form, as active ingredient and at least one auxiliary.

14. A composition according to claim 13 for protecting plants against infestation with phytopathogenic microorganisms.

15. A process for the preparation of a composition according to claim 13, which comprises intimately mixing the active ingredient with the auxiliary(ies).

16. The use of a compound according to claim 1, in free form or in salt form, or of a composition according to claim 13, for protecting plants against infestation with pests.

17. The use according to claim 16 for protecting plants against infestation with phytopathogenic microorganisms.

18. A method for protecting plants against infestation with pests, which comprises applying a compound according to claim 1, in free form or in salt form, as active ingredient to the plants or their locus.

19. A method according to claim 18 for protecting plants against infestation with phytopathogenic microorganisms.

## Patentansprüche

1. Verbindung der Formel 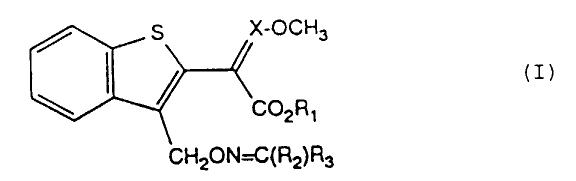 worin bedeuten:
X ein Stickstoffatom oder eine Gruppe der Formel CH (Ia);
R₁ C₁-C₄-Alkyl; und
entweder R₂ und R₃, unabhängig voneinander, Wasserstoff, C₁-C₄-Alkyl, Halogen-C₁-C₄-alkyl, C₁-C₄-Alkoxy, C₁-C₄-Alkoxymethyl, C₁-C₄-Alkylthio, C₁-C₄-Alkylthiomethyl, eine unsubstituierte oder substituierte Aryl-, Heteroaryl-, Aryl-C₁-C₄-alkyl-, Heteroaryl-C₁-C₄-alkyl-, Aryloxy-C₁-C₄-alkyl-, Heteroaryloxy-C₁-C₄-alkyl-, Arylthio-C₁-C₄-alkyl-, Heteroarylthio-C₁-C₄-alkyl-, Arylcarbonyl- oder Heteroarylcarbonyl-Gruppe, C₂-C₄-Alkenyl, eine unsubstituierte oder substituierte Aryl-C₂-C₄-alkenyl- oder Heteroaryl-C₂-C₄-alkenyl-Gruppe, C₂-C₄-Alkinyl, C₃-C₆-Cycloalkyl oder Cyano,
oder R₂ und R₃, zusammen mit dem Kohlenstoffatom, an das R₂ und R₃ gebunden sind, einen unsubstituierten oder substituierten, gesättigten oder ungesättigten, carbo- oder heterocyclischen 4- bis 7-gliedrigen Ring, an den ein unsubstituierter oder substituierter Benzolring kondensiert sein kann;
in freier Form oder gegebenenfalls in Salzform.

2. Verbindung nach Anspruch 1 der Formel I, worin bedeuten:
X ein Stickstoffatom oder eine Gruppe Ia;
R₁ C₁-C₄-Alkyl; und
entweder R₂ und R₃, unabhängig voneinander, Wasserstoff, C₁-C₄-Alkyl, Halogen-C₁-C₄-alkyl, C₁-C₄-Alkoxy, C₁-C₄-Alkoxymethyl, C₁-C₄-Alkylthio, C₁-C₄-Alkylthiomethyl, C₂-C₄-Alkenyl, C₂-C₄-Alkinyl, C₃-C₆-Cycloalkyl, Cyano, eine unsubstituierte oder mit einem, zwei oder drei Substituenten, ausgewählt aus der Gruppe, umfassend Halogen, Cyano, Cyanomethoxy, Nitro, Trimethylsilyl, C₁-C₄-Alkyl, Halogen-C₁-C₄-alkyl, C₁-C₄-Alkoxy, Halogen-C₁-C₄-alkoxy, C₁-C₄-Alkoxymethyl, Aryloxymethyl, Heteroaryloxymethyl, Arylmethoxy, Heteroarylmethoxy, C₃-C₆-Cycloalkylmethoxy, C₁-C₄-Alkylthio, C₁-C₄-Alkylthiomethyl, Arylthiomethyl, Heteroarylthiomethyl, C₂-C₄-Alkenyloxy, C₂-C₄-Alkinyloxy, Aryloxy, Heteroaryloxy, C₁-C₄-Alkansulfinylmethyl, C₁-C₄-Alkansulfonylmethyl, Arylsulfinylmethyl, Arylsulfonylmethyl, Heteroarylsulfinylmethyl, Heteroarylsulfonylmethyl, die Gruppen der Formel N(R₄)R₅ (Ib) und die Gruppen der Formel CH₂N(R₄)R₅ (Ic), wobei entweder R₄ und R₅ jeweils, unabhängig voneinander, Wasserstoff, C₁-C₄-Alkyl, Aryl, Aryl-C₁-C₄-alkyl oder Heteroaryl bedeuten oder R₄ und R₅, zusammen mit dem Stickstoffatom, an das R₄ und R₅ gebunden sind, einen unsubstituierten oder mit einem oder zwei Substituenten, ausgewählt aus der Gruppe, umfassend C₁-C₄-Alkyl, substituierten Pyrrolidin-, Piperidin- oder Morpholin-Ring bilden, substituierte und/oder an zwei benachbarten Kohlenstoffatomen mit unsubstituiertem oder einen, zwei oder drei Substituenten, ausgewählt aus der Gruppe, umfassend C₁-C₄-Alkyl, C₁-C₄-Alkoxy, Halogen, Phenyl und Oxo, aufweisendem, geradkettigem C₃-C₅-Alkylen, in welchem eine oder zwei einander nichtbenachbarte Methylengruppen durch Sauerstoff ersetzt sein können, substituierte Aryl-, Aryl-C₁-C₄-alkyl-, Aryloxy-C₁-C₄-alkyl-, Arylthio-C₁-C₄-alkyl-, Aryl-C₂-C₄-alkenyl- oder Arylcarbonyl-Gruppe oder eine unsubstituierte oder mit einem, zwei oder drei Substituenten, ausgewählt aus der Gruppe, umfassend Halogen, Cyano, Cyanomethoxy, Nitro, Trimethylsilyl, C₁-C₄-Alkyl, Halogen-C₁-C₄-alkyl, C₁-C₄-Alkoxy, Halogen-C₁-C₄-alkoxy, C₁-C₄-Alkoxymethyl, Aryloxymethyl, Heteroaryloxymethyl, Arylmethoxy, Heteroarylmethoxy, C₃-C₆-Cycloalkylmethoxy, C₁-C₄-Alkylthio, C₁-C₄-Alkylthiomethyl, Arylthiomethyl, Heteroarylthiomethyl, C₂-C₄-Alkenyloxy, C₂-C₄-Alkinyloxy, Aryloxy, Heteroaryloxy, C₁-C₄-Alkansulfinylmethyl, C₁-C₄-Alkansulfonylmethyl, Arylsulfinylmethyl, Arylsulfonylmethyl, Heteroarylsulfinylmethyl, Heteroarylsulfonylmethyl, die Gruppen Ib und die Gruppen Ic, wobei entweder R₄ und R₅ jeweils, unabhängig voneinander, Wasserstoff, C₁-C₄-Alkyl, Aryl, Aryl-C₁-C₄-alkyl oder Heteroaryl bedeuten oder R₄ und R₅, zusammen mit dem Stickstoffatom, an das R₄ und R₅ gebunden sind, einen unsubstituierten oder mit einem oder zwei Substituenten, ausgewählt aus der Gruppe, umfassend C₁-C₄-Alkyl, substituierten Pyrrolidin-, Piperidin- oder Morpholin-Ring bilden, substituierte Heteroaryl-, Heteroaryl-C₁-C₄-alkyl-, Heteroaryloxy-C₁-C₄-alkyl-, Heteroarylthio-C₁-C₄-alkyl-, Heteroaryl-C₂-C₄-alkenyl- oder Heteroarylcarbonyl-Gruppe
oder R₂ und R₃, zusammen mit dem Kohlenstoffatom, an das R₂ und R₃ gebunden sind, einen unsubstituierten oder mit einem, zwei oder drei Substituenten, ausgewählt aus der Gruppe, umfassend C₁-C₄-Alkyl, Phenyl und mit einem, zwei oder drei Substituenten, ausgewählt aus der Gruppe, umfassend Halogen, Cyano, Cyanomethoxy, Nitro, Trimethylsilyl, C₁-C₄-Alkyl, Halogen-C₁-C₄-alkyl, C₁-C₄-Alkoxy, Halogen-C₁-C₄-alkoxy, C₁-C₄-Alkoxymethyl, Aryloxymethyl, Heteroaryloxymethyl, Arylmethoxy, Heteroarylmethoxy, C₃-C₆-Cycloalkylmethoxy, C₁-C₄-Alkylthio, Cₗ-C₄-Alkylthiomethyl, Arylthiomethyl, Heteroarylthiomethyl, C₂-C₄-Alkenyloxy, C₂-C₄-Alkinyloxy, Aryloxy, Heteroaryloxy, C₁-C₄-Alkansulfinylmethyl, C₁-C₄-Alkansulfonylmethyl, Arylsulfinylmethyl, Arylsulfonylmethyl, Heteroarylsulfinylmethyl, Heteroarylsulfonylmethyl, die Gruppen Ib und die Gruppen Ic, wobei entweder R₄ und R₅ jeweils, unabhängig voneinander, Wasserstoff, C₁-C₄-Alkyl, Aryl, Aryl-C₁-C₄-alkyl oder Heteroaryl bedeuten oder R₄ und R₅, zusammen mit dem Stickstoffatom, an das R₄ und R₅ gebunden sind, einen unsubstituierten oder mit einem oder zwei Substituenten, ausgewählt aus der Gruppe, umfassend C₁-C₄-Alkyl, substituierten Pyrrolidin-, Piperidin- oder Morpholin-Ring bilden, substituiertes und/oder an zwei benachbarten Kohlenstoffatomen mit unsubstituiertem oder einem, zwei oder drei Substituenten, ausgewählt aus der Gruppe, umfassend C₁-C₄-Alkyl, C₁-C₄-Alkoxy, Halogen, Phenyl und Oxo, aufweisendem, geradkettigem C₃-C₅-Alkylen, in welchem eine oder zwei einander nichtbenachbarte Methylengruppen durch Sauerstoff ersetzt sein können, substituiertes Phenyl, substituierten, gesättigten oder eine einzelne Doppelbindung oder zwei oder drei nichtkumulierte Doppelbindungen, wobei die strukturell mögliche Maximalzahl der Doppelbindungen von der Ringgröße und der Anzahl und der Art der Heteroatome abhängig ist, aufweisenden 4- bis 7-gliedrigen carbo- oder 1 bis 3 Heteroatome, ausgewählt aus der Gruppe, umfassend das Sauerstoff-, das Schwefel- und das Stickstoffatom, aufweisenden heterocyclischen Ring, an den ein unsubstituierter oder mit einem, zwei oder drei Substituenten, ausgewählt aus der Gruppe, umfassend Halogen, Cyano, Cyanomethoxy, Nitro, Trimethylsilyl, C₁-C₄-Alkyl, Halogen-C₁-C₄-alkyl, C₁-C₄-Alkoxy, Halogen-C₁-C₄-alkoxy, C₁-C₄-Alkoxymethyl, Aryloxymethyl, Heteroaryloxymethyl, Arylmethoxy, Heteroarylmethoxy, C₃-C₆-Cycloalkylmethoxy, C₁-C₄-Alkylthio, C₁-C₄-Alkylthiomethyl, Arylthiomethyl, Heteroarylthiomethyl, C₂-C₄-Alkenyloxy, C₂-C₄-Alkinyloxy, Aryloxy, Heteroaryloxy, C₁-C₄-Alkansulfinylmethyl, C₁-C₄-Alkansulfonylmethyl, Arylsulfinylmethyl, Arylsulfonylmethyl, Heteroarylsulfinylmethyl, Heteroarylsulfonylmethyl, die Gruppen Ib und die Gruppen Ic, wobei entweder R₄ und R₅ jeweils, unabhängig voneinander, Wasserstoff, C₁-C₄-Alkyl, Aryl, Aryl-C₁-C₄-alkyl oder Heteroaryl bedeuten oder R₄ und R₅, zusammen mit dem Stickstoffatom, an das R₄ und R₅ gebunden sind, einen unsubstituierten oder mit einem oder zwei Substituenten, ausgewählt aus der Gruppe, umfassend C₁-C₄-Alkyl, substituierten Pyrrolidin-, Piperidin- oder Morpholin-Ring bilden, substituierter und/oder an zwei benachbarten Kohlenstoffatomen mit unsubstituiertem oder einen, zwei oder drei Substituenten, ausgewählt aus der Gruppe, umfassend C₁-C₄-Alkyl, C₁-C₄-Alkoxy, Halogen, Phenyl und Oxo, aufweisendem, geradkettigem C₃-C₅-Alkylen, in welchem eine oder zwei einander nichtbenachbarte Methylengruppen durch Sauerstoff ersetzt sein können, substituierter Benzolring kondensiert sein kann,
wobei "Aryl" - als Gruppe sowie als Strukturelement von anderen Gruppen - in jedem Fall unabhängig voneinander eine carbocyclische aromatische Gruppe mit 6 bis 14 Kohlenstoffatomen bedeutet und "Heteroaryl" - als Gruppe sowie als Strukturelement von anderen Gruppen - in jedem Fall unabhängig voneinander eine heterocyclische aromatische Gruppe mit 5 bis 14 Ringgliedern, von denen 1 bis 3 Glieder Heteroatome sind, ausgewählt aus der Gruppe, umfassend das Sauerstoff-, das Schwefel- und das Stickstoffatom, darstellt;
in freier Form oder gegebenenfalls in Salzform.

3. Verbindung nach Anspruch 1 der Formel I, worin bedeuten:
X ein Stickstoffatom oder eine Gruppe Ia;
R₁ C₁-C₄-Alkyl; und
entweder R₂ und R₃, unabhängig voneinander, Wasserstoff, C₁-C₄-Alkyl, Halogen-C₁-C₄-alkyl, C₁-C₄-Alkoxymethyl, C₁-C₄-Alkylthio, C₁-C₄-Alkylthiomethyl, C₃-C₆-Cycloalkyl, Cyano, eine unsubstituierte oder mit einem, zwei oder drei Substituenten, ausgewählt aus der Gruppe, umfassend Halogen, Nitro, C₁-C₄-Alkyl, Halogen-C₁-C₄-alkyl, C₁-C₄-Alkoxy, Halogen-C₁-C₄-alkoxy, C₃-C₆-Cycloalkylmethoxy, Cyano, Cyanomethoxy, C₁-C₄-Alkylthio, C₁-C₄-Alkylthiomethyl, Pyrimidinylthiomethyl, C₂-C₄-Alkenyloxy, C₂-C₄-Alkinyloxy, Phenoxy und C₁-C₄-Alkansulfinylmethyl, substituierte oder an zwei benachbarten Kohlenstoffatomen mit geradkettigem C₁-C₃-Alkylendioxy substituierte Benzyl-, Phenyl- oder Naphthyl-Gruppe oder eine unsubstituierte oder mit einem, zwei oder drei Substituenten, ausgewählt aus der Gruppe, umfassend Halogen und C₁-C₄-Alkyl, substituierte Pyridyl-, Furyl-, Benzofuryl-, Thienyl-, Benzothienyl-, Pyrrolyl- oder Thiazolyl-Gruppe
oder R₂ und R₃, zusammen mit dem Kohlenstoffatom, an das R₂ und R₃ gebunden sind, einen unsubstituierten oder mit einem, zwei oder drei Substituenten, ausgewählt aus der Gruppe, umfassend mit einem, zwei oder drei Substituenten, ausgewählt aus der Gruppe, umfassend Halogen und C₁-C₄-Alkoxy, substituiertes Phenyl, substituierten Cyclopentan- oder 5,6-Dihydro-2H-1,4-thiazin-Ring, an den ein unsubstituierter Benzolring kondensiert sein kann;
in freier Form oder gegebenenfalls in Salzform.

4. Verbindung nach Anspruch 1 der Formel I, worin bedeuten:
R₁ C₁-C₄-Alkyl; und entweder
(a) X ein Stickstoffatom; und
R₂ und R₃, unabhängig voneinander, C₁-C₄-Alkyl, C₃-C₆-Cycloalkyl, eine unsubstituierte oder mit einem, zwei oder drei Substituenten, ausgewählt aus der Gruppe, umfassend C₁-C₄-Alkoxy, Halogen, Halogen-C₁-C₄-alkyl, Halogen-C₁-C₄-alkoxy, C₃-C₆-Cycloalkylmethoxy, C₂-C₄-Alkenyloxy und C₂-C₄-Alkinyloxy, substituierte oder an zwei benachbarten Kohlenstoffatomen mit geradkettigem C₁-C₃-Alkylendioxy substituierte Phenyl- oder Naphthyl-Gruppe oder eine unsubstituierte Pyridyl- oder Benzofuryl-Gruppe; oder
(b) X eine Gruppe Ia; und
entweder R₂ und R₃, unabhängig voneinander, Wasserstoff, C₁-C₄-Alkyl, Halogen-C₁-C₄-alkyl, C₁-C₄-Alkoxymethyl, C₁-C₄-Alkylthio, C₁-C₄-Alkylthiomethyl, C₃-C₆-Cycloalkyl, Cyano oder eine unsubstituierte oder mit einem, zwei oder drei Substituenten, ausgewählt aus der Gruppe, umfassend Halogen, Nitro, C₁-C₄-Alkyl, Halogen-C₁-C₄-alkyl, C₁-C₄-Alkoxy, Halogen-C₁-C₄-alkoxy, C₃-C₆-Cycloalkylmethoxy, Cyano, Cyanomethoxy, C₁-C₄-Alkylthio, C₁-C₄-Alkylthiomethyl, Pyrimidinylthiomethyl, C₂-C₄-Alkenyloxy, C₂-C₄-Alkinyloxy, Phenoxy und C₁-C₄-Alkansulfinylmethyl, substituierte oder an zwei benachbarten Kohlenstoffatomen mit geradkettigem C₁-C₃-Alkylendioxy substituierte Benzyl-, Phenyl- oder Naphthyl-Gruppe oder eine unsubstituierte oder mit einem, zwei oder drei Substituenten, ausgewählt aus der Gruppe, umfassend Halogen und C₁-C₄-Alkyl, substituierte Pyridyl-, Furyl-, Benzofuryl-, Thienyl-, Benzothienyl-, Pyrrolyl- oder Thiazolyl-Gruppe oder
R₂ und R₃, zusammen mit dem Kohlenstoffatom, an das R₂ und R₃ gebunden sind, einen unsubstituierten oder mit einem, zwei oder drei Substituenten, ausgewählt aus der Gruppe, umfassend mit einem, zwei oder drei Substituenten, ausgewählt aus der Gruppe, umfassend Halogen und C₁-C₄-Alkoxy, substituiertes Phenyl, substituierten Cyclopentan- oder 5,6-Dihydro-2H-1,4-thiazin-Ring, an den ein unsubstituierter Benzolring kondensiert sein kann;
in freier Form oder gegebenenfalls in Salzform.

5. Verbindung nach Anspruch 1 der Formel I, worin bedeuten:
R₁ C₁-C₄-Alkyl; und entweder
(c) X ein Stickstoffatom;
R₂ eine unsubstituierte oder mit einem oder zwei Substituenten, ausgewählt aus der Gruppe, umfassend C₁-C₄-Alkoxy, C₂-C₄-Alkenyloxy, C₂-C₄-Alkinyloxy, C₃-C₄-Cycloalkylmethoxy, Halogen, Halogen-C₁-C₄-alkyl mit 1 bis einschließlich 3 Halogenatomen und Halogen-C₁-C₄-alkoxy mit 1 bis einschließlich 3 Halogenatomen, substituierte oder an zwei benachbarten Kohlenstoffatomen mit geradkettigem C₁-C₃-Alkylendioxy substituierte Phenylgruppe oder eine unsubstituierte Naphth-2-yl-, Benzo[b]fur-2-yl- oder Pyrid-2-yl-Gruppe; und
R₃ C₁-C₄-Alkyl oder C₃-C₄-Cycloalkyl; oder
(d) X eine Gruppe Ia; und
entweder R₂ C₁-C₄-Alkylthio, eine unsubstituierte oder mit einem oder zwei Substituenten, ausgewählt aus der Gruppe, umfassend Halogen, C₁-C₄-Alkyl, Halogen-C₁-C₄-alkyl mit 1 bis einschließlich 3 Halogenatomen, C₁-C₄-Alkoxy, Halogen-C₁-C₄-alkoxy mit 1 bis einschließlich 3 Halogenatomen, C₃-C₄-Cycloalkylmethoxy, C₁-C₄-Alkylthio, C₁-C₄-Alkylthiomethyl, C₂-C₄-Alkenyloxy, C₂-C₄-Alkinyloxy, Cyano, Cyanomethoxy, Nitro, Phenoxy, Pyrimidinylthiomethyl und C₁-C₄-Alkansulfinylmethyl, substituierte oder an zwei benachbarten Kohlenstoffatomen mit geradkettigem C₁-C₃-Alkylendioxy substituierte Phenylgruppe, eine unsubstituierte oder einfach mit Halogen substituierte Thien-2-yl-, Pyrid-2-yl- oder Pyrid-3-yl-Gruppe, eine unsubstituierte oder einfach mit C₁-C₄-Alkyl substituierte Pyrrol-2-yl-Gruppe oder eine unsubstituierte Naphth-2-yl-, Fur-2-yl-, Benzo[b]fur-2-yl- oder Thiazol-2-yl-Gruppe; und
R₃ Wasserstoff, C₁-C₄-Alkyl, Halogen-C₁-C₄-alkyl mit 1 bis einschließlich 3 Halogenatomen, C₁-C₄-Alkoxymethyl, C₁-C₄-Alkylthiomethyl, Cyano oder C₃-C₄-Cycloalkyl;
oder R₂ und R₃, zusammen mit dem Kohlenstoffatom, an das R₂ und R₃ gebunden sind, einen unsubstituierten Indanring, dessen Kohlenstoffatom in 1-Position Bestandteil der in der Formel I dargestellten N=C-Doppelbindung ist, oder einen unsubstituierten oder einfach mit Monohalogenphenyl oder Mono-C₁-C₄-alkoxyphenyl substituierten 5,6-Dihydro-2H-1,4-thiazin-Ring, dessen Kohlenstoffatom in 2-Position Bestandteil der in der Formel I dargestellten N=C-Doppelbindung ist;
in freier Form oder gegebenenfalls in Salzform.

6. Verbindung nach Anspruch 1 der Formel I, worin bedeuten:
R₁ C₁-C₄-Alkyl; und entweder
(e) X ein Stickstoffatom;
R₂ unsubstituiertes Phenyl, 3- oder 4-Halogenphenyl, 3-Halogen-C₁-C₄-alkylphenyl mit 1 bis einschließlich 3 Halogenatomen, 2-Halogen-5-halogen-C₁-C₄-alkylphenyl mit 1 bis einschließlich 3 Halogenatomen im Halogenalkyl-Substituenten, 3-C₁-C₄-Alkoxyphenyl, 3-Halogen-C₁-C₄-alkoxyphenyl mit 1 bis einschließlich 3 Halogenatomen, 3-C₂-C₄-Alkenyloxyphenyl, 3-C₂-C₄-Alkinyloxyphenyl, 3-C₃-C₄-Cycloalkylmethoxyphenyl, in 3- und 4-Position mit geradkettigem C₁-C₃-Alkylendioxy substituiertes Phenyl oder eine unsubstituierte Naphth-2-yl-, Benzo[b]fur-2-yl- oder Pyrid-2-yl-Gruppe; und
R₃ C₁-C₄-Alkyl oder C₃-C₄-Cycloalkyl; oder
(f) X eine Gruppe Ia; und
entweder R₂ C₁-C₄ Alkylthio, unsubstituiertes Phenyl, 2-, 3- oder 4-Halogenphenyl, 3,4-Dihalogenphenyl, worin die beiden Halogensubstituenten gleich oder verschieden sind, 3-oder 4-C₁-C₄-Alkylphenyl, 3-C₁-C₄-Alkyl-4-halogenphenyl, 2-oder 3-Halogen-C₁-C₄-alkylphenyl mit 1 bis einschließlich 3 Halogenatomen, 3,5-Bis(halogen-C₁-C₄-alkyl)-phenyl mit 1 bis einschließlich 3 Halogenatomen, 4-Halogen-3-halogen-C₁-C₄-alkylphenyl mit 1 bis einschließlich 3 Halogenatomen im Halogenalkyl-Substituenten, 2-Halogen-5-halogen-C₁-C₄-alkylphenyl mit 1 bis einschließlich 3 Halogenatomen im Halogenalkyl-Substituenten, 3-C₁-C₄-Alkoxyphenyl, 3-Halogen-C₁-C₄-alkoxyphenyl mit 1 bis einschließlich 3 Halogenatomen, 3-C₃-C₄-Cycloalkylmethoxyphenyl, 2-C₁-C₄-Alkylthio-5-halogen-C₁-C₄-alkylphenyl mit 1 bis einschließlich 3 Halogenatomen im Halogenalkyl-Substituenten, 4-C₁-C₄-Alkoxy-3-C₁-C₄-alkylthiomethylphenyl, 3-C₂-C₄-Alkenyloxyphenyl, 3-C₂-C₄-Alkinyloxyphenyl, 4-Phenoxyphenyl, 3- oder 4-Nitrophenyl, 3-Cyanophenyl, 3-Cyanomethoxyphenyl, 4-C₁-C₄-Alkoxy-3-nitrophenyl, 3-C₁-C₄-Alkansulfinylmethyl-4-C₁-C₄-alkoxyphenyl, 4-C₁-C₄-Alkoxy-3-pyrimidin-2-ylthiomethylphenyl, in 3- und 4-Position mit geradkettigem C₁-C₃-Alkylendioxy substituiertes Phenyl, eine unsubstituierte Thien-2-yl-, Pyrid-2-yl- oder Pyrid-3-yl-Gruppe, 6-Halogenpyrid-2-yl, 5-Halogenthien-2-yl, 1-C₁-C₄-Alkylpyrrol-2-yl oder eine unsubstituierte Naphth-2-yl-, Fur-2-yl-, Benzo[b]fur-2-yl- oder Thiazol-2-yl-Gruppe; und
R₃ Wasserstoff, C₁-C₄-Alkyl, Halogen-C₁-C₄-alkyl mit 1 bis einschließlich 3 Halogenatomen, C₁-C₄-Alkoxymethyl, C₁-C₄-Alkylthiomethyl, Cyano oder C₃-C₄-Cycloalkyl; oder
R₂ und R₃, zusammen mit dem Kohlenstoffatom, an das R₂ und R₃ gebunden sind, einen unsubstituierten Indanring, dessen Kohlenstoffatom in 1-Position Bestandteil der in der Formel I dargestellten N=C-Doppelbindung ist, oder einen einfach mit 4-Halogenphenyl oder 4-C₁-C₄-Alkoxyphenyl substituierten 5,6-Dihydro-2H-1,4-thiazin-Ring, dessen Kohlenstoffatom in 2-Position Bestandteil der in der Formel I dargestellten N=C-Doppelbindung ist;
in freier Form oder gegebenenfalls in Salzform.

7. Verbindung nach Anspruch 1 der Formel I, worin bedeuten:
X ein Stickstoffatom;
R₁ C₁-C₂-Alkyl;
R₂ 3-Fluorphenyl, 3-Trifluormethylphenyl, 3-Trifluormethoxyphenyl oder unsubstituiertes Pyrid-2-yl; und
R₃ C₁-C₂-Alkyl;
in freier Form oder gegebenenfalls in Salzform.

8. Verbindung nach Anspruch 1 der Formel I, worin bedeuten:
X ein Stickstoffatom;
R₁ C₁-C₂-Alkyl,
R₂ 3-Chlorphenyl, 4-Chlorphenyl, 3-Prop-1-in-3-yloxyphenyl, 4-Prop-1-in-3-yloxyphenyl; und
R₃ C₁-C₂-Alkyl;
in freier Form oder gegebenenfalls in Salzform.

9. Verbindung nach Anspruch 1 der Formel I, worin bedeuten:
X eine Gruppe Ia;
R₁ C₁-C₂-Alkyl;
R₂ unsubstituiertes Phenyl, 3-Fluorphenyl, 3-Chlorphenyl, 3-Bromphenyl, 3-Trifluormethylphenyl, 3-Cyclopropylmethoxyphenyl, 3-Allyloxyphenyl, 3,4-Ethylendioxyphenyl oder unsubstituiertes Pyrid-2-yl; und
R₃ C₁-C₂-Alkyl;
in freier Form oder gegebenenfalls in Salzform.

10. Verbindung nach Anspruch 1 der Formel I, ausgewählt aus der Gruppe von Verbindungen, umfassend
2-[3-(3-Aza-2-oxa-4-phenylpent-3-en-1-yl)benzo[b]-thien-2-yl]-3-methoxyacrylsäuremethylester,
2-{3-[3-Aza-2-oxa-4-(3-trifluormethylphenyl)-pent-3-en-1-yl]benzo[b]thien-2-yl}-3-methoxyacrylsäuremethylester,
2-{3-[3-Aza-4-(3-bromphenyl)-2-oxapent-3-en-1-yl]benzo[b]thien-2-yl}-3-methoxyacrylsäuremethylester,
2-{3-[3-Aza-4-(3,4-ethylendioxyphenyl)-2-oxapent-3-en-1-yl]benzo[b]thien-2-yl}-3-methoxyacrylsäuremethylester,
2-[3-(3-Aza-2-oxa-4-pyrid-2-ylpent-3-en-1-yl)benzo-[b]thien-2-yl]-3-methoxyacrylsäuremethylester,
2-{3-[3-Aza-4-(3-fluorphenyl)-2-oxapent-3-en-1-yl]-benzo[b]thien-2-yl}-3-methoxyacrylsäuremethylester,
2-{3-[3-Aza-4-(3-chlorphenyl)-2-oxapent-3-en-1-yl]-benzo[b]thien-2-yl}-3-methoxyacrylsäuremethylester,
2-{3-[3-Aza-2-oxa-4-(3-prop-1-en-3-yloxyphenyl)-pent-3-en-1-yl]benzo[b]thien-2-yl)-3-methoxyacrylsäuremethylester,
2-{3-[3-Aza-4-(3-cyclopropylmethoxyphenyl)-2-oxapent-3-en-1-yl]benzo[b]thien-2-yl}-3-methoxyacrylsäuremethylester,
2-{3-[3-Aza-2-oxa-4-(3-trifluormethylphenyl)-pent-3-en-1-yl]benzo[b]thien-2-yl}-2-methoxyiminoessigsäuremethylester,
2-{3-[3-Aza-2-oxa-4-(3-trifluormethoxyphenyl)-pent-3-en-1-yl]benzo[b]thien-2-yl}-2-methoxyiminoessigsäuremethylester,
2-{3-[3-Aza-4-(3-fluorphenyl)-2-oxapent-3-en-1-yl]-benzo[b]thien-2-yl}-2-methoxyiminoessigsäuremethylester und
2-[3-(3-Aza-2-oxa-4-pyrid-2-ylpent-3-en-1-yl)benzo-[b]thien-2-yl]-2-methoxyiminoessigsäuremethylester;
jeweils in freier Form oder gegebenenfalls in Salzform.

11. Verbindung nach Anspruch 1 der Formel I, ausgewählt aus der Gruppe von Verbindungen, umfassend
2-{3-[3-Aza-2-oxa-4-(3-prop-1-in-3-yloxyphenyl)-pent-3-en-1-yl]benzo[b]thien-2-yl}-3-methoxyiminoessigsäuremethylester;
2-{3-[3-Aza-4-(4-chlorphenyl)-2-oxapent-3-en-1-yl]-benzo[b]thien-2-yl}-3-methoxyiminoessigsäuremethylester;
2-{3-[3-Aza-4-(4-methylphenyl)-2-oxapent-3-en-1-yl]-benzo[b]thien-2-yl}-3-methoxyacrylsäuremethylester;
jeweils in freier Form oder gegebenenfalls in Salzform.

12. Verfahren zur Herstellung einer Verbindung der Formel I, in freier Form oder gegebenenfalls in Salzform, umfassend Umsetzen einer Verbindung der Formel 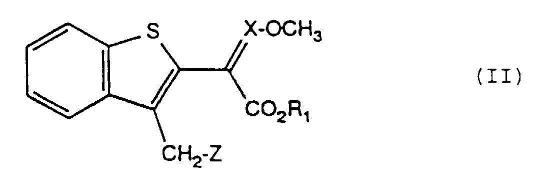 worin R₁ und X die für die Formel I angegebenen Bedeutungen haben und Z einen leicht abspaltbaren nucleofugen Rest, insbesondere Brom, bedeutet, mit einer Verbindung der Formel
HO - N = C(R₂)(R₃) (III),
worin R₂ und R₃ die für die Formel I angegebenen Bedeutungen aufweisen, oder gegebenenfalls einem Salz davon, vorzugsweise in Gegenwart einer Base, gegebenenfalls in Gegenwart eines inerten Lösungs- oder Verdünnungsmittels und/oder Überführen einer Verbindung der Formel I, die verfahrensgemäß oder auf andere Weise erhältlich ist, in freier Form oder gegebenenfalls in Salzform, in eine andere Verbindung der Formel I und/oder Auftrennen eines verfahrensgemäß erhältlichen Gemisches von Isomeren und Isolieren des gewünschten Isomers und/oder, falls erwünscht, Überführen einer verfahrensgemäß erhältlichen freien Verbindung der Formel I in ein Salz oder Überführen eines verfahrensgemäß erhältlichen Salzes einer Verbindung der Formel I in die freie Verbindung der Formel I oder gegebenenfalls in ein anderes Salz.

13. Mittel zum Schutz von Pflanzen gegen den Befall durch Schädlinge, umfassend mindestens eine Verbindung nach Anspruch 1, in freier Form oder in Salzform, als Wirkstoff und mindestens einen Hilfsstoff.

14. Mittel nach Anspruch 13 zum Schutz von Pflanzen gegen den Befall durch phytopathogene Mikroorganismen.

15. Verfahren zur Herstellung eines Mittels nach Anspruch 13, dadurch gekennzeichnet, daß man den Wirkstoff mit dem (den) Hilfsstoff(en) innig vermischt.

16. Verwendung einer Verbindung nach Anspruch 1, in freier Form oder in Salzform, oder eines Mittels nach Anspruch 13 zum Schutz von Pflanzen gegen den Befall durch Schädlinge.

17. Verwendung nach Anspruch 16 zum Schutz von Pflanzen gegen den Befall durch phytopathogene Mikroorganismen.

18. Verfahren zum Schutz von Pflanzen gegen den Befall durch Schädlinge, umfassend Applizieren einer Verbindung nach Anspruch 1, in freier Form oder in Salzform, als Wirkstoff auf die Pflanzen oder deren Standort.

19. Verfahren nach Anspruch 18 zum Schutz von Pflanzen gegen den Befall durch phytopathogene Mikroorganismen.

## Revendications

1. Composé de formule 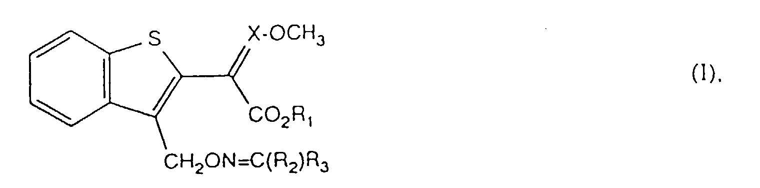 dans laquelle:
X est un atome d'azote ou un groupe de formule CH (Ia) ;
R₁ est un alkyle en C₁ à C₄; et
soit R₂ et R₃ indépendamment l'un de l'autre représentent un hydrogène, un alkyle en C₁ à C₄, un halo-alkyle en C₁ à C₄, un alcoxy en C₁ à C₄-méthyle, un alkylthio en C₁ à C₄, un alkyle en C₁ à C₄-thiométhyle, un aryle non substitué ou substitué, un hétéroaryle, un aryl-alkyle en C₁ à C₄, un hétéroaryl-alkyle en C₁ à C₄, un aryloxy-alkyle en C₁ à C₄, un hétéroaryloxy-alkyle en C₁ à C₄, un arylthio-alkyle en C₁ à C₄, un hétéroarylthio-alkyle en C₁ à C₄, un arylcarbonyle ou un hétéroarylcarbonyle, un alcényle en C₂ à C₄, un groupe aryl-alcényle en C₂ à C₄, ou hétéroaryl-alcényle en C₂ à C₄ non substitué ou substitué, un alcynyle en C₂ à C₄, un cycloalkyle en C₃ à C₆, un cyano,
soit R₂ et R₃ avec l'atome de carbone auquel R₂ et R₃ sont attachés représentent un noyau à 4 à 7 chaînons carbo- ou hétérocyclique, saturé ou insaturé, non-substitué ou substitué, auquel peut être condensé un noyau benzène non-substitué ou substitué; sous forme libre, ou, si besoin, sous forme de sel.

2. Composé selon la revendication 1 de formule I, dans laquelle:
X est un atome d'azote ou un groupe Ia;
R₁ est un alkyle en C₁ à C₄; et
ou bien R₂ et R₃ indépendamment l'un de l'autre représentent un hydrogène, un alkyle en C₁ à C₄, un halo-alkyle en C₁ à C₄, un alcoxy en C₁ à C₄, un alcoxy en C₁ à C₄-méthyle, un alkylthio en C₁ à C₄, un alkyle en C₁ à C₄-thiométhyle, un alcényle en C₂ à C₄, un alcynyle en C₂ à C₄, un cycloalkyle en C₃ à C₆, un cyano, un aryle, un aryl-alkyle en C₁ à C₄, un aryloxy-alkyle en C₁ à C₄, un arylthio-alkyle en C₁ à C₄, un aryl-alcényle en C₂ à C₄ ou un arylcarbonyle qui est non-substitué ou substitué par un, deux ou trois substituants choisis dans le groupe comprenant halogène, cyano, cyanométhoxy, nitro, triméthylsilyle, alkyle en C₁ à C₄, halo-alkyle en C₁ à C₄, alcoxy en C₁ à C₄, halo-alcoxy en C₁ à C₄, alcoxy en C₁ à C₄-méthyle, aryloxyméthyle, hétéroaryloxyméthyle, arylméthoxy, hétéroarylméthoxy, cycloalkyle en C₃ à C₆-méthoxy, alkylthio en C₁ à C₄, alkyle en C₁ à C₄-thiométhyle, arylthiométhyle, hétéroarylthiométhyle, alcényloxy en C₂ à C₄, alcynyloxy C₂ à C₄, aryloxy, hétéroaryloxy, alcane en C₁ à C₄-sulfinylméthyle, alcane en C₁ à C₄-sulfonylméthyle, arylsulfinylméthyle, arylsulfonylméthyle, hétéroarylsulfinylméthyle, hétéroarylsulfonylméthyle, les groupes de formule N(R₄)R₅ (Ib) et les groupes de formule CH₂N(R₄)R₅ (Ic), dans lesquels ou bien R₄ et R₅ représentent dans chaque cas indépendamment l'un de l'autre un hydrogène, un alkyle en C₁ à C₄, un aryle, un aryl-alkyle en C₁ à C₄ ou un hétéroaryle, ou bien R₄ et R₅ avec l'atome d'azote auquel R₄ et R₅ sont attachés forment un noyau pyrrolidine, pipéridine ou morpholine dont chacun est non-substitué ou substitué par un ou deux substituants choisis dans le groupe comprenant alkyle en C₁ à C₄ et/ou ce groupe aryle, aryl-alkyle en C₁ à C₄, aryloxy-alkyle en C₁ à C₄, arylthio-alkyle en C₁ à C₄, aryl-alcényle en C₂ à C₄ ou arylcarbonyle, est substitué sur deux atomes de carbone adjacents par un alcylène en C₃ à C₅ à chaîne droite qui est non substitué ou a un, deux ou trois substituants choisis dans le groupe comprenant alkyle en C₁ à C₄, alcoxy en C₁ à C₄, halogène, phényle et oxo et dans lequel un ou deux groupes méthylène non adjacents peuvent être remplacés par un oxygène, ou un groupe hétéroalkyle, hétéroaryl-alkyle en C₁ à C₄, hétéroaryloxy-alkyle en C₁ à C₄, hétéroarylthio-alkyle en C₁ à C₄, hétéroaryl-alcényle en C₂ à C₄ ou hétéroarylcarbonyle, dont chacun est non-substitué ou substitué par un, deux ou trois substituants choisis dans le groupe comprenant halogène, cyano, cyanométhoxy, nitro, triméthylsilyle, alkyle en C₁ à C₄, halo-alkyle en C₁ à C₄, alcoxy en C₁ à C₄, halo-alcoxy en C₁ à C₄, alcoxy en C₁ à C₄-méthyle, aryloxyméthyle, hétéroaryloxyméthyle, arylméthoxy, hétéroarylméthoxy, cycloalkyle en C₃ à C₆-méthoxy, alkylthio en C₁ à C₄, alkyle en C₁ à C₄-thiométhyle, arylthiométhyle, hétéroarylthiométhyle, alcényloxy en C₂ à C₄, alcynyloxy en C₂ à C₄, aryloxy, hétéroaryloxy, alcane en C₁ à C₄-sulfinylméthyle, alcane en C₁ à C₄-sulfonylméthyle, arylsulfinylméthyle, arylsulfonylméthyle, hétéroarylsulfinylméthyle, hétéroarylsulfonylméthyle, les groupes Ib et les groupes Ic, dans lesquels ou bien R₄ et R₅ représentent dans chaque cas indépendamment l'un de l'autre un hydrogène, un alkyle en C₁ à C₄, un aryle, un aryl-alkyle en C₁ à C₄ ou un hétéroaryle, ou bien R₄ et R₅ avec l'atome d'azote auquel R₄ et R₅ sont attachés forment un noyau pyrrolidine, pipéridine ou morpholine, dont chacun est non-substitué ou substitué par un ou deux substituants choisis dans le groupe comprenant alkyle en C₁ à C₄,
ou bien R₂ et R₃ avec l'atome de carbone auquel R₂ et R₃ sont attachés forment un noyau qui est non-substitué ou substitué par un, deux ou trois substituants choisis dans le groupe comprenant alkyle en C₁ à C₄, phényle et phényle qui est substitué par un, deux ou trois substituants choisis parmi le groupe comprenant halogène, cyano, cyanométhoxy, nitro, triméthylsilyle, alkyle en C₁ à C₄, halo-alkyle en C₁ à C₄, alcoxy en C₁ à C₄, halo-alcoxy en C₁ à C₄, alcoxy en C₁ à C₄-méthyle, aryloxyméthyle, hétéroaryloxyméthyle, arylméthoxy, hétéroarylméthoxy, cycloalkyle en C₃ à C₆-méthoxy, alkylthio en C₁ à C₄, alkyle en C₁ à C₄-thiométhyle, arylthiométhyle, hétéroarylthiométhyle, alcényloxy en C₂ à C₄, alcynyloxy en C₂ à C₄, aryloxy, hétéroaryloxy, alcane en C₁ à C₄-sulfinylméthyme, alcane en C₁ à C₄ sulfonylméthyle, arylsulfinylméthyle, arylsulfonylméthyle, hétéroarylsulfinylméthyle, hétéro-arylsulfonylméthyle, les groupes Ib et les groupes Ic, dans lesquels ou bien R₄ et R₅ représentent dans chaque cas indépendamment l'un de l'autre un hydrogène, un alkyle en C₁ à C₄, un aryle, un aryl-alkyle en C₁ à C₄ ou un hétéroaryle, ou bien R₄ et R₅ ensemble avec l'atome d'azote auquel R₄ et R₅ sont attachés forment un noyau pyrrolidine, pipéridine ou morpholine, dont chacun est non-substitué ou substitué par un ou deux substituants choisis dans le groupe comprenant alkyle en C₁ à C₄, et/ou ce phényle est substitué sur deux atomes de carbone adjacents par un alkylène en C₃ à C₅ à chaîne droite qui est non-substitué ou a un, deux ou trois substituants choisis dans le groupe comprenant alkyle en C₁ à C₄, alcoxy en C₁ à C₄, halogène, phényle et oxo, et où un ou deux groupes méthylène non adjacents peuvent être remplacés par un oxygène, ce noyau étant saturé ou ayant une seule double liaison ou deux ou trois doubles liaisons non cumulatives, le nombre maximum de doubles liaisons qui est possible du point de vue structurel étant fonction de la taille du noyau et du nombre ainsi que de la nature des hétéroatomes, et ce noyau étant un noyau carbocyclique à 4 à 7 chaînons ou un noyau hétérocyclique ayant de 1 à 3 hétéroatomes choisis dans le groupe comprenant les atomes d'oxygène, de soufre et d'azote, et auquel noyau on peut condenser un noyau benzène qui est non-substitué ou substitué par un, deux ou trois substituants choisis dans le groupe comprenant halogène, cyano, cyanométhoxy, nitro, triméthylsilyle, alkyle en C₁ à C₄, halo-alkyle en C₁ à C₄, alcoxy en C₁ à C₄, halo-alcoxy en C₁ à C₄, alcoxy en C₁ à C₄-méthyle, aryloxyméthyle, hétéroaryloxyméthyle, arylméthoxy, hétéroarylméthoxy, cycloalkyle en C₃ à C₆-méthoxy, alkylthio en C₁ à C₄, alkyle en C₁ à C₄-thiométhyle, arylthiométhyle, hétéroarylthiométhyle, alcényloxy en C₂ à C₄, alcynyloxy en C₂ à C₄, aryloxy, hétéroaryloxy, alcane en C₁ à C₄-sulfinyméthyle, alcane en C₁ à C₄-sulfonylméthyle, arylsulfinylméthyle, arylsulfonylméthyle, hétéroarylsulfinylméthyle, hétéroarylsulfonylméthyle, les groupes Ib et les groupes Ic, dans lesquels ou bien R₄ et R₅ représentent dans chaque cas indépendamment l'un de l'autre un hydrogène, un alkyle en C₁ à C₄, un aryle, un aryl-alkyle en C₁ à C₄, ou un hétéroaryle, ou bien R₄ et R₅ avec l'atome d'azote auquel R₄ et R₅ sont attachés forment un noyau pyrrolidine, pipéridine ou morpholine, dont chacun est non-substitué ou substitué par un ou deux substituants choisis dans le groupe comprenant alkyle en C₁ à C₄, et/ou ce noyau benzène est substitué sur deux atomes de carbone adjacents par un alkylène en C₃ à C₅ à chaîne droite qui est non-substitué ou a un, deux ou trois substituants choisis dans le groupe comprenant alkyle en C₁ à C₄, alcoxy en C₁ à C₄, halogène, phényle et oxo, et dans lequel un ou deux groupes méthylène non-adjacents peuvent être remplacés par de l'oxygène,
où "aryle" - comme groupe en tant que tel et comme élément de structure d'autres groupes - dans chaque cas indépendamment l'un de l'autre - est un groupe aromatique carbocyclique ayant de 6 à 14 atomes de carbone, et "hétéroaryle" - comme groupe en tant que tel et comme élément de structure d'autres groupes - dans chaque cas indépendamment l'un de l'autre est un groupe aromatique hétérocyclique ayant de 5 à 14 éléments dans le noyau, dont de 1 à 3 membres sont des hétéroatomes choisis dans le groupe comprenant les atomes d'oxygène, de soufre et d'azote; sous forme libre ou, selon les besoins, sous forme de sel.

3. Composé selon la revendication 1 de formule I, dans laquelle
X est un atome d'azote ou un groupe Ia;
R₁ est un alkyle en C₁ à C₄; et
ou bien R₂ et R₃ indépendamment l'un de l'autre représentent un hydrogène, un alkyle en C₁ à C₄, un halo-alkyle en C₁ à C₄, un alcoxy en C₁ à C₄-méthyle, un alkylthio en C₁ à C₄, un alkyle en C₁ à C₄-thiométhyle, un cycloalkyle en C₃ à C₆, un cyano ou un groupe benzyle, phényle ou naphtyle, chacun étant non-substitué ou substitué par un, deux ou trois substituants choisis dans le groupe comprenant halogène, nitro, alkyle en C₁ à C₄, halo-alkyle en C₁ à C₄, alcoxy en C₁ à C₄, halo-alcoxy en C₁ à C₄, cycloalkyle en C₃ à C₆-méthoxy, cyano, cyanométhyle, alkylthio en C₁ à C₄, alkyle en C₁ à C₄-thiométhyle, pyrimidinylthiométhyle, alcényloxy en C₂ à C₄, alcynyloxy C₂ à C₄, phénoxy et alcane en C₁ à C₄-sulfinylméthyle, ou dont chacun est substitué par deux atomes de carbone adjacents par un alkylènedioxy en C₁ à C₃ à chaîne droite ou représentent un noyau pyridyle, furyle, benzofuryle, thiényle, benzothiényle, pyrrolyle ou thiazolyle, dont chacun est non-substitué ou substitué par un, deux ou trois substituants choisis dans le groupe comprenant halogène et alkyle en C₁ à C₄,
ou bien R₂ et R₃ avec l'atome d'azote auquel R₂ et R₃ sont attachés représentent un noyau cyclopentane ou 5,6-dihydro-2H-1,4-thiazine, dont chacun est non-substitué ou substitué par un, deux ou trois substituants choisis dans le groupe comprenant un phényle qui est substitué par un, deux ou trois substituants choisis dans le groupe comprenant halogène et alcoxy en C₁ à C₄, auquel noyau cyclopentane ou 5,6-dihydro-2H-1,4-thiazine peut être condensé un noyau benzène non substitué; sous forme libre ou, selon les besoins, sous forme de sel.

4. Composé selon la revendication 1 de formule I, dans laquelle:
R₁ est un alkyle en C₁ à C₄; et ou bien
(a) X est un atome d'azote, et
R₂ et R₃ représentent indépendamment l'un de l'autre un groupe alkyle en C₁ à C₄, cycloalkyle en C₃ à C₆, phényle ou naphtyle, dont chacun est non-substitué ou substitué par un, deux ou trois substituants choisis dans le groupe comprenant alcoxy en C₁ à C₄, halogène, halo-alkyle en C₁ à C₄, halo-alcoxy en C₁ à C₄, cycloalkyle en C₃ à C₆-méthoxy, alcényloxy en C₂ à C₄ et alcynyloxy en C₂ à C₄, ou bien ce groupe phényle ou naphtyle est substitué sur deux atomes de carbone adjacents par un alkylène en C₁ à C₃-dioxy à chaîne droite, ou sont un groupe pyridyle ou benzofuryle non substitué; ou bien
(b) X est un groupe Ia; et
ou bien R₂ et R₃ représentent indépendamment l'un de l'autre un hydrogène, un alkyle en C₁ à C₄, un halo-alkyle en C₁ à C₄, alcoxy en C₁ à C₄-méthyle, un alkylthio en C₁ à C₄, un alkyle en C₁ à C₄-thiométhyle, un cycloalkyle en C₃ à C₆, un cyano ou un groupe benzyle, phényle ou naphtyle qui est non substitué ou substitué par un, deux ou trois substituants choisis dans le groupe comprenant halogène, nitro, alkyle en C₁ à C₄, halo-alkyle en C₁ à C₄, alcoxy en C₁ à C₄, halo-alcoxy en C₁ à C₄, cycloalkyle en C₃ à C₆-méthoxy, cyano, cyanométhoxy, alkylthio en C₁ à C₄, alkyle en C₁ à C₄-thiométhyle, pyrimidinylthiométhyle, alcényloxy en C₂ à C₄, alcynyloxy en C₂ à C₄, phénoxy et alcane en C₁ à C₄-sulfinylméthyle, ou ce groupe benzyle, phényle ou naphtyle est substitué sur deux atomes de carbone adjacents par un alkylène en C₁ à C₃-dioxy à chaîne droite, ou représentent un groupe pyridyle, furyle, benzofuryle, thiényle, benzothiényle, pyrrolyle ou thiazolyle, dont chacun est non-substitué ou substitué par un, deux ou trois substituants choisis dans le groupe comprenant halogène et alkyle en C₁ à C₄,
ou bien R₂ et R₃ ensemble avec l'atome de carbone auquel R₂ et R₃ sont attachés représentent un noyau cyclopentane ou 5,6-dihydro-2H-1,4-thiazine qui est non-substitué ou substitué par un, deux ou trois substituants choisis dans le groupe comprenant phényle substitué par un, deux ou trois substituants choisis dans le groupe comprenant halogène et alcoxy en C₁ à C₄, auquel noyau cylopentane ou 5,6-dihydro-2H-1,4-thiazine peut être condensé un noyau benzène non substitué; sous forme libre, ou, selon les besoins, sous forme de sel.

5. Composé selon la revendication 1 de formule I dans laquelle:
R₁ est un alkyle en C₁ à C₄; et ou bien
(c) X est un atome d'azote;
R₂ est un groupe phényle qui est non substitué ou substitué par un ou deux substituants choisis dans le groupe comprenant alcoxy en C₁ à C₄, alcényloxy en C₂ à C₄, alcynyloxy en C₂ à C₄, cycloalkyle en C₃ à C₄-méthoxy, halogène, halo-alkyle en C₁ à C₄ ayant de 1 à 3 atomes d'halogène compris et halo-alcoxy en C₁ à C₄ ayant de 1 à 3 atomes d'halogène compris, ou ce groupe phényle est substitué sur deux atomes de carbone adjacents par un alkylènedioxy en C₁ à C₃, ou bien R₂ est un groupe napht-2-yle, benzo[b]fur-2-yle ou pyrid-2-yle non substitué; et
R₃ est un alkyle en C₁ à C₄ ou un cycloalkyle en C₁ à C₄, ou un cycloalkyle en C₃ à C₄ ; ou
(d) X est un groupe Ia; et
ou bien R₂ est un alkylthio en C₁ à C₄, un groupe phényle qui est non substitué ou substitué par un ou deux substituants choisis dans le groupe comprenant halogène, alkyle en C₁ à C₄, halo-alkyle en C₁ à C₄ ayant de 1 à 3 atomes d'halogène compris, alcoxy en C₁ à C₄, halo-alcoxy en C₁ à C₄ ayant de 1 à 3 atomes d'halogène compris, cycloalkyle en C₃ à C₄-méthoxy, alkylthio en C₁ à C₄, alkyle en C₁ à C₄-thiométhyle, alcényloxy en C₂ à C₄, alcynyloxy en C₂ à C₄, cyano, cyanométhoxy, nitro, phénoxy, pyrimidinylthiométhyle et alcane en C₁ à C₄-sulfinylméthyle, ou qui est substitué sur deux atomes de carbone adjacents par un alkylènedioxy en C₁ à C₃ à chaîne droite, ou R₂ est un groupe thién-2-yle, pyrid-2-yle ou pyrid-3-yle qui est non substitué ou mono-substitué par un halogène ou un groupe pyrrol-2-yle non-substitué ou mono-substitué par un alkyle en C₁ à C₄, ou un groupe naphth-2-yle, fur-2-yle, benzo[b]fur-2-yle ou thiazol-2-yle non-substitué; et
R₃ est un hydrogène, un alkyle en C₁ à C₄, un halo-alkyle en C₁ à C₄ ayant de 1 à 3 atomes d'halogène compris, un alcoxy en C₁ à C₄-méthyle, un alkyle en C₁ à C₄-thiométhyle, un cyano ou un cycloalkyle en C₃ à C₄;
ou bien R₂ et R₃ ensemble avec l'atome d'azote auquel R₂ et R₃ sont attachés représentent un noyau indane substitué dont l'atome de carbone en position 1 est un élément de la double liaison N=C présentée dans la formule I, ou sont un noyau 5,6-dihydro-2H-1,4-thiazine qui est non-substitué ou mono-substitué par un monohalophényle ou un mono-alcoxy en C₁ à C₄-phényle et dont l'atome de carbone en position 2 est un élément de la double liaison N=C présentée dans la formule I; sous forme libre ou, selon les besoins, sous forme de sel.

6. Composé selon la revendication 1 de formule I, dans laquelle
R₁ est un alkyle en C₁ à C₄ ; et ou bien
(e) X est un atome d'azote;
R₂ est un phényle non substitué, un 3- ou 4-halophényle, un 3-halo-alkyle en C₁ à C₄-phényle ayant de 1 à 3 atomes d'halogène compris, un 2-halo-5-halo-alkyle en C₁ à C₄-phényle ayant de 1 à 3 atomes d'halogène compris dans le substituant halo-alkyle, un 3-alcoxy en C₁ à C₄-phényle, un 3-halo-alcoxy en C₁ à C₄-phényle ayant de 1 à 3 atomes d'halogène compris, un 3-alcényloxy en C₂ à C₄-phényle, un 3-alcynyloxy en C₂ à C₄-phényle, un 3-cycloalkyle en C₃ à C₄-méthoxyphényle, un phényle qui est non substitué dans les positions 3 et 4 par un alkylènedioxy en C₁ à C₃ à chaîne droite, ou un groupe napht-2-yle, benzo[b]fur-2-yle ou ou pyrid-2-yle non substitué; et
R₃ est un alkyle en C₁ à C₄ ou un cycloalkyle en C₃ à C₄; ou bien
(f) X est un groupe Ia; et
ou bien R₂ est un alkylthio en C₁ à C₄, un phényle non substitué, un 2-, 3- ou 4-halophényle, un 3,4-dihalophényle dans lequel les deux substituants halogène sont identiques ou différents, un 3- ou 4-alkyle en C₁ à C₄-phényle, un 3-alkyle en C₁ à C₄-4-halophényle, un 2-ou 3-halo-alkyle en C₁ à C₄-phényle ayant de 1 à 3 atomes d'halogène compris, un 3,5-bis(halo-alkyle en C₁ à C₄)phényle ayant de 1 à 3 atomes d'halogène compris, un 4-halo-3-halo-alkyle en C₁ à C₄-phényle ayant de 1 à 3 atomes d'halogène compris dans le substituant haloalkyle, un 2-halo-5-halo-alkyle en C₁ à C₄-phényle ayant de 1 à 3 atomes d'halogène compris dans le substituant halo-alkyle, un 3-alcoxy en C₁ à C₄-phényle, un 3-halo-alcoxy en C₁ à C₄-phényle ayant de 1 à 3 atomes d'halogène compris, un 3-cycloalkyle en C₃ à C₄-méthoxyphyényle, un 2-alkylthio en C₁ à C₄-5-halo-alkyle en C₁ à C₄-phényle ayant de 1 à 3 atomes d'halogène compris dans le substituant halo-alkyle, un 4-alcoxy en C₃ à C₄-alkyle en C₁ à C₄-thiométhylphényle, un 3-alcényloxy en C₂ à C₄-phényle, un 3-alcynyloxy en C₂ à C₄-phényle, un 4-phénoxyphényle, un 3 ou 4-nitrophényle, un 3-cyanophényle, un 3-cyanométhoxyphényle, un 4-alcoxy en C₁ à C₄-3-nitrophényle, un 3-alcane en C₁ à C₄-sulfinylméthyl-4-alcoxy en C₁ à C₄-phényle, un 4-alcoxy en C₁ à C₄-3-pyrimidin-2-ylthiométhylphényle, un phényle qui est substitué dans les positions 3 et 4 par un alkylènedioxy en C₁ à C₃ à chaîne droite, ou un thièn-2-yle, pyrid-2-yle ou pyrid-3-yle non substitué, un 6-halopyrid-2-yle, un 5-halothièn-2-yle, un 1-alkyle en C₁ à C₄-pyrrol-2-yle ou un groupe napht-2-yle, fur-2-yle, benzo[b]fur-2-yle ou thiazol-2-yle non-substitué; et
R₃ est un hydrogène, un alkyle en C₁ à C₄, un halo-alkyle en C₁ à C₄ ayant de 1 à 3 atomes d'halogène compris, un alcoxy en C₁ à C₄-méthyle, un alkyle en C₁ à C₄-thiométhyle, un cyano ou un cycloalkyle en C₃ à C₄;
ou bien R₂ et R₃ avec l'atome de carbone auquel R₂ et R₃ sont attachés représentent un noyau indane non substitué dont l'atome de carbone en position 1 est un élément de la double liaison N=C présentée dans la formule I, ou bien un noyau 5,6-dihydro-2H-1,4-thiazine qui est mono-substitué par un 4-halophényle ou un 4-alcoxy en C₁ à C₄-phényle et dont l'atome de carbone en position 2 est un élément de la double liaison N=C présentée dans la formule I; sous forme libre, ou, selon les besoins, sous forme de sel.

7. Composé selon la revendication 1 de formule I, dans laquelle:
X est un atome d'azote;
R₁ est un alkyle en C₁ à C₂;
R₂ est un 3-fluorophényle, 3-trifluorométhylphényle, 3-trifluorométhoxyphényle ou pyrid-2-yle non-substitué; et
R₃ est un alkyle en C₁ à C₂; sous forme libre ou, selon les besoins, sous forme de sel.

8. Composé selon la revendication 1 de formule I dans laquelle:
X est un atome d'azote;
R₁ est un alkyle en C₁ à C₂;
R₂ est un 3-chlorophényle, 4-chlorophényle, 3-prop-1-yn-3-yloxyphényle ou 4-prop-1-yn-3-yloxyphényle;
R₃ est un alkyle en C₁ à C₂; sous forme libre ou, selon les besoins, sous forme de sel.

9. Composé selon la revendication 1 de formule I dans laquelle:
X est un groupe Ia;
R₁ est un alkyle en C₁ à C₂;
R₂ est un phényle non substitué, un 3-fluorophényle, 3-chlorophényle, 3-bromophényle, 3-trifluorométhylphényle, 3-cyclopropylméthoxyphényle, 3-allyloxyphényle, 3,4-éthylènedioxyphényle ou un pyrid-2-yle non substitué; et
R₃ est un alkyle en C₁ à C₂; sous forme libre ou, selon les besoins, sous forme de sel.

10. Composé selon la revendication 1 de formule I, choisi dans le groupe de composés comprenant les suivants:
2-[3-(3-aza-2-oxa-4-phénylpent-3-én-1-yl)benzo[b]thièn-2-yl]-3-méthoxyacrylate de méthyle ;
2-{3-[3-aza-2-oxa-4-(3-trifluorométhylphényl)pent-3-én-1-yl]benzo[b]thién-2-yl}-3-méthoxyacrylate de méthyle;
2-{3-[3-aza-4-(3-bromophényl)-2-oxapent-3-én-1-yl]benzo[b] thién-2-yl}-3-méthoxyacrylate de méthyle;
2-{3-[3-aza-4-(3,4-éthylènedioxyphényl)-2-oxapent-3-én-1-yl]benzo[b)thién-2-yl}3-méthoxyacrylate de méthyle;
2-[3-(3-aza-2-oxa-4-pyrid-2-ylpent-3-én-1-yl)benzo[b] thién-2-yl]-3-méthoxy-acrylate de méthyle;
2-{3-[3-aza-4-(3-fluorophényl)-2-oxapent-3-én-1-yl]benzo[b ]thién-2-yl}-3-méthoxyacrylate de méthyle;
2-{3-[3-aza-4-(3-chlorophényl)-2-oxapent-3-én-1-yl]benzo[b ]thién-2-yl}-3-méthoxyacrylate de méthyle;
2-{3-[3-aza-2-oxa-4-(3-prop-1-én-3-yloxyphényl)pent-3-én-1-yl]benzo[b]thién-2-yl}-3-méthoxyacrylate de méthyle;
2-{3-[3-aza-4-(3-cyclopropylméthoxyphényl)-2-oxapent-3-én-1-yl]benzo[b]thién-2-yl}-3-méthoxyacrylate de méthyle;
2-{3-[3-aza-2-oxa-4-(3-trifluorométhylphényl)pent-3-én-1-yl]benzo[b]thién-2-yl}-2-méthoxyiminoacétate de méthyle;
2-{3-[3-aza-2-oxa-4-(3-trifluorométhoxyphényl)pent-3-én-1-yl]benzo[b]thién-2-yl}-2-méthoxyiminoacétate de méthyle;
2-{3-[3-aza-4-(3-fluorophényl)-2-oxapent-3-én-1-yl]benzo[b ]thién-2-yl}-2-méthoxyiminoacétate de méthyle;
2-[3-(3-aza-2-oxa-4-pyrid-2-ylpent-3-én-1-yl)benzo [b] thién-2-yl]-2-méthoxyiminoacétate de méthyle;
dans chaque cas sous forme libre, ou selon les besoins, sous forme de sel.

11. Composé selon la revendication 1 de formule I, choisis parmi le groupe de composés comprenant:
2-{3-[3-aza-2-oxa-4-(3-prop-1-yn-3-yloxyphényl)pent-3-én-1-yl]benzo[b]thién-2-yl]-2-méthoxyiminoacétate de méthyle;
2-{3-[3-aza-4-(4-chlorophényl)-2-oxapent-3-én-1-yl]benzo[b ]thién-2-yl}-2-méthoxyiminoacétate de méthyle;
2-{3-[3-aza-4-(4-méthylphényl)-2-oxapent-3-én-1-yl)benzo[ b]thién-2-yl}-3-méthoxyacryalte de méthyle;
dans chaque cas, sous forme libre ou, selon les besoins, sous forme de sel.

12. Procédé de préparation d'un composé de formule I, sous forme libre, ou selon les besoins, sous forme de sel dans lequel on fait réagir un composé de formule : 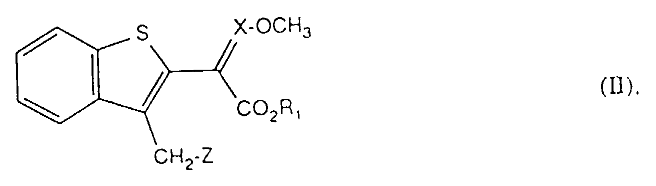 dans laquelle R₁ et X sont tels que définis dans la formule I et Z est un radical nucléofuge facilement détachable, en particulier le brome, avec un composé de formule
HO - N=C(R₂)(R₃) (II)
dans laquelle R₂ et R₃ sont tels que définis dans la formule I, ou, selon les besoins, avec un de ses sels, de préférence en présence d'une base, en présence ou en l'absence d'un solvant ou diluant inertes, et/ou on transforme un composé de formule I que l'on peut obtenir par le procédé ou par une autre voie, sous forme libre, ou, selon les besoins, sous forme de sel, en un composé de formule I différent, et/ou on dédouble un mélange d'isomères que l'on peut obtenir selon le procédé et en isolant l'isomère désiré, et/ou si on le désire, on transforme un composé libre de formule I que l'on peut obtenir selon le procédé en un sel, ou on transforme un sel d'un composé de formule I que l'on peut obtenir selon le procédé en le composé libre de formule I, ou, selon les besoins, en un sel différent.

13. Composition de protection des plantes contre l'infestation par des parasites, qui comprend au moins un composé selon la revendication 1, sous forme libre ou sous forme de sel, comme ingrédient actif et au moins un agent auxiliaire.

14. Composition selon la revendication 13 pour protéger les plantes contre l'infestation par les microorganismes phytopathogènes.

15. Procédé de préparation d'une composition selon la revendication 13, dans lequel on mélange intimement l'ingrédient actif avec le ou les auxiliaires.

16. Application d'un composé selon la revendication 1 sous forme libre ou sous forme de sel, ou d'une composition selon la revendication 13, à la protection des plantes contre l'infestation par les parasites.

17. Application selon la revendication 16 pour protéger les plantes contre l'infestation par les microorganismes phytopathogènes.

18. Procédé de protection des plantes contre l'infestation des plantes par des pesticides, dans lequel on applique un composé selon la revendication 1, sous forme libre ou sous forme de sel, comme ingrédient actif aux plantes ou à leur habitat.

19. Procédé selon la revendication 18 pour protéger les plantes contre l'infestation par des microorganismes phytopathogènes.
